(19) Europäisches Patentamt
European Patent Office
Office européen des brevets

(11) EP 0 579 833 B1

# (12) EUROPEAN PATENT SPECIFICATION

(45) Date of publication and mention of the grant of the patent: **20.08.1997 Bulletin 1997/34**

(21) Application number: **92908496.0**

(22) Date of filing: **10.04.1992**

(51) Int. Cl.$^6$: **C07C 217/74**

(86) International application number: **PCT/JP92/00449**

(87) International publication number: **WO 92/18461 (29.10.1992 Gazette 1992/27)**

## (54) ETHANOLAMINE DERIVATIVES WITH ANTI-POLLAKIURIA ACTIVITY

ETHANOLAMINDERIVATE MIT ANTI-POLLAKIURAWIRKUNG

DERIVES D'ETHANOLAMINE PRESENTANT UNE ACTIVITE ANTI-POLLAKIURIE

(84) Designated Contracting States:
**AT BE CH DE DK ES FR GB GR IT LI LU NL SE**

(30) Priority: **12.04.1991 GB 9107827**

(43) Date of publication of application: **26.01.1994 Bulletin 1994/04**

(73) Proprietor:
**FUJISAWA PHARMACEUTICAL CO., LTD.**
**Osaka-shi Osaka 541 (JP)**

(72) Inventors:
- **SHIOKAWA, Youichi**
**Ibarake-shi, Osaka 567 (JP)**
- **NAGANO, Masanobu**
**Kawanishi-shi Hyogo 666-01 (JP)**
- **TANIGUCHI, Kıyoshi**
**Kobe-shi Hyogo 654-01 (JP)**
- **TAKE Kazuhiko**
**Tondabayashi-shi Osaka 584 (JP)**
- **KATO, Takeshi**
**Nishinomiya-shi Hyogo 663 (JP)**
- **TSUBAKI, Kazunori**
**Suita-shi Osaka 565 (JP)**

(74) Representative: **Hrabal, Ulrich, Dr. et al**
**Türk, Gille, Hrabal, Leifert**
**Brucknerstrasse 20**
**40593 Düsseldorf (DE)**

(56) References cited:
**EP-A- 0 211 721**
**EP-A- 0 303 545**
**EP-A- 0 303 546**
**EP-A- 0 383 686**
**EP-A- 0 403 360**

- **Chemical Abstracts, vol. 111, no. 17, 23 October 1989, Columbus, Ohio, US, L. MANARA et al, "New developments in beta-adrenergic-mediated control of intestinal motility: gut-specific phenylethanolami notetralines", page 12, column 2, abstract no. 146279e**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Rank Xerox (UK) Business Services
2.14.12/3.4

## Description

This invention relates to new ethanolamine derivatives and pharmaceutically acceptable salts thereof which are useful as a medicament.

BACKGROUND ART

Some ethanolamine derivatives having spasmolytic activity and relaxing activity on smooth muscle contraction have been known as described, for example, in European Patent Application Publication Nos. 0 255 415, 0 303 546 and 0 383 686. However, it has not been known that they have an anti-pollakisuria activity.

DISCLOSURE OF INVENTION

This invention relates to new ethanolamine derivatives and pharmaceutically acceptable salts thereof.

More particularly, it relates to new ethanolamine derivatives and pharmaceutically acceptable salts thereof which have got selective sympathomimetic and anti-pollakisuria activities, to processes for the preparation thereof, to a pharmaceutical composition comprising the same and to a method of using the same therapeutically in the treatment and/or prevention of gastro-intestinal disorders caused by smooth muscle contractions in human beings or animals, and more particularly to a method for the treatment and/or prevention of spasm or hyperanakinesia in case of irritable bowel syndrome, gastritis, gastric ulcer, duodenal ulcer, enteritis, cholecystopathy, cholangitis, urinary calculus and the like; and for the treatment and/or prevention of dysuria such as pollakisuria, urinary incontinence or the like in case of nervous pollakisuria, neurogenic bladder dysfunction, nocturia, unstable bladder, cystospasm, chronic cystitis, chronic prostatitis or the like. Additionally, the object compound is expected to be useful as therapeutical and/or preventive agents for obesity and glaucoma.

One object of this invention is to provide new and useful ethanolamine derivatives and pharmaceutically acceptable salts thereof which have gut selective sympathomimetic and anti-pollakisuria activities.

Another object of this invention is to provide processes for the preparation of said ethanolamine derivatives and salts thereof.

A further object of this invention is to provide a pharmaceutical composition comprising, as an active ingredient, said ethanolamine derivatives and pharmaceutically acceptable salts thereof.

The object ethanolamine derivatives of this invention are new and can be represented by the following general formula [I]:

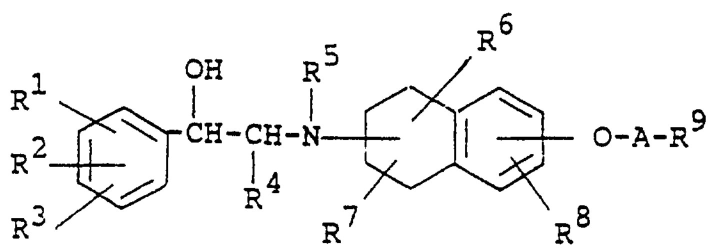

[I]

wherein

| | |
|---|---|
| $R^1$ is | hydrogen or halogen, |
| $R^2$ is | halogen, hydroxy, protected hydroxy, aryloxy, lower alkoxy, halo(lower)alkoxy, nitro, cyano, amino or acylamino, |
| $R^3$ is | hydrogen or halogen, |
| $R^4$ is | hydrogen or lower alkyl, |
| $R^5$ is | hydrogen or lower alkyl, |
| $R^6$ is | hydrogen, hydroxy or lower alkyl, |
| $R^7$ is | hydrogen or lower alkyl, |
| $R^8$ is | hydrogen or halogen, |
| $R^9$ is | carboxy or esterified carboxy, and |
| A is | lower alkylene, |

provided that $R^4$ is lower alkyl; $R^5$ is lower alkyl; $R^6$ is hydroxy or lower alkyl; or $R^8$ is halogen when $R^1$ is hydrogen, $R^2$ is halogen and $R^3$ is hydrogen,
and pharmaceutically acceptable salts thereof.

The above proviso excludes the prior art compounds of formula I, see: EP-A-383 686; EP-A-403 360; EP-A-303 546; EP-A-211 721; Chem. Abstracts, 111, n°17, 23-10-89, abstract n°146279e, & L. Manara et al. Fidia Res. Found. Symp. Ser. 1989, 2, 131-47.

The object compound [I] or its salt can be prepared by the following processes.

## Process 1

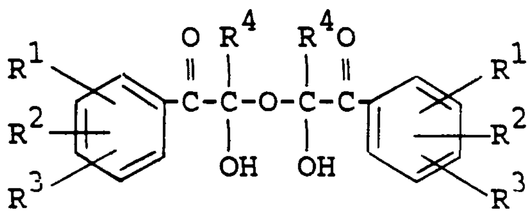

+

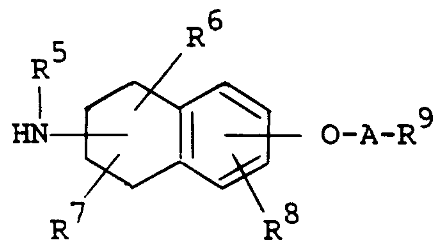

[II]
or its salt

[III]
or its salt

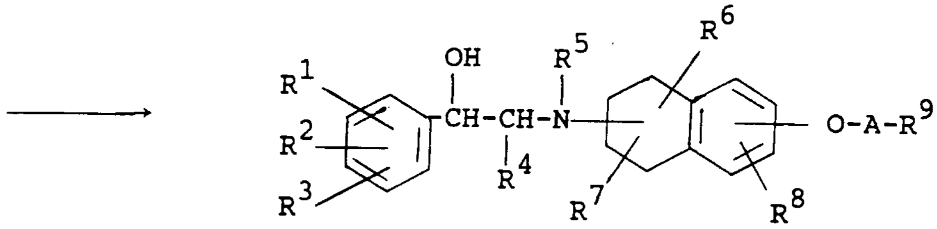

[I]
or its salt

Process 2

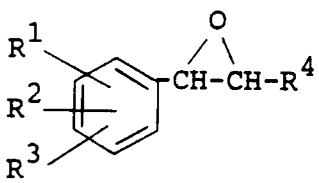

+

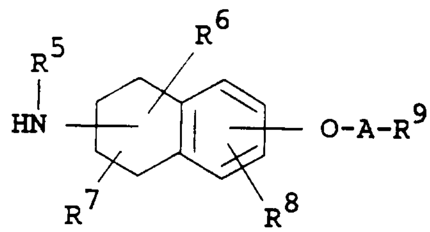

[IV]
or its salt

[III]
or its salt

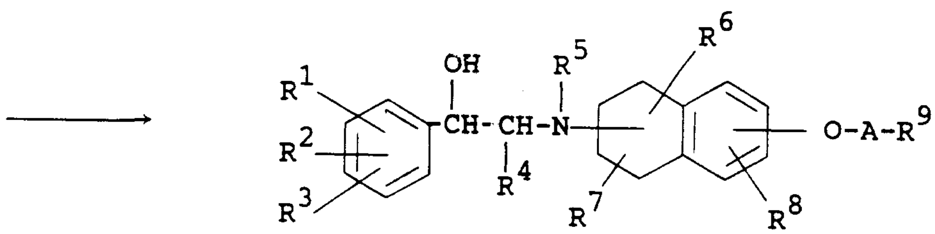

[I]
or its salt

Process 3

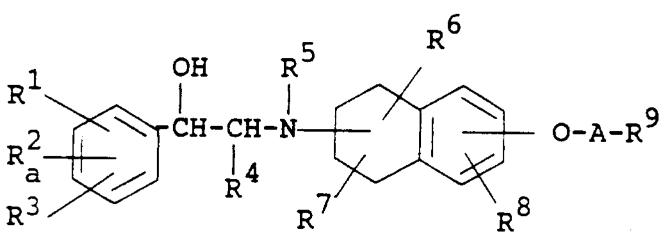

[Ia]
or its salt

elimination of the hydroxy-protective group

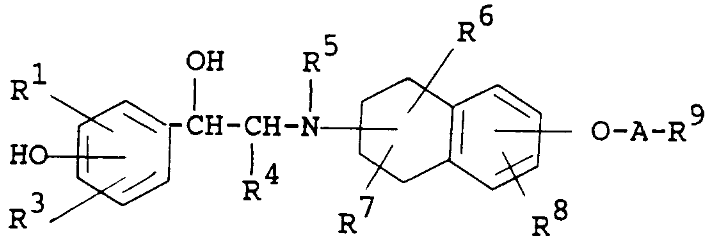

[Ib]
or its salt

Process 4

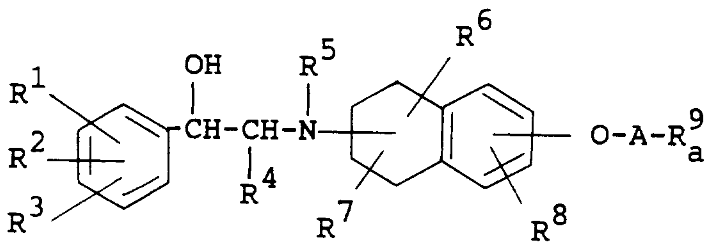

[Ic]
or its salt

deesterification

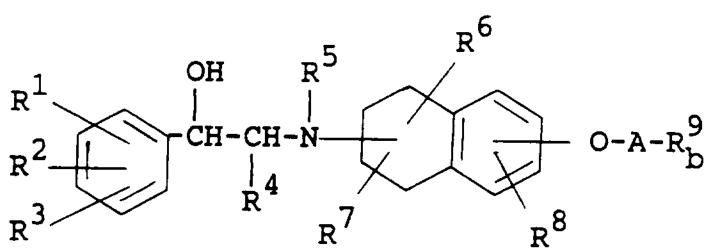

[Id]
or its salt

Process 5

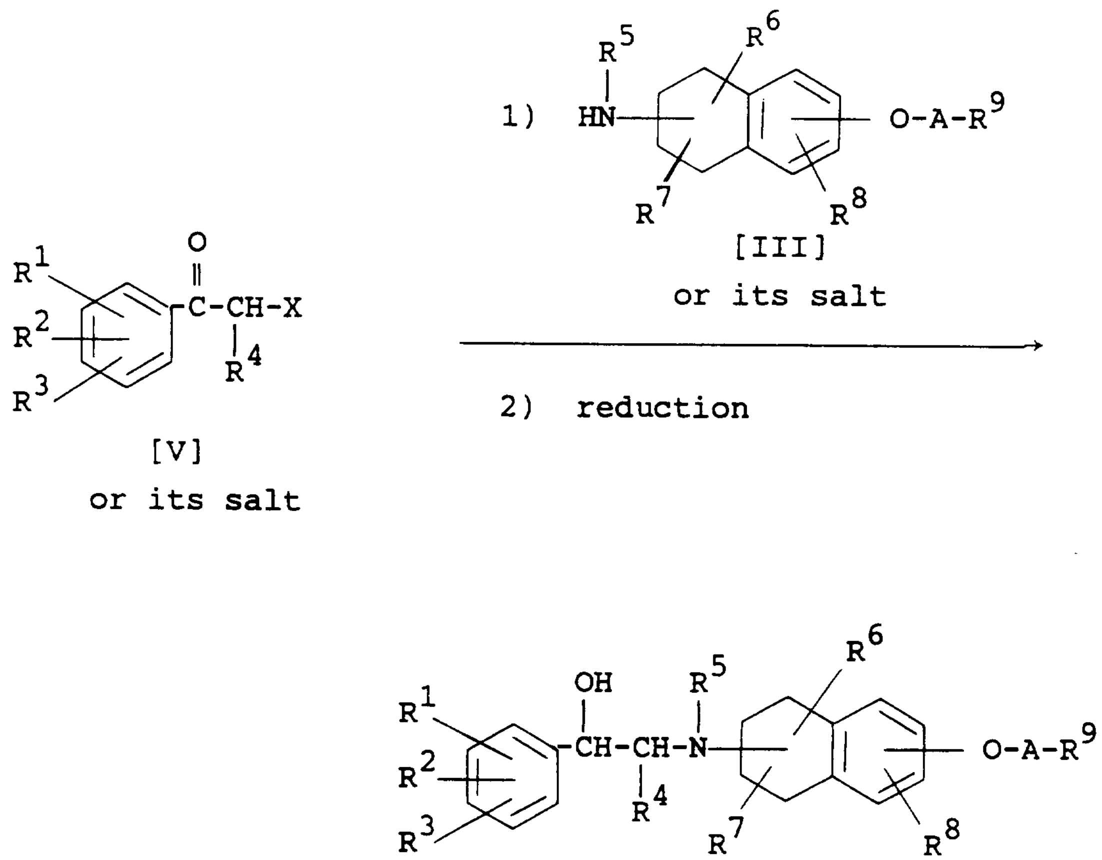


[V]
or its salt

[III]
or its salt

[I]
or its salt

wherein

$R^1$, $R^2$, $R^3$, $R^4$, $R^5$, $R^6$, $R^7$, $R^8$, $R^9$ and A are each as defined above,
$R_a^2$ is protected hydroxy,
$R_a^9$ is esterified carboxy,
$R_b^9$ is carboxy, and
X is halogen.

In the above and subsequent description of the present specification, suitable examples of the various definition to be included within the scope of the invention are explained in detail in the following.

The term "lower" is intended to mean a group having 1 to 6 carbon atom(s), unless otherwise provided.

Suitable "halogen" may be fluorine, chlorine, bromine, and iodine, in which preferable one is chlorine.

Suitable "lower alkylene" may be straight or branched one such as methylene, ethylene, trimethylene, propylene, pentamethylene, hexamethylene, ethylethylene or the like, in which preferable one is methylene.

Suitable "protected hydroxy" may be substituted lower alkoxy such as lower alkoxy(lower)alkoxy [e.g. methoxymethoxy, etc.], lower alkoxy(lower)alkoxy(lower)alkoxy [e.g. methoxyethoxymethoxy, etc.], substituted or unsubstituted ar(lower)alkoxy [e.g. benzyloxy, nitrobenzyloxy, etc.], etc., acyloxy such as lower alkanoyloxy [e.g. acetoxy, propionyloxy, pivaloyloxy, etc.], aroyloxy [e.g. benzoyloxy, fluorenecarbonyloxy, etc.], lower alkoxycarbonyloxy [e.g. methoxycarbonyloxy, ethoxycarbonyloxy, propoxycarbonyloxy, isopropoxycarbonyloxy, butoxycarbonyloxy, isobutoxycarbonyloxy, tert-butoxycarbonyloxy, pentyloxycarbonyloxy, hexyloxycarbonyloxy, etc.], substituted or unsubstituted

ar(lower)alkoxycarbonyloxy [e.g. benzyloxycarbonyloxy, bromobenzyloxycarbonyloxy, etc.] etc., tri(lower)alkylsilyloxy [e.g. trimethylsilyloxy, etc.] and the like.

The esterified carboxy may be substituted or unsubstituted lower alkoxycarbonyl [e.g. methoxycarbonyl, ethoxycarbonyl, propoxycarbonyl, butoxycarbonyl, hexyloxycarbonyl, 2-iodoethoxycarbonyl, 2,2,2-trichloroethoxycarbonyl, etc.], substituted or unsubstituted aryloxycarbonyl [e.g. phenoxycarbonyl, 4-nitrophenoxycarbonyl, 2-naphthyloxycarbonyl, etc.], substituted or unsubstituted ar(lower)alkoxycarbonyl [e.g. benzyloxycarbonyl, phenethyloxycarbonyl, benzhydryloxycarbonyl, 4-nitrobenzyloxycarbonyl, etc.] and the like, in which preferable one is lower alkoxycarbonyl.

Suitable "lower alkyl" and lower alkyl moiety in the term "lower alkylsulfonyl" may be a straight or branched one such as methyl, ethyl, propyl, isopropyl, butyl, isobutyl, tert-butyl, pentyl, isopentyl, hexyl or the like, in which preferable one is methyl.

Suitable "lower alkoxy" and lower alkoxy moiety in the term "halo(lower)alkoxy" may be methoxy, ethoxy, propoxy, isopropoxy, butoxy, isobutoxy, tert-butoxy and the like, in which preferable one is methoxy.

Suitable acyl moiety in the term "acylamino" may be carboxy, esterified-carboxy, lower alkanoyl, carbamoyl optionally substituted with lower alkyl, lower alkylsulfonyl and the like, in which preferable one is lower alkylsulfonyl.

Suitable "lower alkylsulfonyl" may be methylsulfonyl, ethylsulfonyl, propylsulfonyl and the like, in which preferable one is methylsulfonyl.

Suitable "lower alkanoyl" may be formyl, acetyl, propionyl, butyryl, isobutyryl, valeryl, isovaleryl, pivaloyl, hexanoyl and the like.

Suitable "halo(lower)alkoxy" may be chloromethoxy, fluoromethoxy, bromomethoxy, difluoromethoxy, dichloromethoxy, trifluoromethoxy, trichloromethoxy, 2-fluoroethoxy and the like, in which preferable one is trifluoromethoxy.

Suitable "aryloxy" may be phenoxy, tolyloxy, xylyloxy, naphthyloxy and the like, in which preferable one is phenoxy.

Suitable "N-protective group" may be substituted or unsubstituted lower alkanoyl [e.g. formyl, acetyl, propionyl, trifluoroacetyl, etc.], phthaloyl, lower alkoxycarbonyl [e.g. tert-butoxycarbonyl, tert-amyloxycarbonyl, etc.], substituted or unsubstituted aralkyloxycarbonyl [e.g. benzyloxycarbonyl, p-nitrobenzyloxycarbonyl, etc.], substituted or unsubstituted arenesulfonyl [e.g. benzenesulfonyl, tosyl, etc.], nitrophenylsulfenyl, aralkyl [e.g. trityl, benzyl, etc.] or the like.

Suitable "acid residue" may be halogen [e.g. fluro, chloro, bromo, iodo], arenesulfonyloxy [e.g. benzenesulfonyloxy, tosyloxy, etc.], alkanesulfonyloxy [e.g. mesyloxy, ethanesulfonyloxy, etc.], and the like, in which preferable one is halogen.

Preferable compound [I] is one which has halogen for $R^1$ and hydroxy or protected hydroxy for $R^2$, or hydrogen for $R^1$ and halogen for $R^2$; hydrogen for $R^3$; hydrogen for $R^4$; hydrogen for $R^5$; hydrogen or hydroxy for $R^6$; hydrogen for $R^7$; hydrogen for $R^8$; esterified carboxy for $R^9$; and lower alkylene for A provided that $R^6$ is hydroxy when $R^1$ is hydrogen and $R^2$ is halogen.

Suitable pharmaceutically acceptable salts of the object compound [I] are conventional non-toxic salts such as an inorganic acid addition salt [e.g. hydrochloride, hydrobromide, sulfate, phosphate, etc.], an organic acid addition salt [e.g. formate, acetate, trifluoroacetate, oxalate, maleate, fumarate, tartrate, methanesulfonate, benzenesulfonate, toluenesulfonate, etc.], an alkali metal salt [e.g. sodium salt, potassium salt, etc.] or the like.

The processes for preparing the object compound [I] are explained in detail in the following.

Process 1

The compound [I] or its salt can be prepared by reacting a compound [II] or its salt with a compound [III] or its salt in the present of a reducing agent.

Suitable salts of the compounds [II] and [III] may be the same as those exemplified for the compound [I].

Suitable reducing agent may be borohydride compound such as alkali metal borohydride [e.g. sodium borohydride, sodium cyanoborohydride, lithium cyanoborohydride, lithium triethylborohydride, etc.], tetrabutylammonium cyanoborohydride or the like, in which preferable one is alkali metal borohydride.

The reaction is preferably carried out in the presence of a base such as an alkali metal carbonate [e.g. sodium carbonate, potassium carbonate, etc.], an alkaline earth metal carbonate [e.g. magnesium carbonate, calcium carbonate, etc.], an alkali metal bicarbonate [e.g. sodium bicarbonate, potassium bicarbonate, etc.], tri(lower)alkylamine [e.g. trimethylamine, triethylamine, etc.], picoline or the like.

The reaction is usually carried out in a conventional solvent, such as water, an alcohol [e.g. methanol, ethanol, propanol, isopropanol, etc.], dioxane, or any other organic solvent which does not adversely influence the reaction, or a mixture thereof.

The reaction temperature is not critical, and the reaction can be carried out under cooling to heating.

Process 2

The compound [I] or its salt can be prepared by reacting a compound [IV] or its salt with a compound [III] or its salt.

Suitable salt of the compound [IV] may be an alkali metal salt as exemplified for the compound [I].

The reaction is preferably carried out in the presence of a base. Suitable base may be referred to those as explained in Process 1.

The reaction is usually carried out in a conventional solvent, such as an alcohol [e.g. methanol, ethanol, propanol, isopropanol, etc.], diethyl ether, tetrahydrofuran, dioxane, or any other organic solvent which does not adversely influence the reaction.

The reaction temperature is not critical, and the reaction can be carried out under cooling to heating.

Process 3

The compound [Ib] or its salt can be prepared by subjecting a compound [Ia] or its salt to elimination reaction of the hydroxy-protective group.

This reaction is carried out in accordance with a conventional method such as hydrolysis, reduction or the like.

The hydrolysis is preferably carried out in the presence of a base or an acid including Lewis acid.

Suitable base may include an inorganic base and an organic base such as an alkali metal [e.g. sodium, potassium, etc.], an alkaline earth metal [e.g. magnesium, calcium, etc.], the hydroxide or carbonate or bicarbonate thereof, trialkylamine [e.g. trimethylamine, triethylamine, etc.], picoline I,5-diazabicyclo[4.3.0]non-5-ene, I,4-diazabicyclo[2.2.2]octane, I,8-diazabicyclo[5.4.0]undec-7-ene, or the like.

Suitable acid may include an organic acid [e.g. formic acid, acetic acid, propionic acid, trichloroacetic acid, trifluoroacetic acid, methanesulfonic acid, p-toluenesulfonic acid, etc.] and an inorganic acid [e.g. hydrochloric acid, hydrobromic acid, sulfuric acid, hydrogen chloride, hydrogen bromide, hydrogen fluoride, etc.].

The reaction is usually carried out in a solvent such as water, an alcohol [e.g. methanol, ethanol, etc.], methylene chloride, chloroform, tetrachloromethane, tetrahydrofuran, a mixture thereof or any other solvent which does not adversely influence the reaction A liquid base or acid can be also used as the solvent. The reaction temperature is not critical and the reaction is usually carried out under cooling to heating.

The reduction method applicable for the elimination reaction may include chemical reduction and catalytic reduction.

Suitable reducing agents to be used in chemical reduction are a combination of metal [e.g. tin, zinc, iron, etc.] or metallic compound [e.g. chromium chloride, chromium acetate, etc.] and an organic or inorganic acid [e.g. formic acid, acetic acid, propionic acid, trifluoroacetic acid, p-toluenesulfonic acid, hydrochloric acid, hydrobromic acid, etc.].

Suitable catalysts to be used in catalytic reduction are conventional ones such as platinum catalysts [e.g. platinum plate, spongy platinum, platinum black, colloidal platinum, platinum oxide, platinum wire, etc.], palladium catalysts [e.g. spongy palladium, palladium black, palladium oxide, palladium on carbon, colloidal palladium, palladium on barium sulfate, palladium on barium carbonate, etc.], nickel catalysts [e.g. reduced nickel, nickel oxide, Raney nickel, etc.], cobalt catalysts [e.g. reduced cobalt, Raney cobalt, etc.], iron catalysts [e.g. reduced iron, Raney iron, etc.], copper catalysts [e.g. reduced copper, Raney copper, Ullman copper, etc.] and the like.

In this reaction, in case that the hydroxy-protective group is benzyl, the reduction can be also carried out in the presence of a combination of palladium catalyst [e.g. palladium black, palladium on carbon, etc.] and formic acid or its salt [e.g. ammonium formate, etc.].

The reduction is usually carried out in a conventional solvent which does not adversely influence the reaction such as water, methanol, ethanol, propanol, N,N-dimethylformamide, or a mixture thereof. Additionally, in case that the above-mentioned acids to be used in chemical reduction are in liquid, they can also be used as a solvent. Further, a suitable solvent to be used in catalytic reduction may be the above-mentioned solvent, and other conventional solvent such as diethyl ether, dioxane, tetrahydrofuran, etc., or a mixture thereof.

The reaction temperature of this reduction is not critical and the reaction is usually carried out under cooling to heating.

In this reaction, in case that the compound [Ia] having halogen for $R^1$ and/or halogen for $R^3$ and/or esterified carboxy for $R^9$ is used as a starting compound, the compound [Ib] having hydrogen for $R^1$ and/or hydrogen for $R^3$ and/or carboxy for $R^9$ may be obtained according to reaction conditions. This case is included within the scope of the present reaction.

Process 4

The compound [Id] or its salt can be prepared by subjecting a compound [Ic] or its salt to deesterification reaction.

This reaction can be carried out in substantially the same manner as Process 3, and therefore the reaction mode and the reaction condition [e.g. solvent, reaction temperature, etc.] of this reaction are to be referred to those as explained in Process 3.

In this reaction, in case that the compound [Ic] having protected hydroxy for $R^2$ is used as a starting compound, the compound [Id] having hydroxy for $R^2$ may be obtained according to reaction conditions. This case is included within the scope of the present reaction.

Process 5

The compound [I] or its salt can be prepared by the following method. Namely, 1) the compound [V] or its salt is firstly reacted with a compound [III] or its salt, and then 2) subjecting the resultant product to reduction.

Suitable salt of the compound [V] may be the same as those exemplified for the compound [I].

In the first step, the reaction is preferably carried out in the presence of a base such as an alkali metal carbonate [e.g. sodium carbonate, potassium carbonate, etc.], an alkaline earth metal carbonate [e.g. magnesium carbonate, calcium carbonate, etc.], an alkali metal bicarbonate [e.g. sodium bicarbonate, potassium bicarbonate, etc.], tri(lower)alkylamine [e.g. trimethylamine, triethylamine, etc.], picoline, pyridine or the like.

The reaction is usually carried out in a conventional solvent such as water, an alcohol [e.g. methanol, ethanol, propanol, isopropanol, etc.], dioxane or any other organic solvent which does not adversely influence the reaction, or a mixture thereof.

The reaction temperature is not critical, and the reaction can be carried out under cooling to heating.

In the second step, suitable reducing agent to be used in this reduction may be the same as those exemplified in Process 1.

The reaction is usually carried out in a conventional solvent, such as water, an alcohol [e.g. methanol, ethanol, propanol, isopropanol, etc.], dioxane, tetrahydrofuran or any other organic solvent which does not adversely influence the reaction, or a mixture thereof.

The reaction temperature is not critical, and the reaction can be carried out under cooling to heating.

The starting compounds [II], [III], [IV] and [V] or salts thereof can be prepared by the following processes.

Process A

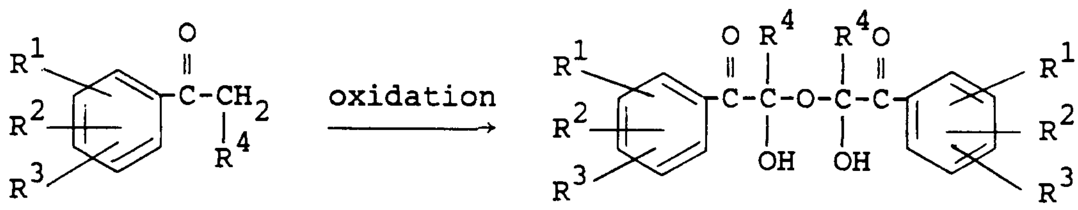


[VI] or its salt

[II] or its salt

Process B

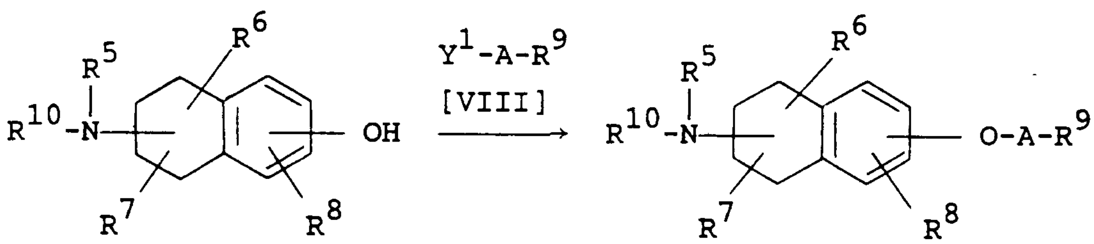


[VII] or its salt

[IX] or its salt

Process C

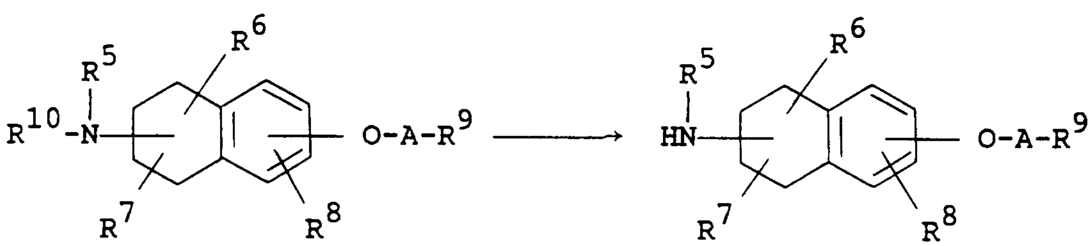

[IX] or its salt

[III] or its salt

Process D

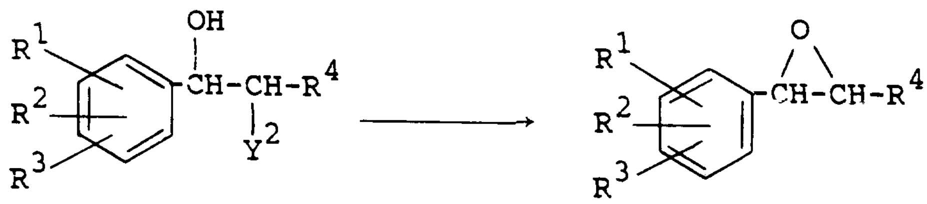

[X] or its salt

[IV] or its salt

Process E

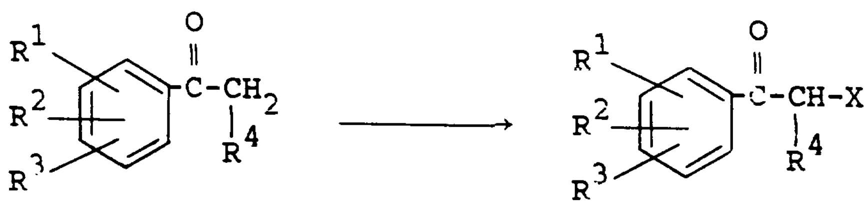

[VI] or its salt

[V] or its salt

wherein

$R^1$, $R^2$, $R^3$, $R^4$, $R^5$, $R^6$, $R^7$, $R^8$, $R^9$, A and X are each as defined above,
$R^{10}$ is an N-protective group,
$Y^1$ is acid residue, and
$Y^2$ is acid residue.

The above-mentioned processes for preparing the starting compounds [II], [III], [IV] and [V] are explained in detail in the following.

Process A

The compound [II] or its salt can be prepared by subjecting a compound [VI] or its salt to oxidation.

Suitable salt of the compound [VI] may be the same as those exemplified for the compound [I].

Suitable oxidizing agent to be used in this oxidation may be selenium dioxide and the like.

The reaction is usually carried out in a conventional solvent such as water, dioxane, acetic anhydride or any other organic solvent which does not adversely influence the reaction, or a mixture thereof.

The reaction temperature is not critical, and the reaction is usually carried out under heating.

Process B

The compound [IX] or its salt can be prepared by reacting a compound [VII] or its salt with a compound [VIII].

Suitable salts of the compounds [VII] and [IX] may be the same as those exemplified for the compound [I].

When the compound [VIII] having halogen for $Y^1$ is used in this reaction, the reaction is preferably carried out in the presence of a base such as alkali metal [e.g. lithium, sodium, potassium, etc.], the hydroxide or carbonate or bicarbonate thereof [e.g. sodium hydroxide, potassium carbonate, potassium bicarbonate, etc.], alkaline earth metal [e.g. calcium, magnesium, etc.], alkali metal hydride [e.g. sodium hydride, etc.], alkaline earth metal hydride [e.g. calcium hydride, etc.], alkali metal alkoxide [e.g. sodium methoxide, sodium ethoxide, potassium tert-butoxide, etc.], alkaline earth metal alkoxide [e.g. magnesium methoxide, magnesium ethoxide, etc.] or the like, or alkali metal iodide [e.g. sodium iodide, potassium iodide, etc.] and the like.

Additionally, the reaction is also preferably carried out in the presence of phase transfer catalyst [e.g. tetra-n-butylammonium bromide, etc.].

This reaction is usually carried out in a conventional solvent such as tetrahydrofuran, dioxane, aromatic hydrocarbon [e.g. benzene, toluene, xylene, etc.], N,N-dimethylformamide, acetone, a mixture thereof, or any other solvent which does not adversely influence the reaction. Additionally, in case that the compound [VIII] is in liquid, it can also be used as a solvent.

The reaction temperature is not critical, and the reaction is usually carried out under cooling to heating.

Process C

The compound [III] or its salt can be prepared by subjecting a compound [IX] or its salt to elimination reaction of the N-protective group.

This reaction is carried out in accordance with a conventional method such as hydrolysis, reduction or the like.

The hydrolysis is preferably carried out in the presence of a base or an acid including Lewis acid.

Suitable base may include an inorganic base and an organic base such as an alkali metal [e.g. sodium, potassium, etc.], an alkaline earth metal [e.g. magnesium, calcium, etc.], the hydroxide or carbonate or bicarbonate thereof, hydrazine, trialkylamine [e.g. trimethylamine, triethylamine, etc.], picoline, 1,5-diazabicyclo[4.3.0]non-5-ene, 1,4-diazabicyclo[2.2.2]octane, 1,8-diazabicyclo[5.4.0]undec-7-ene, or the like.

Suitable acid may include an organic acid [e.g. formic acid, acetic acid, propionic acid, trichloroacetic acid, trifluoroacetic acid, etc.], an inorganic acid [e.g. hydrochloric acid, hydrobromic acid, sulfuric acid, hydrogen chloride, hydrogen bromide, hydrogen fluoride, etc.] and an acid addition salt compound [e.g. pyridine hydrochloride, etc.].

The elimination using trihaloacetic acid [e.g. trichloroacetic acid, trifluoroacetic acid, etc.] or the like is preferably carried out in the presence of cation trapping agents [e.g. anisole, phenol, etc.].

The reaction is usually carried out in a solvent such as water, an alcohol [e.g. methanol, ethanol, etc.], methylene chloride, chloroform, tetrachloromethane, tetrahydrofuran, a mixture thereof or any other solvent which does not adversely influence the reaction. A liquid base or acid can be also used as the solvent. The reaction temperature is not critical and the reaction is usually carried out under cooling to heating.

The reduction method applicable for the elimination reaction may include chemical reduction and catalytic reduction.

Suitable reducing agents to be used in chemical reduction are a combination of metal [e.g. tin, zinc, iron, etc.] or metallic compound [e.g. chromium chloride, chromium acetate, etc.] and an organic or inorganic acid [e.g. formic acid, acetic acid, propionic acid, trifluoroacetic acid, p-toluenesulfonic acid, hydrochloric acid, hydrobromic acid, etc.].

Suitable catalysts to be used in catalytic reduction are conventional ones such as platinum catalysts [e.g. platinum plate, spongy platinum, platinum black, colloidal platinum, platinum oxide, platinum wire, etc.], palladium catalysts [e.g. spongy palladium, palladium black, palladium oxide, palladium on carbon, colloidal palladium, palladium on barium sulfate, palladium on barium carbonate, etc.], nickel catalysts [e.g. reduced nickel, nickel oxide, Raney nickel, etc.], cobalt catalysts [e.g. reduced cobalt, Raney cobalt, etc.], iron catalysts [e.g. reduced iron, Raney iron, etc.], copper catalysts [e.g. reduced copper, Raney copper, Ullman copper, etc.] and the like.

In case that the N-protective group is benzyl, the reduction is preferably carried out in the presence of a combination of palladium catalyst [e.g. palladium black, palladium on carbon, etc.] and formic acid or its salt [e.g. ammonium formate, etc.].

The reduction is usually carried out in a conventional solvent which does not adversely influence the reaction such as water, methanol, ethanol, propanol, N,N-dimethylformamide, or a mixture thereof. Additionally, in case that the above-mentioned acids to be used in chemical reduction are in liquid, they can also be used as a solvent. Further, a suitable solvent to be used in catalytic reduction may be the above-mentioned solvent, and other conventional solvent such as diethyl ether, dioxane, tetrahydrofuran, etc. or a mixture thereof.

The reaction temperature of this reduction is not critical and the reaction is usually carried out under cooling to heating.

Process D

The compound [IV] or its salt can be prepared by reacting a compound [X] or its salt with a base.

Suitable salt of the compound [X] may be the same as those exemplified for the compound [I].

Suitable base may be referred to those as explained in Process B.

The reaction is usually carried out in a conventional solvent such as water, an alcohol [e.g. methanol, ethanol, etc.], diethyl ether, tetrahydrofuran, dioxane, dimethoxyethane or any other organic solvent which does not adversely influence the reaction, or a mixture thereof.

The reaction temperature is not critical, and the reaction can be carried out under cooling to heating.

Process E

The compound [V] or its salt can be prepared by subjecting a compound [VI] or its salt to halogenation reaction.

Suitable salt of the compound [VI] may be the same as those exemplified for the compound [I].

Suitable halogenating agent to be used in this reaction may be chlorine, bromine, N-bromosuccinimide, N-chlorosuccinimide, a combination of trimethylbromosilane and dimethyl sulfoxide, tetrabutylammonium tribromide, hydrogen halide [e.g. hydrogen bromide, hydrogen chloride, etc.] and the like.

The reaction is usually carried out in a conventional solvent such as water, acetic acid, tetrachloromethane, chloroform, dichloromethane, dimethoxyethane, tetrahydrofuran, acetonitrile or any other organic solvent which does not adversely influence the reaction, or a mixture thereof.

The reaction temperature is not critical, and the reaction can be carried out under cooling to heating.

The compounds obtained by the above processes can be isolated and purified by a conventional method such as pulverization, recrystallization, column chromatography, reprecipitation, or the like, and converted to the desired salt in conventional manners, if necessary.

It is to be noted that the compound [I] and the other compounds may include one or more stereoisomers due to asymmetric carbon atoms, and all of such isomers and mixture thereof are included within the scope of this invention.

The object compound [I] and pharmaceutically acceptable salts thereof possess gut selective sympathomimetic and anti-pollakiuria activities, and are useful for the treatment and/or prevention gastrointestinal disorders caused by smooth muscle contractions in human beings or animals, and more particularly for the treatment and/or prevention of spasm or hyperanakinesia in case of irritable bowel syndrome, gastritis, gastric ulcer, duodenal ulcer, enteritis, cholecystopathy, cholangitis, urinary calculus and the like; and for the treatment and/or prevention of dysuria such as pollakisuria, urinary incontinence or the like in case of nervous pollakisuria, neurogenic bladder dysfunction, nocturia, unstable bladder, cystospasm, chronic cystitis, chronic prostatitis or the like. Additionally, the object compound is expected to be useful as therapeutical and/or preventive agents for obesity and glaucoma.

In order to illustrate the usefulness of the object compound [I], the pharmacological data of the compound [I] are shown in the following.

Test 1

Effect on isolated rat distal colon :

(i) Test Method :

Male SD rats (l80~230 g) were used. Animals were fasted for 24 hours prior to experiment. Distal colon was removed immediately after sacrifice and placed in an organ bath containing 25 ml Tyrode solution aerating with 95% $O_2$, 5% $CO_2$ at 37°C. The strip was mounted under 0.5 g tension and spontaneous contractions were recorded isometrically. After the motility was of a uniform size, test compound was added to an organ bath and the contractions were observed over a 30 minutes period. Effect of test compound was calculated by comparing contractions before and after test compound.

(ii) Test Results :

| Test Compound (Example No.) | $IC_{50}$ (M) |
|---|---|
| 1 | $8.0 \times 10^{-10}$ |
| 2 | $1.9 \times 10^{-9}$ |

Test 2

Effect on isolated non-pregnant rat uterus :

(i) Test Method :

Female SD rats (l50~l80 g) were used. 48 and 24 hours prior to use, rats were given estradiol (ovahormon benzoat : Trademark, Teikoku Hormone Mfg. Co., Ltd.) subcutaneously at a dose of 40 µg/rat to induce oestrus. The animals were killed and uterine horns were removed. Each strip was placed in an organ bath containing 25 ml Locke solution aerating with 95% $O_2$, 5% $CO_2$ at 37°C under l g tension. Contractions were recorded isometrically. After the spontaneous contractions were of a uniform size, test compound was added to organ bath. The motility was observed over a 20 minutes period. Effect of test compound was calculated by comparing contractions before and after test compound.

(ii) Test Results :

| Test Compound (Example No.) | $IC_{50}$ (M) |
|---|---|
| 1 | $3.2 \times 10^{-7}$ |
| 2 | $5.1 \times 10^{-7}$ |

Test 3

Effect on cystometrogram

(i) Test Method :

Male S.D. rats, aged 7-8 weeks, were anesthetized with ether and placed supine on a board. After a midline incision in the abdomen, the bladder was exposed. Polyethylene catheters, which were used to measure the intravesical pressure and to infuse xylene and saline, were inserted into the bladder through a small incision at the apex of the bladder dome. The rats were placed in a restraining cage and were allowed to recover from anesthesia for about 3 hours. The bladder catheters were connected to an infusion pump and a pressure transducer respectively. 30% xylene (silicone oil solution) was infused at a rate of 3.3 ml/hr for 1 hour and then saline was infused at same rate.

Cystometrography was performed at 30 minutes intervals for 2 hours. The bladder capacity (volume) was calculated from the time required to fill the bladder.

Test compound was administered intravenously via femoral artery.

(ii) Test Results : (relative % of control)

| Group / Time (minutes) | Normal | Vehicle | Test Compound Example 4 Dose: 1.0mg/kg |
|---|---|---|---|
| Before | 100 | 100 | 100 |
| 0-30 | 99.3 | 99.8 | 153.4 |
| 30-60 | 98.1 | 106.5 | 143.0 |
| 60-90 | 98.7 | 104.6 | 148.9 |
| 90-120 | 95.3 | 111.5 | 156.9 |

For therapeutic purpose, the compound [I] and a pharmaceutically acceptable salt thereof of the present invention can be used in a form of pharmaceutical preparation containing one of said compounds, as an active ingredient, in admixture with a pharmaceutically acceptable carrier such as an organic or inorganic solid, semi-solid or liquid excipient suitable for oral, parenteral or external (topical) administration. The pharmaceutical preparations may be capsules, tablets, dragees, granules, suppositories, solutions, suspension, emulsion, ointment, gel, ophthalmic solutions, and the like. If desired, there may be included in these preparations, auxiliary substances, stabilizing agents, wetting or emulsifying agents, buffers and other commonly used additives.

While the dosage of the compound [I] will vary depending upon the age and condition of the patient, an average single dose of about 0.l mg, l mg, l0 mg, 50 mg, l00 mg, 250 mg, 500 mg and l000 mg of the compound [I] may be effective for treating the above-mentioned diseases. In general, amounts between 0.l mg/body and about l,000 mg/body may be administered per day.

The following Preparations and Examples are given for the purpose of illustrating this invention.

Preparation 1

A mixture of 3'-chloro-4'-hydroxyacetophenone (52l mg) and selenium dioxide (444 mg) in dioxane (20 ml) and water (0.08 ml) was stirred under reflux for 6 hours and filtered. The filtrate was concentrated in vacuo and extracted with diethyl ether. The extract was washed twice with brine, dried over magnesium sulfate, and concentrated in vacuo. The residue was crystallized from diisopropyl ether to give colorless crystals of 2,2"-oxybis[3'-chloro-2,4'-dihydroxyacetophenone] (334 mg).

mp : l37-l40°C
IR (Nujol) : 3360, 3180, 3100, 1685, l680 $cm^{-1}$
NMR ($DMSO-d_6$, δ) : 5.87 (2H, d, J=9Hz), 7.04 (2H, d, J=8.5Hz), 7.45 (2H, d, J=9Hz), 7.90 (2H, dd, J=2 and 8.5Hz), 8.04 (2H, d, J=2Hz), 11.32 (2H, br s)
MASS (m/z) : 369 ($M^+$-OH), l55

Preparation 2

To a solution of trans-2-amino-l,2,3,4-tetrahydronaphthalene-1,7-diol (0.40 g), triethylamine (0.68 ml), in N,N-dimethylformamide (8 ml) was added di-tert-butyl dicarbonate (0.54 g). After stirring for 4 hours at ambient temperature, the reaction mixture was poured into water and extracted three times with ethyl acetate. The combined organic layers were washed with water and brine, dried over anhydrous magnesium sulfate, and concentrated in vacuo. A mixture of chloroform and methanol (20:l) was added to the residue, and the resulting precipitate was collected and dried to afford trans-2-(tert-butoxycarbonylamino)-1,2,3,4-tetrahydronaphthalen-1,7-diol (0.38 g). Further product was obtained (0.26 g) from the filtrate after column chromatography on silica gel (eluent:chloroform-methanol = 20:1).

mp : 182-184°C

IR (Nujol) : 3370, 3280, 1660 $cm^{-1}$

NMR (DMSO-$d_6$, δ) : 1.39 (9H, s), 1.4-1.8 (1H, m), 1.8-2.0 (1H, m), 2.5-2.7 (2H, m), 3.3-3.6 (1H, m), 4.29 (1H, t-like, J=ca. 8Hz), 5.18 (1H, d, J=8Hz), 6.54 (1H, dd, J=2 and 8Hz), 6.6-6.8 (1H, m), 6.82 (1H, d, J=2Hz), 6.85 (1H, s), 9.06 (1H, s)

MASS (m/z) : 223 (($M^+$+1)-57)

Preparation 3

To an ice-cooled mixture of trans-2-(tert-butoxycarbonylamino)-l,2,3,4-tetrahydronaphthalen-l,7-diol (0.38 g), potassium carbonate (0.28 g), and acetone (l.4 ml) was added ethyl bromoacetate (0.23 ml). After stirring for 20 hours at ambient temperature, the reaction mixture was diluted with ethyl acetate. The insoluble precipitate which formed was filtered off and washed with ethyl acetate. Removal of the solvent and purification of the residue by column chromatography on silica gel (eluent:chloroform-ethyl acetate = l0:l) afforded trans-2-(tert-butoxycarbonylamino)-7-ethoxycarbonylmethoxy-1,2,3,4-tetrahydro-1-naphthol (0.44 g).

mp : l29-l30°C

IR (Nujol) : 3500, 3360, l7l5, l670 $cm^{-1}$

NMR (DMSO-$d_6$, δ) : 1.21 (3H, t, J=7Hz), 1.40 (9H, s), 1.4-1.8 (1H, m), 1.8-2.0 (1H, m), 2.6-2.8 (2H, m), 3.3-3.6 (1H, m), 4.15 (2H, q, J=7Hz), 4.33 (1H, t-like, J=ca. 8Hz), 4.70 (2H, s), 5.30 (1H, d, J=7Hz), 6.6-6.8 (2H, m), 6.9-7.0 (2H, m)

MASS (m/z) : 309 (($M^+$+1)-57)

Preparation 4

To a solution of trans-2-(tert-butoxycarbonylamino)-7-ethoxycarbonylmethoxy-l,2,3,4-tetrahydro-l-naphthol (0.26 g) in ethanol (7.0 ml) was added 5N hydrogen chloride in ethanol (3.5 ml). After stirring for 3 hours at ambient temperature, the reaction mixture was concentrated in vacuo. Toluene was added to the residue, and the mixture was concentrated to afford trans-2-amino-7-ethoxycarbonylmethoxy-1,2,3,4-tetrahydro-l-naphthol hydrochloride (0.21 g).

mp : l35-l37°C

IR (Nujol) : 3250, 3l50, l730 $cm^{-1}$

NMR (DMSO-$d_6$, δ) : l.22 (3H, t, J=7Hz), l.7-2.0 (1H, m), 2.0-2.2 (1H, m), 2.7-2.9 (2H, m), 3.0-3.2 (1H, m), 4.l6 (2H, q, J=7Hz), 4.59 (1H, t-like, J=ca. 8Hz), 4.73 (2H, s), 6.l6 (1H, d, J=7Hz), 6.78 (1H, dd, J=2 and 8Hz), 6.9-7.l (2H, m), 8.32 (3H, br s)

MASS (m/z) : 265

Preparation 5

A mixture of 2,3'-dichloro-4'-hydroxyacetophenone (3.77 g), 2-methoxyethoxymethyl chloride (3.44 g), and potassium carbonate (2.80 g) in N,N-dimethylformamide (38 ml) was stirred at ambient temperature for 3.5 hours, then diluted with water, and extracted with ethyl acetate. The extract was washed twice with brine, dried over magnesium sulfate, and concentrated in vacuo. The residue was purified by column chromatography (silica gel) using a mixture of toluene and ethyl acetate to give an oil of 2,3'-dichloro-4'-(2-methoxyethoxymethoxy)acetophenone (4.03 g).

IR (Film) : l695, l685, l245, l200, ll65, ll35, ll05 $cm^{-1}$

NMR ($CDCl_3$, δ) : 3.37 (3H, s), 3.56 (2H, m), 3.88 (2H, m), 4.63 (2H, s), 5.44 (2H, s), 7.3l (1H, d, J=8.5Hz), 7.84 (1H, dd, J=2 and 8.5Hz), 8.02 (1H, d, J=2Hz)

Preparation 6

A solution of 2,3'-dichloro-4'-(2-methoxyethoxymethoxy)acetophenone (4.40 g) in tetrahydrofuran (l2 ml) and a solution of borane in tetrahydrofuran (l.0M, 9.0 ml) were added simultaneously to a mixture of a solution of (R)-tetrahydro-3,3-diphenyl-1H,3H-pyrrolo[l,2-c][l,3,2]oxazaborole in tetrahydrofuran (ca. 0.5M, l.20 ml) and a solution of borane in tetrahydrofuran (l.0M, l.50 ml) at l5-25°C under nitrogen over 30 minutes. The resulting mixture was stirred at the same temperature for 2 hours, and then methanol (3.6 ml) was added dropwise to the mixture under ice cooling. The mixture was stirred at ambient temperature and concentrated in vacuo. The residue was partitioned between diethyl ether and 0.5N hydrochloric acid. The organic layer was separated, washed with brine, sodium bicarbonate aqueous solution, and brine, dried over magnesium sulfate, and concentrated in vacuo. The residue was purified by column chromatography on silica gel using toluene and ethyl acetate to give an oil containing (-)-l-[3-chloro-4-(2-methoxyethoxymethoxy)phenyl]-2-chloroethanol (4.l8 g); $[\alpha]_D^{l9.6}$ = -26.46° (C=0.99, $CH_2Cl_2$).

The obtained oil (4.l8 g), isopropenyl acetate (2.84 g), and lipase PS Amano prepared by Amano Pharmaceutical Co., Ltd. (6.44 g) in dry diisopropyl ether (l34 ml) was stirred at ambient temperature for l5 hours and filtered. The filtrate was concentrated in vacuo and purified by column chromatography on silica gel using a mixture of toluene and ethyl acetate to give an oil of (-)-l-[3-chloro-4-(2-methoxyethoxymethoxy)phenyl]-2-chloroethanol (3.62 g).

$[\alpha]_D^{l7.6}$ = -29.90° (C=0.993, $CH_2Cl_2$)
IR (Film) : 3400, l240, ll05, l080 $cm^{-1}$
NMR ($CDCl_3$, δ) : 2.64 (1H, br s), 3.37 (3H, s), 3.54-3.75 (4H, m), 3.85-3.90 (2H, m), 4.84 (1H, quartet, J=3.5 and 8Hz), 5.34 (2H, s), 7.22 (2H, m), 7.43 (1H, d, J=1Hz)

Preparation 7

A mixture of (-)-l-[3-chloro-4-(2-methoxyethoxymethoxy)phenyl]-2-chloroethanol (4.38 g), 1N sodium hydroxide aqueous solution (29.7 ml), and diethyl ether (29.7 ml) was stirred at ambient temperature for 4 hours, saturated with sodium sulfate, and extracted four times with n-hexane. The extracts were combined, dried over magnesium sulfate, and concentrated under reduced pressure to give a colorless oil of (+)-[3-chloro-4-(2-methoxyethoxymethoxy)phenyl]oxirane (3.79 g).

$[\alpha]_D^{18.4}$ = +22.40° (C=l.25, benzene)
IR (Film) : l235, ll05 $cm^{-1}$
NMR ($CDCl_3$, δ) : 2.76 (1H, quartet, J=2.5 and 5.5Hz), 3.l2 (1H, quartet, J=4 and 5.5Hz), 3.37 (3H, s), 3.56 (2H, m), 3.79 (1H, quartet, J=2.5 and 4Hz), 3.87 (2H, m), 5.34 (2H, s), 7.l2 (1H, quartet, J=2 and 8.5Hz), 7.2l (1H, d, J=8.5Hz), 7.28 (1H, d, J=2Hz)
MASS (m/z) : 258 ($M^+$), 89 (base), 59

Preparation 8

The following compounds were obtained according to a similar manner to that of Preparation 1.

1) 2,2''-Oxybis[4'-benzyloxy-3'-chloro-2-hydroxyacetophenone]

mp : 116-123.5°C
IR (Nujol) : 3410, 3330, 1680, 1280 $cm^{-1}$
NMR (DMSO-$d_6$, δ) : 5.33 (4H, s), 5.90 (2H, d, J=9Hz), 7.3-7.55 (12H, m), 7.57 (2H, d, J=9Hz), 8.03 (2H, dd, J=9 and 2Hz), 8.10 (2H, d, J=2Hz)
FAB-MASS (m/z) : 549 ($M^+$-$H_2O$+1)

2) 2,2''-Oxybis[3'-chloro-2-hydroxy-4'-methoxyacetophenone]

mp : 98-104°C
IR (Nujol) : 3370, 1685, 1280 $cm^{-1}$
NMR (DMSO-$d_6$, δ) : 3.95 (6H, s), 5.80 and 5.90 (2H, d, J=9Hz), 7.2-7.3 (2H, m), 7.48 and 7.57 (2H, d, J=9Hz), 7.95-8.1 (4H, m)
FAB-MASS (m/z) : 397 ($M^+$-$H_2O$+l)

3) 2,2''-Oxybis[2-hydroxy-3'-phenoxyacetophenone]

IR (Film) : 3430, 1680, 1300-1200 $cm^{-1}$
NMR ($CDCl_3$, δ) : 4.51 (2H, d, J=10Hz), 6.10 (2H, d, J=10Hz), 7.0-7.9 (18H, m)

4) 2,2''-Oxybis[3'-chloro-2-hydroxy-4'-methylsulfonylaminoacetophenone]
(This compound was used for the next step without purification.)

Preparation 9

The following compound was obtained according to a similar manner to that of Preparation 6.

(-)-1-(4-Benzyloxy-3-chlorophenyl)-2-chloroethanol

mp : 86-88°C
$[\alpha]_D^{29}$ = -28.26° (C=0.895, $CH_2Cl_2$)
IR (Nujol) : 3370, 3300, 1260 $cm^{-1}$
NMR ($CDCl_3$, δ) : 2.36 (1H, br), 3.5-3.75 (2H, m), 4.82 (1H, m), 5.16 (2H, s), 6.95 (1H, d, J=8.5Hz), 7.19 (1H, dd, J=8.5 and 2Hz), 7.25-7.5 (6H, m)

Preparation 10

The following compound was obtained according to a similar manner to that of Preparation 7.

(+)-(4-Benzyloxy-3-chlorophenyl)oxirane

mp : 79-84°C
$[\alpha]_D^{28.8}$ = +19.50° (C=1.02, benzene)
IR (Nujol) : 1265, 1240 $cm^{-1}$
NMR ($CDCl_3$, δ) : 2.76 (1H, dd, J=5.5 and 2.5Hz), 3.11 (1H, dd, J=5.5 and 4Hz), 3.78 (1H, dd, J=4 and 2.5Hz), 5.16 (2H, s), 6.93 (1H, d, J=8.5Hz), 7.10 (1H, dd, J=8.5 and 2Hz), 7.25-7.5 (6H, m)

Preparation 11

A suspension of 3-chloro-4-hydroxyacetophenone (34.12 g), benzylbromide (34.21 g), and potassium carbonate (38.70 g) in N,N-dimethylformamide (171 ml) was stirred at ambient temperature for 5 hours and filtered. The filtrate was evaporated in vacuo and the residue was partitioned between methylene chloride and water. The methylene chloride layer was separated, washed with 5% sodium hydroxide solution and brine, dried over magnesium sulfate, and evaporated in vacuo. The residue was washed with a mixture of n-hexane and toluene to give a pale yellow powder of 4'-benzyloxy-3'-chloroacetophenone (40.23 g).

mp : 112-115°C
IR (Nujol) : 1665, 1270 $cm^{-1}$
NMR ($CDCl_3$, δ) : 2.54 (3H, s), 5.23 (2H, s), 7.00 (1H, d, J=8.5Hz), 7.25-7.5 (5H, m), 7.82 (1H, dd, J=8.5 and 2Hz), 8.01 (1H, d, J=2Hz)
MASS (m/z) : 260 ($M^+$), 91 (base)

Preparation 12

Bromine (27.69 g) was added dropwise to a solution of 4'-benzyloxy-3'-chloroacetophenone (43.02 g) in dimethoxyethane (430 ml) at ambient temperature over 1 hour and the mixture was stirred at the same temperature for 2 hours. After evaporation, the resulting residue was dissolved in methylene chloride, washed with sodium hydrogen carbonate aqueous solution and brine, dried over magnesium sulfate. The solution was evaporated in vacuo and the residue was triturated with a mixture of n-hexane and ethyl acetate to afford 4'-benzyloxy-2-bromo-3'-chloroacetophenone (41.45 g).

mp : 114-118°C
IR (Nujol) : 1675, 1190 $cm^{-1}$
NMR ($CDCl_3$, δ) : 4.36 (2H, s), 5.24 (2H, s), 7.02 (1H, d, J=8.5Hz), 7.25-7.5 (5H, m), 7.85 (1H, dd, J=8.5 and 2Hz), 8.04 (1H, d, J=2Hz)
MASS (m/z) : 339 ($M^+$+1), 338 ($M^+$), 91 (base)

Preparation 13

A solution of 4'-benzyloxy-2-bromo-3'-chloroacetophenone (41.08 g) and tetra-n-butylammonium bromide (3.90 g) in 1,2-dichloroethane (973 ml) was refluxed for 6 hours, evaporated in vacuo, and extracted with dichloromethane. The extract was washed with brine, dried over magnesium sulfate, and evaporated in vacuo. The residue was recrystallized from a mixture of diisopropyl ether and ethyl acetate to afford 4-benzyloxy-2,3'-dichloroacetophenone (31.38 g).

mp : 114-117.5°C
IR (Nujol) : 1685, 1205 $cm^{-1}$
NMR ($CDCl_3$, δ) : 4.62 (2H, s), 5.25 (2H, s), 7.03 (1H, d, J=8.5Hz), 7.3-7.5 (5H, m), 7.83 (1H, dd, J=8.5 and 2Hz),

| | |
|---|---|
| | 8.02 (1H, d, J=2Hz) |
| MASS (m/z) : | 294 ($M^+$), 91 (base) |

Preparation 14

To an ice-cooled solution of potassium t-butoxide (0.64 g) in tetrahydrofuran (5.2 ml) was added a solution of trimethylsulfonium iodide (1.2 g) in dimethyl sulfoxide (5.2 ml). During the addition, the reaction temperature was maintained below 5°C. After the addition was complete, 3-trifluoromethoxybenzaldehyde (1.05 g) was added to the reaction mixture. After 5 minutes stirring, the reaction mixture was poured into water, and extracted once with ethyl acetate. The extract was washed twice with water and once with brine, dried over anhydrous sodium sulfate, and evaporated in vacuo to give 3-trifluoromethoxyphenyloxirane (1.1 g).

This compound was used for the next step without further purification.

| | |
|---|---|
| NMR ($CDCl_3$, δ) : | 2.7-2.8 (1H, m), 3.1-3.2 (1H, m), 3.8-3.9 (1H, m), 7.1-7.5 (4H, m) |
| MASS (m/z) : | 204 |

Preparation 15

A mixture of 7-methoxy-2-methyl-1,2,3,4-tetrahydro-2-naphthalenecarboxylic acid (3.0 g), diphenylphosphoryl azide (2.93 ml) and triethylamine (1.90 ml) in tert-butanol (60 ml) was refluxed for 1.5 hours. After cooling, the mixture was evaporated in vacuo. To the residue were added ethyl acetate and water, and the organic layer was separated, washed with saturated sodium hydrogencarbonate and brine, dried over sodium sulfate, and evaporated in vacuo. The residue was purified by column chromatography on silica gel with a mixture of n-hexane and ethyl acetate (50:1) as an eluent to afford 7-methoxy-2-methyl-1,2,3,4-tetrahydro-2-naphthyl isocyanate (2.33 g) as an oil.

| | |
|---|---|
| IR (Film) : | 2250 $cm^{-1}$ |
| NMR ($CDCl_3$, δ) : | 1.48 (3H, s), 1.68-1.89 (1H, m), 1.91-2.09 (1H, m), 2.65-3.06 (4H, m), 3.77 (3H, s), 6.57 (1H, d, J=2.7Hz), 6.73 (1H, dd, J=2.7 and 8.4Hz), 7.04 (1H, d, J=8.4Hz) |
| MASS (m/z) : | 217 ($M^+$) |

Preparation 16

To a solution of 7-methoxy-2-methyl-1,2,3,4-tetrahydro-2-naphthyl isocyanate (192 mg) in tetrahydrofuran (5 ml) and tert-butanol (0.2 ml) was added sodium hydride (60% dispersion in oil; 42 mg) at 0°C and the mixture was refluxed for 15 minutes. After cooling, brine was added to the mixture. The organic layer was separated, washed with brine, dried over magnesium sulfate, and evaporated in vacuo. The residue was purified by column chromatography on silica gel with a mixture of n-hexane and ethyl acetate (10:1) as an eluent to afford N-tert-butoxycarbonyl-(7-methoxy-2-methyl-1,2,3,4-tetrahydro-2-naphthyl)amine (258 mg) as an oil.

| | |
|---|---|
| IR (Film) : | 3430, 3350, 1700 $cm^{-1}$ |
| NMR ($CDCl_3$, δ) : | 1.42 (9H, s), 1.44 (3H, s), 1.50-1.78 (1H, m), 2.27-2.48 (1H, m), 2.74 (2H, pseudo t, J=6.5Hz), 2.84 (2H, s), 3.77 (3H, s), 4.45 (1H, br s), 6.58 (1H, d, J=2.7Hz), 6.69 (1H, dd, J=2.7 and 8.4Hz), 7.02 (1H, d, J=8.4Hz) |
| MASS (m/z) : | 291 ($M^+$) |

Preparation 17

A suspension of N-tert-butoxycarbonyl-(7-methoxy-2-methyl-1,2,3,4-tetrahydro-2-naphthyl)amine (2.0 g) in 47% hydrobromic acid (11.8 ml) was refluxed for 2.5 hours. After cooling, the obtained solution was evaporated in vacuo and further evaporated with ethanol twice. The residue was crystallized from diethyl ether and isopropyl alcohol to afford 7-hydroxy-2-methyl-1,2,3,4-tetrahydro-2-naphthylamine hydrobromide.

| | |
|---|---|
| mp : | 280-282°C |
| IR (Nujol) : | 3225 $cm^{-1}$ |
| NMR (DMSO-$d_6$, δ) : | 1.26 (3H, s), 1.74-2.01 (2H, m), 2.57-3.00 (4H, m), 6.49 (1H, d, J=2.5Hz), 6.58 (1H, dd, J=2.5 and 8.2Hz), 6.92 (1H, d, J=8.2Hz), 8.04 (3H, br s), 9.16 (1H, br s) |

Preparation 18

To a solution of 7-hydroxy-2-methyl-1,2,3,4-tetrahydro-2-naphthylamine hydrobromide (1.31 g) in water (20 ml) was added sodium hydrogencarbonate (1.28 g) and tetrahydrofuran (8 ml). To the mixture was added a solution of benzyloxycarbonyl chloride (0.76 ml) in tetrahydrofuran (4 ml) at 25°C. After stirring for 2 hours, sodium chloride was added to the mixture. The organic layer was separated, washed with brine, dried over sodium sulfate, and evaporated in vacuo. The residue was purified by column chromatography on silica gel with a mixture of chloroform and methanol (10:1) as an eluent to afford N-benzyloxycarbonyl-(7-hydroxy-2-methyl-1,2,3,4-tetrahydro-2-naphthyl)amine (1.59 g) as an oil. This compound was crystallized from a mixture of ethyl acetate (3 ml) and cyclohexane (14.5 ml) to afford a white powder (1.24 g).

| | |
|---|---|
| mp : | 119-121°C |
| IR (Nujol) : | 3340, 1660 $cm^{-1}$ |
| NMR ($CDCl_3$, δ) : | 1.46 (3H, s), 1.64-1.82 (1H, m), 2.21-2.45 (1H, m), 2.65-3.02 (4H, m), 4.73 (1H, br s), 5.04 (2H, s), 5.10 (1H, br s), 6.51 (1H, d, J=2.7Hz), 6.63 (1H, dd, J=2.7 and 8.2Hz), 6.95 (1H, d, J=8.2Hz), 7.32 (5H, s) |

Preparation 19

A mixture of N-benzyloxycarbonyl-(7-hydroxy-2-methyl-1,2,3,4-tetrahydro-2-naphthyl)amine (1.37 g), potassium carbonate (0.67 g) and ethyl bromoacetate (0.59 ml) in dimethylformamide (14 ml) was stirred at ambient temperature overnight. To the mixture was added cold water (60 ml) and the aqueous layer was decanted twice. The residue was dissolved in diisopropyl ether and the solution was washed with water, dried over magnesium sulfate, and evaporated in vacuo. The residue was crystallized from petroleum ether to afford N-benzyloxycarbonyl-(7-ethoxycarbonylmethoxy-2-methyl-1,2,3,4-tetrahydro-2-naphthyl)amine (1.66 g) as a powder.

| | |
|---|---|
| mp : | 83.5-84.5°C |
| IR (Nujol) : | 3360, 1750, 1720 $cm^{-1}$ |
| NMR ($CDCl_3$, δ) : | 1.30 (3H, t, J=7.1Hz), 1.46 (3H, s), 1.62-1.79 (1H, m), 2.25-2.46 (1H, m), 2.74 (2H, pseudo t, J=7Hz), 2.87 (2H, s), 4.27 (2H, q, J=7.1Hz), 4.57 (2H, s), 4.70 (1H, s), 5.04 (2H, s), 6.58 (1H, d, J=2.7Hz), 6.70 (1H, dd, J=2.7 and 8.4Hz), 7.00 (1H, d, J=8.4Hz), 7.32 (5H, s) |

Preparation 20

A solution of N-benzyloxycarbonyl-(7-ethoxycarbonylmethoxy-2-methyl-1,2,3,4-tetrahydro-2-naphthyl)amine (0.30 g) in a mixture of tetrahydrofuran (1.5 ml) and ethanol (1.5 ml) was hydrogenated at ambient temperature using 10% palladium on carbon (15 mg). After removal of the catalyst, ethyl acetate and saturated sodium hydrogencarbonate solution were added to the solution. The organic layer was separated, washed with brine, dried over sodium sulfate, and evaporated in vacuo to afford 7-ethoxycarbonylmethoxy-2-methyl-1,2,3,4-tetrahydro-2-naphthylamine (191 mg) as an oil.

| | |
|---|---|
| IR (Film) : | 3350, 1750, 1730 $cm^{-1}$ |
| NMR ($CDCl_3$, δ) : | 1.20 (3H, s), 1.30 (3H, t, J=7.1Hz), 1.77 (2H, pseudo t, J=6.8Hz), 2.80-3.10 (6H, m), 4.27 (2H, q, J=7.1Hz), 4.57 (3H, s), 6.59 (1H, d, J=2.7Hz), 6.71 (1H, dd, J=2.7 and 8.4Hz), 7.03 (1H, d, J=8.4Hz) |
| MASS (m/z) : | 263 ($M^+$) |

Preparation 21

To a suspension of N,O-dimethylhydroxylamine hydrochloride (12.66 g) in dichloromethane (127 ml) was added triethylamine (39.8 ml) at -5°C to 3°C and the whole was stirred for 15 minutes. To the suspension was added a solution of 3-chlorobenzoylchloride (22.72 g) in dichloromethane (33 ml) and the whole was stirred for 30 minutes. The mixture was poured into ice water (440 ml) and the organic layer was separated. The aqueous layer was extracted with dichloromethane (100 ml) and the combined organic layer was washed with brine, dried over sodium sulfate, and evaporated in vacuo. The residue was purified by distillation under reduced pressure to afford N-methoxy-N-methyl-3-chlorobenzamide (25.53 g).

| | |
|---|---|
| bp : | 106°C/0.3 mmHg |
| IR (Film) : | 1660, 1640 $cm^{-1}$ |

NMR ($CDCl_3$, δ) : 3.36 (3H, s), 3.55 (3H, s), 7.24-7.73 (4H, m)
MASS (m/z) : 199 ($M^+$)

Preparation 22

To a solution of n-propylmagnesium iodide (7.29 g : prepared from n-propyliodide 6.38 g and magnesium 0.91 g) in diethyl ether (9.3 ml) was added a solution of N-methoxy-N-methyl-3-chlorobenzamide (5.0 g) in diethyl ether (25 ml) at 6-9°C and the whole was stirred overnight. The solution was poured into saturated ammonium chloride solution (75 ml) and the pH of the mixture was adjusted to 1. The organic layer was separated and the aqueous layer was extracted with ethyl acetate. The combined organic layer was washed with brine, dried over sodium sulfate, and evaporated in vacuo. The residue was purified by column chromatography on silica gel with a mixture of n-hexane and ethyl acetate (20:1) as an eluent to afford 3'-chlorobutyrophenone (2.46 g) as an oil.

IR (Film) : 1690 $cm^{-1}$
NMR ($CDCl_3$, δ) : 1.00 (3H, t, J=7.4Hz), 1.65-1.90 (2H, m), 2.93 (2H, t, J=7.4Hz), 7.33-7.60 (2H, m), 7.79-8.00 (2H, m)
MASS (m/z) : 182 ($M^+$)

Preparation 23

To a solution of 3'-chlorobutyrophenone (0.50 g) in dimethoxyethane (5 ml) was dropwise added bromine (0.26 ml) at 0°C and the whole was stirred for 30 minutes at ambient temperature. The solution was evaporated in vacuo and water and ethyl acetate were added to the residue. The organic layer was separated, washed with saturated sodium thiosulfate solution and brine, dried over sodium sulfate, and evaporated in vacuo to afford 2-bromo-3'-chlorobutyrophenone (0.72 g) as an oil. This compound was used to the next reaction without further purification.

IR (Film) : 1685 $cm^{-1}$
NMR ($CDCl_3$, δ) : 1.09 (3H, t, J=7.3Hz), 2.00-2.39 (2H, m), 4.99 (1H, dd, J=6.4 and 7.7Hz), 7.38-7.64 (2H, m), 7.84-8.03 (2H, m)

Preparation 24

To an ice-cooled solution of (S)-2-benzyloxycarbonylamino-7-ethoxycarbonylmethoxy-1,2,3,4-tetrahydronaphthalene (0.30 g) and methyl iodide (0.24 ml) in N,N-dimethylformamide (7.8 ml) was added sodium hydride (60% dispersion in mineral oil; 78 mg). The mixture was stirred in an ice-bath for 4.5 hours. The reaction mixture was poured into brine, and extracted with ethyl acetate. The extract was washed twice with water and once with brine, dried over anhydrous sodium sulfate, and concentrated in vacuo. The residue was subjected to column chromatography on silica gel (gradient elution; 5:1 to 4:1 n-hexane-ethyl acetate) which afforded the desired product (0.10 g). Resubjection of some mixed fractions from the chromatography to medium pressure column chromatography (5:1 n-hexane-ethyl acetate as an eluent) gave an additional product (0.090 g), for a total of 0.19 g of (S)-2-(N-benzyloxycarbonyl-N-methyl)amino-7-ethoxycarbonylmethoxy-1,2,3,4-tetrahydronaphthalene.

IR (Film) : 3600, 3500, 3350, 1750, 1680 $cm^{-1}$
NMR ($CDCl_3$, δ) : 1.29 (3H, t, J=7Hz), 1.7-2.1 (2H, m), 2.7-3.0 (4H, m), 2.87 (3H, s), 4.26 (2H, q, J=7Hz), 4.2-4.6 (1H, m), 4.57 (2H, s), 5.15 (2H, s), 6.60 (1H, d, J=2Hz), 6.69 (1H, dd, J=2 and 8Hz), 6.99 (1H, d, J=8Hz), 7.35 (5H, s)
MASS (m/z) : 397

Preparation 25

The following compound was obtained according to a similar manner to that of Preparation 20.

(S)-2-Methylamino-7-ethoxycarbonylmethoxy-1,2,3,4-tetrahydronaphthalene

IR (Nujol) : 3400, 1760 $cm^{-1}$
NMR ($CDCl_3$, δ) : 1.30 (3H, t, J=7Hz), 1.87 (1H, br s), 1.9-2.2 (1H, m), 2.3-2.6 (1H, m), 2.76 (3H, s), 2.7-3.0 (2H, m), 3.0-3.5 (3H, m), 4.26 (2H, q, J=7Hz), 4.56 (2H, s), 6.61 (1H, d, J=2Hz), 6.74 (1H, dd, J=2 and 8Hz), 7.00 (1H, d, J=8Hz)
MASS (m/z) : 263

Preparation 26

To 2-amino-1,1-dimethyl-7-methoxy-1,2,3,4-tetrahydronaphthalene (8.28 g) was added a solution of D-(-)-mandelic acid (6.15 g) in ethanol (114 ml). The mixture was allowed to stand at ambient temperature overnight. The salt was collected and recrystallized twice from ethanol to give a diastereoisomer (3.12 g) with purity of 97% diastereomeric excess by high performance liquid chromatography analysis.

$[\alpha]_D^{23}$ = -65.7° (C=0.545, DMSO)

The salt was neutralized with 28% ammonium hydroxide, and the whole was extracted twice with chloroform. The extract was washed with brine, dried over anhydrous magnesium sulfate, and concentrated in vacuo to give (S)-2-amino-1,1-dimethyl-7-methoxy-1,2,3,4-tetrahydronaphthalene (1.76 g).

IR (Film) : 3350 $cm^{-1}$
NMR ($CDCl_3$, δ) : 1.23 (3H, s), 1.33 (3H, s), 1.65 (2H, s), 1.6-2.1 (2H, m), 2.7-3.0 (3H, m), 3.78 (3H, s), 6.68 (1H, dd, J=2 and 8Hz), 6.88 (1H, d, J=2Hz), 6.98 (1H, d, J=8Hz)
MASS (m/z) : 205

Preparation 27

The following compound was obtained according to similar manners to those of Preparations 2 and 17.

(S)-2-tert-Butoxycarbonylamino-1,1-dimethyl-7-hydroxy-1,2,3,4-tetrahydronaphthalene

IR (Nujol) : 3600-3100, 1680 $cm^{-1}$
NMR ($CDCl_3$, δ) : 1.22 (3H, s), 1.28 (3H, s), 1.45 (9H, s), 1.7-2.2 (2H, m), 2.78 (2H, t-like, J=ca. 6Hz), 3.7-3.9 (1H, m), 4.60 (1H, br d, J=9Hz), 5.51 (1H, s), 6.62 (1H, dd, J=2 and 8Hz), 6.80 (1H, d, J=2Hz), 6.90 (1H, d, J=8Hz)
MASS (m/z) : 291

Preparation 28

The following compound was obtained according to a similar manner to that of Preparation 19.

(S)-2-tert-Butoxycarbonylamino-1,1-dimethyl-7-ethoxycarbonylmethoxy-1,2,3,4-tetrahydronaphthalene

IR (Film) : 3380, 1750, 1700 $cm^{-1}$
NMR ($CDCl_3$, δ) : 1.23 (3H, s), 1.30 (3H, s), 1.30 (3H, t, J=7Hz), 1.44 (9H, s), 1.7-2.2 (2H, m), 2.81 (2H, t-like, J=ca. 6Hz), 3.7-3.9 (1H, m), 4.27 (2H, q, J=7Hz), 4.5-4.6 (1H, m), 4.59 (2H, s), 6.66 (1H, dd, J=2 and 8Hz), 6.91 (1H, d, J=2Hz), 6.98 (1H, d, J=8Hz)
MASS (m/z) : 337

Preparation 29

The following compound was obtained according to a similar manner to that of Preparation 4.

(S)-2-Amino-1,1-dimethyl-7-ethoxycarbonylmethoxy-1,2,3,4-tetrahydronaphthalene hydrochloride

NMR (DMSO-$d_6$, δ) : 1.21 (3H, t, J=7Hz), 1.23 (3H, s), 1.35 (3H, s), 1.8-2.1 (2H, m), 2.74 (2H, m), 3.2-3.3 (1H, m), 4.16 (2H, q, J=7Hz), 4.74 (2H, s), 6.71 (1H, dd, J=2 and 8Hz), 6.92 (1H, d, J=2Hz), 6.98 (1H, d, J=8Hz), 8.13 (3H, br s)

Preparation 30

To a mixture of 2-benzylamino-7-methoxy-1,2,3,4-tetrahydronaphthalene hydrochloride (15.1 g), triethylamine (20.8 ml), and N,N-dimethylformamide (50 ml) was added methyl chloroformate (5.7 ml). The mixture was stirred at ambient temperature for 4 hours and then a solution consisting of methyl chloroformate (5.7 ml) and triethylamine (10.4 ml) in N,N-dimethylformamide (25 ml) was added. After an additional stirring at ambient temperature for 4 hours and then at 60°C for 4 hours, the reaction mixture was diluted with ethyl acetate. The formed precipitate was filtered off, and

rinsed several times with ethyl acetate. The filtrate was concentrated in vacuo and the residue was taken up in ethyl acetate. The organic layer was successively washed twice with 3N hydrochloric acid, once with water and once with brine, dried over anhydrous magnesium sulfate, and concentrated in vacuo. The residue was purified by column chromatography on silica gel (elution with 4:1 n-hexaneethyl acetate) to give 2-(N-benzyl-N-methoxycarbonyl)amino-7-methyl-1,2,3,4-tetrahydronaphthalene (11.9 g).

NMR ($CDCl_3$, δ) : 1.8-2.0 (2H, m), 2.7-3.0 (4H, m), 3.73 (3H, s), 3.74 (3H, s), 4.0-4.8 (1H, br m), 4.51 (2H, br s), 6.52 (1H, d, J=2Hz), 6.66 (1H, dd, J=2 and 8Hz), 6.95 (1H, d, J=8Hz), 7.2-7.5 (5H, m)
MASS (m/z) : 325

Preparation 31

To a solution of 2-(N-benzyl-N-methoxycarbonyl)amino-7-methoxy-1,2,3,4-tetrahydronaphthalene (11.9 g) in 1,4-dioxane (119 ml) and water (11.9 ml) was added 2,3-dichloro-5,6-dicyano-1,4-benzoquinone (16.6 g). After stirring at ambient temperature for 3 hours, the mixture was filtered and the filtrate was concentrated in vacuo. The residue was triturated with ethyl acetate, and the precipitate was filtered off. The filtrate was successively washed with 10% sodium hydroxide, water and brine, dried over anhydrous sodium sulfate, and concentrated in vacuo. The residue was purified by column chromatography on silica gel (elution with 3:1 n-hexane-ethyl acetate) to give 2-(N-benzyl-N-methoxycarbonyl)amino-7-methoxy-4-oxo-1,2,3,4-tetrahydronaphthalene (9.8 g).

NMR ($CDCl_3$, δ) : 2.6-3.5 (4H, m), 3.77 (3H, s), 3.82 (3H, s), 4.3-4.5 (1H, m), 4.57 (2H, br s), 6.63 (1H, d, J=2Hz), 6.82 (1H, dd, J=2 and 8Hz), 7.2-7.5 (5H, m), 7.95 (1H, d, J=8Hz)
MASS (m/z) : 339

Preparation 32

A mixture of 2-(N-benzyl-N-methoxycarbonyl)amino-7-methoxy-4-oxo-1,2,3,4-tetrahydronaphthalene (6.9 g), N-methylanilinium trifluoroacetate (8.9 g), paraformaldehyde (1.2 g), and tetrahydrofuran (69 ml) was heated to reflux for 4 hours. After cooling, the reaction mixture was concentrated in vacuo. The residue was extracted three times with hot ethyl acetate, and the combined extracts were washed with saturated aqueous sodium carbonate and brine, dried over anhydrous magnesium sulfate, and concentrated in vacuo. Solvent removal was followed by column chromatography on silica gel (elution with 4:1 n-hexane-ethyl acetate) to give crude 2-(N-benzyl-N-methoxycarbonyl)amino-7-methoxy-3-methylene-4-oxo-1,2,3,4-tetrahydronaphthalene.

IR (Nujol) : 1700, 1670, 1600 $cm^{-1}$
NMR ($CDCl_3$, δ) : 2.80 (1H, dd, J=5 and 15Hz), 3.19 (1H, dd, J=12 and 15Hz), 3.76 (1H, d, J=16Hz), 3.77 (3H, s), 3.80 (3H, s), 3.85 (1H, d, J=16Hz), 4.6-5.0 (1H, m), 5.42 (1H, br s), 6.51 (2H, br s), 6.84 (1H, dd, J=2 and 8Hz), 7.1-7.4 (5H, m), 8.03 (1H, d, J=8Hz)
MASS (m/z) : 351

The crude product was dissolved in tetrahydrofuran (6 ml) and water (6 ml). To this solution was added 10% palladium on carbon (0.6 g). The slurry was stirred under an atmosphere of hydrogen for half an hour. The catalyst was filtered off, and rinsed with tetrahydrofuran. The filtrate was concentrated in vacuo to give cis-2-(N-benzyl-N-methoxycarbonyl)amino-7-methoxy-3-methyl-4-oxo-1,2,3,4-tetrahydronaphthalene.

NMR ($CDCl_3$, δ) : 1.26 (3H, d, J=7Hz), 2.8-3.2 (3H, m), 3.69 (3H, s), 3.74 (3H, s), 3.7-3.9 (1H, m), 4.7-5.2 (2H, m), 6.7-7.0 (2H, m), 7.0-7.4 (5H, m), 7.97 (1H, d, J=8Hz)
MASS (m/z) : 353

The product was dissolved in boron trifluoride etherate (20 ml) and treated with triethylsilane (12.9 ml). After stirring at ambient temperature for 12 hours, the reaction mixture was poured into water, and extracted three times with ethyl acetate. The combined organic layers were washed with water and brine, dried over anhydrous sodium sulfate, and concentrated in vacuo. The residue was purified by column chromatography on silica gel (elution with 8:1 n-hexane-ethyl acetate) to give a mixture of cis- and trans-2-(N-benzyl-N-methoxycarbonyl)amino-7-methoxy-3-methyl-1,2,3,4-tetrahydronaphthalene (1.5 g).

IR (Film) : 1690 $cm^{-1}$
NMR ($CDCl_3$, δ) : 0.8-1.1 (6H, m), 2.0-3.2 (10H, m), 3.7-3.8 (12H, m), 3.8-4.2 (2H, m), 4.2-4.4 (2H, m), 4.5-4.8 (2H, m), 6.4-6.6 (2H, m), 6.6-6.8 (2H, m), 6.8-7.0 (2H, m), 7.1-7.5 (10H, m)

MASS (m/z) : 339

Preparation 33

The following compound was obtained according to a similar manner to that of Preparation 17.

cis- and trans-2-Benzylamino-7-hydroxy-3-methyl-1,2,3,4-tetrahydronaphthalene

IR (Film) : 3600-2200 $cm^{-1}$
NMR ($CDCl_3$, δ) : 0.98 (3H, d, J=7Hz), 1.07 (3H, d, J=7Hz), 1.4-3.2 (16H, m), 3.7-4.1 (4H, m), 6.5-6.7 (4H, m), 6.91 (2H, d, J=8Hz), 7.2-7.6 (16H, m)
MASS (m/z) : 267

Preparation 34

The following compounds were obtained by subjecting the mixture of cis- and trans-compounds, which was prepared according to a similar manner to that of Preparation 19, to silica gel column chromatography.

1) cis-2-Benzylamino-7-ethoxycarbonylmethoxy-3-methyl-1,2,3,4-tetrahydronaphthalene

IR (Film) : 3350, 1750 $cm^{-1}$
NMR ($CDCl_3$, δ) : 0.96 (3H, d, J=7Hz), 1.29 (3H, t, J=7Hz), 1.4-2.1 (1H, m), 2.1-2.4 (1H, m), 2.56 (1H, m), 2.6-3.1 (4H, m), 3.83 (1H, d, J=13Hz), 3.89 (1H, d, J=13Hz), 4.26 (2H, q, J=7Hz), 4.57 (2H, s), 6.61 (1H, d, J=2Hz), 6.68 (1H, dd, J=2 and 8Hz), 6.97 (1H, d, J=8Hz), 7.2-7.5 (5H, m)
MASS (m/z) : 353

2) trans-2-Benzylamino-7-ethoxycarbonylmethoxy-3-methyl-1,2,3,4-tetrahydronaphthalene

IR (Film) : 3350, 1750 $cm^{-1}$
NMR ($CDCl_3$, δ) : l.09 (3H, d, J=7Hz), 1.30 (3H, t, J=7Hz), 1.8-2.2 (1H, m), 2.40 (1H, m), 2.6-3.0 (4H, m), 3.0-3.2 (1H, m), 3.84 (1H, d, J=13Hz), 3.99 (1H, d, J=13Hz), 4.26 (2H, q, J=7Hz), 4.56 (2H, s), 6.6-6.8 (2H, m), 6.96 (1H, d, J=8Hz), 7.1-7.6 (5H, m)
MASS (m/z) : 353

Example 1

A solution of 2,2''-oxybis[3'-chloro-2,4'-dihydroxyacetophenone] (47 mg), (S)-2-amino-7-ethoxycarbonylmethoxy-l,2,3,4-tetrahydronaphtahalene hydrochloride (57 mg) and triethylamine (24 mg) in ethanol (l ml) was stirred at ambient temperature for 50 minutes and cooled with ice water. Sodium borohydride (37.5 mg) was added to the mixture, and the resulting mixture was stirred under ice cooling for l5 minutes and at ambient temperature overnight. The reaction mixture was diluted with water and extracted twice with dichloromethane. The extracts were combined, dried over magnesium sulfate, and filtered. The filtrate was purified by two times column chromatography using chloroform, ethanol and methanol to give an oil, which was converted to oxalate to give a pale yellow powder of a mixture of (1R,2'S)- and (1S,2'S)-1-(3-chloro-4-hydroxyphenyl)-2-[(7-ethoxycarbonylmethoxy-l,2,3,4-tetrahydro-2-naphthyl)amino]ethanol oxalate (l2 mg).

mp : 105°C (dec.)
IR (Nujol) : 3350, 2750-2350, 1745, 1615, 1205 $cm^{-1}$
NMR (DMSO-$d_6$, δ) : 1.21 (6H, t, J=7Hz), 1.65 (2H, m), 2.1 (2H, m), 2.6-3.5 (14H, m), 4.16 (4H, quartet, J=7Hz), 4.71 (6H, m), 6.65 (4H, m), 7.0 (4H, m), 7.l5 (2H, m), 7.35 (2H, m)

Example 2

A mixture of trans-2-amino-7-ethoxycarbonylmethoxy-l,2,3,4-tetrahydro-l-naphthol hydrochloride (0.20 g), (R)-3-chlorostyrene oxide (0.ll g), triethylamine (0.l0 ml), and ethanol (l.3 ml) was refluxed for 4 hours with stirring. An additional amount of (R)-3-chlorostyrene oxide (0.054 g) in ethanol (l.5 ml) was added, and stirring was continued for an additional 4 hours under reflux. After cooling to ambient temperature, the reaction mixture was concentrated in vacuo. Purification of the residue by column chromatography on silica gel (eluent:chloroform-methanol = 20:l) afforded a mixture of (1R,1'R,2'R)- and (1R,l'S,2'S)-1-(3-chlorophenyl)-2-[(7-ethoxycarbonylmethoxy-l-hydroxy-l,2,3,4-tetrahydro-2-naphthyl)amino]ethanol (0.074 g).

The obtained compound (0.074 g) was treated with 5N hydrogen chloride in ethanol to afford a mixture of (1R,1'R,2'R)- and (1R,1'S,2'S)-1-(3-chlorophenyl)-2-[(7-ethoxycarbonylmethoxy-l-hydroxy-l,2,3,4-tetrahydro-2- naphthyl)amino]ethanol hydrochloride.

| | |
|---|---|
| mp : | l22-l27°C |
| IR (Nujol) : | 3300, 3250, 1740, 1720 $cm^{-1}$ |
| NMR (DMSO-$d_6$, δ) : | 1.21 (6H, t, J=7Hz), 1.7-2.0 (2H, m), 2.2-2.4 (2H, m), 2.7-2.9 (4H, m), 3.l-3.6 (6H, m), 4.l6 (4H, q, J=7Hz), 4.74 (4H, s), 4.7-4.9 (2H, m), 5.0-5.2 (2H, m), 6.2-6.4 (4H, m), 6.79 (2H, dd, J=2 and 8Hz), 7.0-7.l (4H, m), 7.4-7.6 (8H, m), 8.4-9.5 (4H, m) |
| MASS (m/z) : | 422 and 420 ($M^+$(free)+1) |

Example 3

(S)-2-Amino-7-ethoxycarbonylmethoxy-l,2,3,4-tetrahydronaphthalene hydrochloride (2.ll g) was converted to a free amine by treatment with sodium bicarbonate aqueous solution. A solution of the obtained free amine and (+)-[3-chloro-4-(2-methoxyethoxymethoxy)phenyl]oxirane (l.9l g) in ethanol (20 ml) was refluxed for 9 hours and concentrated under reduced pressure. The residue was purified by column chromatography on silica gel using a mixture of dichloromethane and methanol as an eluent to give an oil of (-)-1-[3-chloro-4-(2-methoxyethoxymethoxy)phenyl]-2-[{(2S)-7-ethoxycarbonylmethoxy-l,2,3,4-tetrahydro-2-naphthyl}amino]ethanol (l.65 g) (a single stereoisomer).

| | |
|---|---|
| $[\alpha]_D^{20.0}$ | = -45.38° (C=0.52, MeOH) |
| IR (Film) : | 3400-3300, l755, l735, l235, ll95, ll60, ll25, ll00, l070 $cm^{-1}$ |
| NMR ($CDCl_3$, δ) : | l.30 (3H, t, J=7Hz), l.62 (lH, m), 2.03 (lH, m), 2.43 (lH, broad), 2.55-3.l (7H, m), 3.37 (3H, s), 3.56 (2H, m), 3.87 (2H, m), 4.27 (2H, quartet, J=7Hz), 4.58 (2H, s), 4.64 (lH, dd, J=3.5 and 9Hz), 5.33 (2H, s), 6.6l (lH, d, J=2.5Hz), 6.70 (lH, dd, J=2.5 and 8.5Hz), 7.00 (lH, d, J=8.5Hz), 7.20 (2H, s), 7.4l (lH, s) |

Example 4

A solution of (-)-l-[3-chloro-4-(2-methoxyethoxymethoxy)phenyl]-2-[{(2S)-7-ethoxycarbonylmethoxy-l,2,3,4-tetrahydro-2-naphthyl}amino]ethanol (a single stereoisomer) (l.62 g) in 0.2M methanesulfonic acid ethanolic solution (24.5 ml) was heated at 50°C for 6 hours, concentrated in vacuo, and partitioned between ethyl acetate and sodium bicarbonate aqueous solution. The organic layer was separated, and the aqueous layer was further extracted twice with ethyl acetate. The organic layers were combined, dried over magnesium sulfate, and concentrated in vacuo. The residue was purified by column chromatography on silica gel using methylene chloride and methanol as an eluent to give an oil of (-)-1-(3-chloro-4-hydroxyphenyl)-2-[{(2S)-7-ethoxycarbonylmethoxy-l,2,3,4-tetrahydro-2-naphthyl}amino]ethanol.

The obtained oil was treated with 4N hydrogen chloride in ethyl acetate and crystallized from diethyl ether to give a colourless powder of (-)-l-(3-chloro-4-hydroxyphenyl)-2-[{(2S)-7-ethoxycarbonylmethoxy-l,2,3,4-tetrahydro-2-naphthyl}amino]ethanol hydrochloride (0.95 g) (a single stereoisomer).

| | |
|---|---|
| $[\alpha]_D^{22}$ | = -82.49° (C=0.48, MeOH) |
| IR (Nujol) : | 3400-3l00, 2800-2300, l735, l2l0 $cm^{-1}$ |
| NMR (DMSO-$d_6$, δ) : | l.2l (3H, t, J=7Hz), l.8 (lH, m), 2.3 (lH, m), 2.6-3.6 (7H, m), 4.l6 (2H, quartet, J=7Hz), 4.72 (2H, s), 4.95 (lH, m), 6.l7 (lH, d, J=4Hz), 6.66 (lH, d, J=2.5Hz), 6.7l (lH, dd, J=2.5 and 8.5Hz), 7.0l (2H, d, J=8.5Hz), 7.l9 (lH, dd, J=2 and 8.5Hz), 7.39 (lH, d, J=2Hz), 8.85 (lH, broad), 9.3 (lH, broad) |
| MASS (m/z) : | 422 and 420 ($M^+$(free)+l), 402, 262, 233 |

| Analysis Calcd. for $C_{22}H_{26}ClNO_5 \cdot HCl$ | | | |
|---|---|---|---|
| | C 57.90, | H 5.96, | N 3.07 |
| Found : | C 57.56, | H 6.l0, | N 3.06 |

Example 5

The following compounds were obtained according to a similar manner to that of Example 1.

1) (1R,2'S)- and (1S,2'S)-1-(4-Benzyloxy-3-chlorophenyl)-2-[(7-ethoxycarbonylmethoxy-1,2,3,4-tetrahydro-2-naphthyl)amino]ethanol hydrochloride

mp : 109-119°C
IR (Nujol) : 3320, 2750, 2350, 1740, 1255, 1200 $cm^{-1}$
NMR ($DMSO-d_6$, δ) : 1.21 (6H, t, J=7Hz), 1.78 (2H, m), 2.31 (2H, m) 2.78 (6H, m), 3.18 (6H, m), 3.45 (2H, m), 4.15 (4H, quartet, J=7Hz), 4.72 (4H, s), 4.96 (2H, m), 5.24 (4H, s), 6.25 (2H, m), 6.66 (2H, m), 6.74 (2H, m), 7.02 (2H, d, J=8.5Hz), 7.2-7.55 (16H, m), 8.86 (2H, br), 9.36 (2H, br)
FAB-MASS (m/z) : 510 ($M^+$(free)+1), 492, 262, 233

2) (1R,2'R)- and (1S,2'R)-1-(4-Benzyloxy-3-chlorophenyl)-2-[(7-ethoxycarbonylmethoxy-1,2,3,4-tetrahydro-2-naphthyl)amino]ethanol hydrochloride

mp : 117-123°C
IR (Nujol) : 3370, 2750-2350, 1720, 1255, 1200 $cm^{-1}$
NMR ($DMSO-d_6$, δ) : 1.21 (6H, t, J=7Hz), 1.8 (2H, m), 2.3 (2H, m), 2.77 (6H, m), 3.18 (6H, m), 3.45 (2H, m), 4.16 (4H, quartet, J=7Hz), 4.72 (4H, s), 4.94 (2H, m), 5.24 (14H, s), 6.24 (2H, m), 6.69 (2H, m), 6.74 (2H, m), 7.02 (2H, d, J=8.5Hz), 7.2-7.55 (16H, m), 8.85 (2H, br), 9.35 (2H, br)

3) (1R,2'S)- and (1S,2'S)-1-(3-Chloro-4-methoxyphenyl)-2-[(7-ethoxycarbonylmethoxy-1,2,3,4-tetrahydro-2-naphthyl)amino]ethanol hydrochloride

mp : 73-98°C
IR (Nujol) : 3300, 1745, 1255, 1200 $cm^{-1}$
NMR ($DMSO-d_6$, δ) : 1.21 (6H, t, J=7Hz), 1.78 (2H, m), 2.30 (2H, m), 2.78 (6H, m), 3.18 (6H, m), 3.45 (2H, m), 3.86 (6H, s), 4.16 (4H, quartet, J=7Hz), 4.72 (4H, s), 4.96 (2H, m), 6.25 (2H, m), 6.65-6.75 (4H, m), 7.02 (2H, d, J=8Hz), 7.18 (2H, d, J=8.5Hz), 7.37 (2H, dd, J=8.5 and 2Hz), 7.50 (2H, d, J=2Hz), 8.85 (2H, br), 9.35 (2H, br)

4) (1R,2'S)- and (1S,2'S)-1-(3-Chloro-4-methylsulfonylphenyl)-2-[(7-ethoxycarbonylmethoxy-1,2,3,4-tetrahydro-2-naphthyl)amino]ethanol hydrochloride

mp : 214-220°C (dec.)
IR (Nujol) : 3600-3300, 3230, 2750-2350, 1750, 1320, 1200, 1150 $cm^{-1}$
NMR ($DMSO-d_6$, δ) : 1.21 (6H, t, J=7Hz), 1.79 (2H, m), 2.31 (2H, m), 2.78 (6H, m), 3.05 (6H, s), 3.20 (6H, m), 3.45 (2H, m), 4.16 (4H, quartet, J=7Hz), 4.72 (4H, s), 5.04 (2H, m), 6.36 (2H, m), 6.65-6.75 (4H, m), 7.02 (2H, d, J=8.5Hz), 7.35-7.6 (6H, m), 8.93 (2H, br), 9.49 (4H, br)

5) (1R,2'S)- and (1S,2'S)-2-[(7-Ethoxycarbonylmethoxy-1,2,3,4-tetrahydro-2-naphthyl)amino]-1-(3-phenoxyphenyl)ethanol hydrochloride

mp : 55-56°C
IR (Film) : 3320, 2800-2300, 1745, 1240, 1205 $cm^{-1}$
NMR ($DMSO-d_6$, δ) : 1.21 (6H, t, J=7Hz), 1.78 (2H, m), 2.29 (2H, m), 2.55-3.6 (14H, m), 4.16 (4H, quartet, J=7Hz), 4.72 (4H, s), 5.02 (2H, m), 6.28 (2H, m), 6.65-6.75 (4H, m), 6.9-7.25 (14H, m), 7.35-7.5 (6H, m), 8.89 (2H, br), 9.43 (2H, br)

6) (1R,2'S)- and (1S,2'S)-1-(4-Amino-3,5-dichlorophenyl-2-[(7-ethoxycarbonylmethoxy-1,2,3,4-tetrahydro-2- naphthyl)amino]ethanol hydrochloride

IR (Film) : 3310, 1745 $cm^{-1}$
NMR ($DMSO-d_6$, δ) : 1.21 (6H, t, J=7.1Hz), 1.60-1.95 (2H, m), 2.21-2.42 (2H, m), 2.58-3.58 (14H, m), 4.16 (4H, q, J=7.1Hz), 4.72 (4H, s), 4.80-4.95 (2H, m), 6.60-6.76 (4H, m), 7.02 (2H, d, J=8.4Hz), 7.31 (4H, m)

7) (1R,2'S)- and (1S,2'S)-1-(3-Chloro-4-fluorophenyl)-2-[(7-ethoxycarbonylmethoxy-1,2,3,4-tetrahydro-2-naphthyl)amino]ethanol hydrochloride

| | |
|---|---|
| mp : | 181-184°C |
| IR (Nujol) : | 3300, 2700-2300, 1760 $cm^{-1}$ |
| NMR (DMSO-$d_6$, δ) : | 1.20 (6H, t, J=7Hz), 1.6-2.0 (2H, m), 2.2-3.6 (14H, m), 4.15 (4H, q, J=7Hz), 4.72 (4H, s), 5.0-5.2 (2H, m), 6.3-6.5 (2H, br s), 6.6-6.8 (6H, m), 6.9-7.1 (2H, m), 7.4-7.5 (4H, m), 7.6-7.7 (2H, m), 8.8-9.2 (2H, m), 9.4-9.8 (2H, m) |
| FAB-MASS (m/z) : | 424 ($M^+$(free)+3), 422 ($M^+$(free)+1) |

8) (1R,2'S)- and (1S,2'S)-2-[7-(Ethoxycarbonylmethoxy-1,2,3,4-tetrahydro-2-naphthyl)amino]-1-(3-methoxyphenyl)ethanol hydrochloride

| | |
|---|---|
| mp : | 152-154°C |
| IR (Nujol) : | 3330, 1745, 1610 $cm^{-1}$ |
| NMR (DMSO-$d_6$, δ) : | 1.21 (6H, t, J=7Hz), 1.65-1.9 (2H, m), 2.2-3.6 (16H, m), 3.78 (6H, s), 4.15 (4H, q, J=7Hz), 4.72 (4H, s), 4.95-5.1 (2H, m), 6.2-6.3 (2H, m), 6.6-6.75 (4H, m), 6.85-7.05 (8H, m), 7.25-7.4 (2H, m), 8.7-9.0 (2H, m), 9.2-9.5 (2H, m) |

9) (1R,2'S)- and (1S,2'S)-2-[7-(Ethoxycarbonylmethoxy-1,2,3,4-tetrahydro-2-naphthyl)amino]-1-(3- nitrophenyl)ethanol hydrochloride

| | |
|---|---|
| mp : | 177-179°C |
| IR (Nujol) : | 3300 1730, 1620 $cm^{-1}$ |
| NMR (DMSO-$d_6$, δ) : | 1.21 (6H, t, J=7Hz), 1.65-1.95 (2H, m), 2.25-3.6 (16H, m), 4.15 (4H, q, J=7Hz), 4.72 (4H, s), 5.15-5.30 (2H, m), 6.5-6.6 (2H, m), 6.6-6.8 (4H, m), 6.95-7.1 (2H, m), 7.7-7.8 (2H, m), 7.9-8.0 (2H, m), 8.15-8.3 (2H, m), 8.35 (2H, s), 8.9-9.1 (2H, m), 9.3-9.5 (2H, m) |
| MASS (m/z) : | 415 ($M^+$(free)+1) |

10) (1R,2'S)- and (1S,2'S)-1-(3-Cyanophenyl)-2-[7-ethoxycarbonylmethoxy-1,2,3,4-tetrahydro-2-naphtyl)amino]ethanol hydrochloride

| | |
|---|---|
| mp : | 171-174°C |
| IR (Nujol) : | 3300, 2220, 1720, 1605 $cm^{-1}$ |
| NMR (DMSO-$d_6$, δ) : | 1.21 (6H, t, J=7Hz), 1.6-1.9 (2H, m), 2.2-3.6 (16H, m), 4.15 (4H, q, J=7Hz), 4.72 (4H, s), 5.05-5.2 (2H, m), 6.4-6.5 (2H, m), 6.6-6.8 (4H, m), 6.95-7.1 (2H, m), 7.6-7.75 (2H, m), 7.75-7.85 (4H, m), 7.91 (2H, s), 8.8-9.1 (2H, m), 9.3-9.6 (2H, m) |

Example 6

The following compounds were obtained by reacting the compounds, which were prepared according to a similar manner to that of Example 3, with the corresponding acid.

1) (-)-1-(4-Benzyloxy-3-chlorophenyl)-2-[{(2S)-7-ethoxycarbonylmethoxy-1,2,3,4-tetrahydro-2-naphthyl}amino]ethanol hydrochloride (a single stereoisomer)

| | |
|---|---|
| mp : | 61-69°C |
| $[\alpha]_D^{31.2}$ | = -67.54° (C=0.53, MeOH) |
| IR (Nujol) : | 3350-3150, 2750-2350, 1740, 1255, 1200 $cm^{-1}$ |
| NMR (DMSO-$d_6$, δ) : | 1.21 (3H, t, J=7Hz), 1.78 (1H, m), 2.27 (1H, m), 2.78 (3H, m), 3.18 (3H, m), 3.45 (1H, m), 4.15 (2H, quartet, J=7Hz), 4.71 (2H, s), 4.98 (1H, m), 5.24 (2H, s), 6.25 (1H, d, J=4Hz), 6.66 (1H, d, J=2Hz), 6.72 (1H, dd, J=8.5 and 2Hz), 7.02 (1H, d, J=8.5Hz), 7.2-7.55 (8H, m), 8.92 (1H, br), 9.88 (1H, br) |

2) (+)-1-(4-Benzyloxy-3-chlorophenyl)-2-[{(2R)-7-ethoxycarbonylmethoxy-1,2,3,4-tetrahydro-2-naphthyl)amino]ethanol hydrochloride (a single stereoisomer)

| | |
|---|---|
| mp : | 135-137.5°C |
| $[\alpha]_D^{31.6}$ | = 25.88° (C=0.255, MeOH) |

| | |
|---|---|
| IR (Nujol) : | 3340, 2750-2250, 1720, 1255, 1205 $cm^{-1}$ |
| NMR (DMSO-$d_6$, δ) : | 1.21 (3H, t, J=7Hz), 1.71 (1H, m), 2.30 (1H, m), 2.77 (3H, m), 3.15 (3H, m), 3.44 (1H, m), 4.16 (2H, quartet, J=7Hz), 4.72 (2H, s), 4.95 (1H, m), 5.24 (2H, s), 6.23 (1H, d, J=4Hz), 6.65-6.75 (2H, m), 7.02 (1H, d, J=8.5Hz), 7.2-7.55 (8H, m), 8.83 (1H, br), 9.35 (1H, br) |

3) (-)-1-[3-Chloro-4-(2-methoxyethoxymethoxy)phenyl]-2-[{(2S)-7-ethoxycarbonylmethoxy-1,2,3,4-tetrahydro-2-naphthyl}amino]ethanol oxalate (a single stereoisomer)

| | |
|---|---|
| mp : | 68-70°C |
| $[\alpha]_D^{23.6}$ | = -72.80° (C=0.25, MeOH) |
| IR (Film) : | 3440, 3400, 2800-2300, 1740, 1605, 1230 $cm^{-1}$ |
| NMR (DMSO-$d_6$, δ) : | 1.21 (3H, t, J=7Hz), 1.74 (1H, m), 2.19 (1H, m), 2.77 (3H, m), 3.12 (3H, m), 3.22 (3H, s), 3.44 (1H, m), 3.47 (2H, m), 3.75 (2H, m), 4.15 (2H, quartet, J=7Hz), 4.71 (2H, s), 4.91 (1H, br d, J=8Hz), 5.35 (2H, s), 5.8 (4H, br), 6.6-6.75 (2H, m), 7.02 (1H, d, J=8.5Hz), 7.2-7.4 (2H, m), 7.51 (1H, d, J=2Hz) |

4) (1R,2'S)- and (1S,2'S)-1-(5-Chloro-2-fluorophenyl-2-[(7-ethoxycarbonylmethoxy-1,2,3,4-tetrahydro-2-naphthyl)amino]ethanol hydrochloride

| | |
|---|---|
| mp : | 150-152°C |
| IR (Nujol) : | 3280, 2750-2350, 1740 $cm^{-1}$ |
| NMR (DMSO-$d_6$, δ) : | 1.28 (6H, t, J=7Hz), 1.6-2.0 (2H, m), 2.2-3.6 (14H, m), 4.15 (4H, q, J=7Hz), 4.72 (4H, s), 5.2-5.4 (2H, m), 6.4-6.5 (2H, m), 6.6-6.8 (4H, m), 6.9-7.1 (2H, m), 7.2-7.7 (6H, m), 8.8-9.2 (2H, m), 9.5-9.9 (2H, m) |
| FAB-MASS (m/z) : | 424 ($M^+$(free)+3), 422 ($M^+$(free)+1) |

5) (1R,2'S)- and (1S,2'S)-2-[(7-Ethoxycarbonylmethoxy-1,2,3,4-tetrahydro-2-naphthyl)amino]-1-(3-trifluoromethoxy)ethanol hydrochloride

| | |
|---|---|
| mp : | 163-164°C |
| IR (Nujol) : | 3300, 2700-2400, 1740 $cm^{-1}$ |
| NMR (DMSO-$d_6$, δ) : | 1.20 (6H, t, J=7Hz), 1.6-2.0 (2H, m), 2.2-3.6 (14H, m), 4.15 (4H, q, J=7Hz), 4.71 (4H, s), 5.0-5.2 (2H, m), 6.4-6.5 (2H, m), 6.6-6.8 (4H, m), 6.9-7.1 (2H, m), 7.3-7.6 (8H, m), 8.8-9.2 (2H, m), 9.3-9.5 (2H, m) |
| FAB-MASS (m/z) : | 456 ($M^+$(free)+3), 454 ($M^+$(free)+1) |

6) (1R,2'S)-2-[(6-Chloro-7-ethoxycarbonylmethoxy-1,2,3,4-tetrahydro-2-naphthyl)amino]-1-(3-chlorophenyl)ethanol hydrochloride

| | |
|---|---|
| mp : | 97-101°C |
| IR (Nujol) : | 3300, 3100-2000, 1740, 1720 $cm^{-1}$ |
| NMR (DMSO-$d_6$, δ) : | 1.21 (3H, t, J=7Hz), 1.6-2.0 (1H, m), 2.2-2.5 (1H, m), 2.6-3.8 (5H, m), 4.16 (2H, q, J=7Hz), 4.84 (2H, s), 5.0-5.2 (1H, m), 6.3-6.5 (1H, m), 6.78 (1H, s), 7.20 (1H, s), 7.3-7.6 (4H, m), 8.8-9.2 (1H, m), 9.4-9.8 (1H, m) |
| FAB-MASS (m/z) : | 442 ($M^+$+5-HCl), 440 ($M^+$+3-HCl), 438 ($M^+$+1-HCl) |

7) (1R,2'S)-1-(3-Chlorophenyl)-2-[{N-(7-ethoxycarbonylmethoxy-1,2,3,4-tetrahydro-2-naphthyl)-N-methyl}amino]ethanol hydrochloride

| | |
|---|---|
| NMR (DMSO-$d_6$, δ) : | 1.30 (3H, t, J=7Hz), 1.5-2.1 (4H, m), 2.1-4.0 (8H, m), 4.1-4.4 (2H, m), 4.58 (2H, s), 5.0-5.8 (2H, m), 6.4-6.9 (2H, m), 6.9-7.1 (1H, m), 7.1-7.6 (4H, m), 11.0-12.0 (1H, m) |

8) (1R,2'S)-1-(3-Chlorophenyl)-2-[(1,1-dimethyl-7-ethoxycarbonylmethoxy-1,2,3,4-tetrahydro-2-naphthyl)amino]ethanol hydrochloride

| | |
|---|---|
| NMR (DMSO-$d_6$, δ) : | 1.21 (3H, t, J=7Hz), 1.34 (3H, s), 1.51 (3H, s), 1.7-2.0 (1H, m), 2.1-2.4 (1H, m), 2.6-3.0 (2H, m), 3.1-3.7 (3H, m), 4.16 (2H, q, J=7Hz), 4.75 (2H, s), 5.0-5.2 (1H, m), 6.43 (1H, d, J=4Hz), 6.71 (1H, dd, J=2 and 8Hz), 6.9-7.1 (2H, m), 7.3-7.6 (4H, m), 8.0-8.4 (1H, m), 9.1-9.5 (1H, m) |
| MASS (m/z) : | 433 ($M^+$+2-HCl), 431 ($M^+$-HCl) |

Example 7

(-)-1-(4-Benzyloxy-3-chlorophenyl)-2-[{(2S)-7-ethoxycarbonylmethoxy-1,2,3,4-tetrahydro-2-naphthyl}amino]ethanol hydrochloride (a single stereoisomer) (5.32 g) was catalytically hydrogenated at ambient temperature in ethanol (400 ml) using 10% palladium on carbon. After removal of the catalyst, the solution was evaporated in vacuo. The residue was purified by silica gel column chromatography using a mixture of dichloromethane and methanol as an eluent, treated with 4N hydrogen chloride in ethyl acetate, and crystallized from diethyl ether. The obtained powder was triturated with ethyl acetate, a mixture of ethyl acetate and ethanol, and ethyl acetate, successively to afford (-)-1-(3-chloro-4-hydroxyphenyl)-2-[{(2S)-7-ethoxycarbonylmethoxy-1,2,3,4-tetrahydro-2-naphthyl}amino]ethanol hydrochloride (a single stereoisomer) (2.38 g) as a colorless powder.

| | |
|---|---|
| mp : | 137-142°C |
| $[\alpha]_D^{22.4}$ | = -82.49° (C=0.48, MeOH) |
| IR (Nujol) : | 3400-3100, 2800-2300, 1735, 1210 $cm^{-1}$ |
| NMR ($DMSO-d_6$, δ) : | 1.21 (3H, t, J=7Hz), 1.8 (1H, m), 2.3 (1H, m), 2.6-3.6 (7H, m), 4.16 (2H, quartet, J=7Hz), 4.72 (2H, s), 4.95 (1H, m), 6.17 (1H, d, J=4Hz), 6.66 (1H, d, J=2.5Hz), 6.71 (1H, dd, J=2.5 and 8.5Hz), 7.01 (2H, d, J=8.5Hz), 7.19 (1H, dd, J=2 and 8.5Hz), 7.39 (1H, d, J=2Hz), 8.85 (1H, br), 9.3 (1H, br) |
| MASS (m/z) : | 422 and 420 ($M^+$(free)+1), 402, 262, 233 |

Example 8

The following compounds were obtained according to a similar manner to that of Example 7.

1) (1R,2'S)- and (1S,2'S)-1-(3-Chloro-4-hydroxyphenyl)-2-[(7-ethoxycarbonylmethoxy-1,2,3,4-tetrahydro-2-naphthyl)amino]ethanol hydrochloride

| | |
|---|---|
| mp : | 168-171°C (dec.) |
| IR (Nujol) : | 3500, 3330, 3200, 3120, 2800-2300, 1750, 1220, 1200 $cm^{-1}$ |
| NMR ($DMSO-d_6$, δ) : | 1.21 (6H, t, J=7Hz), 1.78 (2H, m), 2.31 (2H, m), 2.78 (6H, m), 3.15 (6H, m), 3.4 (2H, m), 4.16 (4H, quartet, J=7Hz), 4.72 (4H, s), 4.93 (2H, m), 6.17 (2H, br s), 6.65-6.75 (4H, m), 7.02 (4H, d, J=8.5Hz), 7.19 (2H, dd, J=8.5 and 2Hz), 7.89 (2H, d, J=2Hz), 8.85 (2H, br), 9.90 (2H, br), 10.81 (2H, s) |
| FAB-MASS (m/z) : | 422 and 420 ($M^+$(free)+1), 402, 262, 233 |

2) (1R,2'S)- and (1S,2'S)-1-(3-Chloro-4-hydroxyphenyl)-2-[(7-ethoxycarbonylmethoxy-1,2,3,4-tetrahydro-2-naphthyl)amino]ethanol oxalate

| | |
|---|---|
| mp : | 105°C (dec.) |
| IR (Nujol) : | 3350, 2750-2350, 1745, 1615, 1205 $cm^{-1}$ |
| NMR ($DMSO-d_6$, δ) : | 1.21 (6H, t, J=7Hz), 1.65 (2H, m), 2.1 (2H, m), 2.6-3.5 (14H, m), 4.16 (4H, quartet, J=7Hz), 4.71 (6H, m), 6.65 (4H, m), 7.0 (4H, m), 7.15 (2H, m), 7.35 (2H, m) |
| FAB-MASS (m/z) : | 422 and 420 ($M^+$(free)+1), 402, 262, 233 |

3) (1R,2'R)- and (1S,2'R)-1-(3-Chloro-4-hydroxyphenyl)-2-[(7-ethoxycarbonylmethoxy-1,2,3,4-tetrahydro-2-naphthyl)amino]ethanol hydrochloride

| | |
|---|---|
| mp : | 156-169°C |
| IR (Nujol) : | 3500, 3330, 3200, 3120, 2750-2300, 1745, 1215, 1200 $cm^{-1}$ |
| NMR ($DMSO-d_6$, δ) : | 1.21 (6H, t, J=7Hz), 1.78 (2H, m), 2.30 (2H, m), 2.78 (6H, m), 3.15 (6H, m), 3.4 (2H, m), 4.16 (4H, quartet, J=7Hz), 4.72 (4H, s), 4.93 (2H, m), 6.17 (2H, br s), 6.65-6.75 (4H, m), 7.02 (4H, d, J=8.5Hz), 7.19 (2H, dd, J=8.5 and 2Hz), 7.89 (2H, d, J=2Hz), 8.85 (2H, br), 9.90 (2H, br), 10.81 (2H, s) |

4) (+)-1-(3-Chloro-4-hydroxyphenyl)-2-[{(2R)-7-ethoxycarbonylmethoxy-1,2,3,4-tetrahydro-2-naphthyl}amino]ethanol hydrochloride (a single stereoisomer)

| | |
|---|---|
| mp : | 147-150°C (dec.) |
| $[\alpha]_D^{28.8}$ | = 31.51° (C=0.165, MeOH) |

IR (Nujol) : 3650-3100, 1735, 1215, 1200 $cm^{-1}$

NMR (DMSO-$d_6$, δ) : 1.21 (3H, t, J=7Hz), 1.72 (1H, m), 2.30 (1H, m), 2.6-3.0 (3H, m), 3.14 (3H, m), 3.40 (1H, m), 4.16 (2H, quartet, J=7Hz), 4.72 (2H, s), 4.92 (1H, m), 6.15 (1H, br s), 6.65-6.75 (2H, m), 7.01 (2H, d, J=8.5Hz), 7.19 (1H, dd, J=8.5 and 2Hz), 7.39 (1H, d, J=2Hz), 8.80 (1H, br), 9.39 (1H, br), 10.29 (1H, s)

Example 9

A mixture of (1R,2'S)- and (1S,2'S)-1-(4-benzyloxy-3-chlorophenyl)-2-[(7-ethoxycarbonylmethoxy-1,2,3,4-tetrahydro-2-naphthyl)amino]ethanol hydrochloride (55 mg), ammonium formate (19 mg), and 10% palladium on carbon (10 mg) in refluxing ethanol (3 ml) was stirred for 1.5 hours. After filtration, the mixture was evaporated in vacuo and partitioned between ethyl acetate and sodium hydrogencarbonate aqueous solution. The organic layer was washed with brine, dried, evaporated in vacuo, and chromatographed over silica gel using a mixture of dichloromethane and methanol as an eluent. The obtained oil was converted to hydrochloride in a usual manner and the hydrochloride was crystallized from diethyl ether to afford a mixture of (1R,2'S)- and (1S,2'S)-2-[(7-ethoxycarbonylmethoxy-1,2,3,4-tetrahydro-2-naphthyl)amino]-1-(4-hydroxyphenyl)ethanol hydrochloride (29 mg) as a pale yellow powder.

mp : 64-80°C

IR (Nujol) : 3600-3120, 2700-2050, 1735, 1215, 1200 $cm^{-1}$

NMR (DMSO-$d_6$, δ) : 1.21 (6H, t, J=7Hz), 1.75 (2H, m), 2.3 (2H, m), 2.77 (6H, m), 3.15 (6H, m), 3.59 (2H, m), 4.16 (4H, quartet, J=7Hz), 4.72 (4H, s), 4.95 (2H, m), 6.65-7.25 (14H, m), 8.2 (2H, br s), 8.8 (2H, br), 9.2 (2H, br), 9.5 (2H, br)

FAB-MASS (m/z) : 386 ($M^+$(free)+1), 368, 262, 233

Example 10

A solution of (-)-1-(3-chloro-4-hydroxyphenyl)-2-[{(2S)-7-ethoxycarbonylmethoxy-1,2,3,4-tetrahydro-2-naphthyl}amino]ethanol hydrochloride (a single stereoisomer) (240 mg) and 0.1 N sodium hydroxide aqueous solution (25.6 ml) in ethanol (24 ml) was stirred in a nitrogen atmosphere at ambient temperature for 1 hour, neutralized with 0.1N hydrochloric acid, and concentrated in vacuo. The residue was triturated with a mixture of diethyl ether and water to afford a powder, which was converted to oxalate in a usual manner. The oxalate was crystallized from ethyl acetate and washed with diethyl ether and isopropanol to afford (-)-2-[{(2S)-7-carboxymethoxy-1,2,3,4-tetrahydro-2-naphthyl}amino]-1-(3-chloro-4-hydroxyphenyl)ethanol oxalate (a single stereoisomer) (68 mg) as a colorless powder.

mp : 108°C (dec.)

NMR (DMSO-$d_6$, δ) : 1.72 (1H, m), 2.16 (1H, m), 2.74 (3H, m), 3.10 (3H, m), 3.36 (1H, m), 4.52 (2H, s), 4.85 (1H, m), 6.5 (6H, br), 6.55-7.4 (6H, m)

Example 11

The following compounds were obtained according to similar manners to those of Preparation 20 and Example 3.

1) (1R,2'R,3'S)- and (1R,2'S,3'R)-1-(3-Chlorophenyl)-2-[(7-ethoxycarbonylmethoxy-3-methyl-1,2,3,4-tetrahydro-2-naphthyl)amino]ethanol hydrochloride

IR (Film) : 3300, 3100-2300, 1730 $cm^{-1}$

NMR ($CDCl_3$, δ) : 1.18 (6H, br d, J=5Hz), 1.30 (6H, t, J=7Hz), 1.4-2.4 (4H, m), 2.6-2.9 (4H, m), 2.9-3.8 (10H, m), 4.26 (4H, q, J=7Hz), 4.49 (2H, s), 4.52 (2H, s), 5.4-5.7 (2H, m), 6.5-6.6 (2H, m), 6.6-6.8 (2H, m), 6.97 (2H, d, J=8Hz), 7.2-7.5 (6H, m), 7.5-7.6 (2H, m), 8.1-8.4 (1H, m), 8.4-8.7 (1H, m), 10.0-10.2 (1H, m), 10.2-10.6 (1H, m)

FAB-MASS (m/z) : 420 ($MH^+$+2-HCl), 418 ($MH^+$-HCl)

2) (1R,2'R,3'R)- and (1R,2'S,3'S)-1-(3-Chlorophenyl)-2-[(7-ethoxycarbonylmethoxy-3-methyl-1,2,3,4-tetrahydro-2-naphthyl)amino]ethanol hydrochloride

IR (Film) : 3250, 3100-2300, 1730 $cm^{-1}$

NMR ($CDCl_3$, δ) : 1.1-1.4 (12H, m), 2.2-2.6 (4H, m), 2.8-3.6 (12H, m), 4.25 (4H, q, J=7Hz), 4.51 (6H, s), 5.3-5.6 (2H, m), 6.5-6.6 (2H, m), 6.7-6.8 (2H, m), 6.96 (2H, d, J=8Hz), 7.2-7.4 (6H, m), 7.46 (2H, br s), 8.0-8.3 (1H, m), 8.4-8.8 (1H, m), 9.8-10.1 (1H, m), 10.2-10.4 (1H, m)

FAB-MASS (m/z) : 420 ($MH^+$+2-HCl), 418 ($MH^+$-HCl)

Example 12

A solution of (R)-3-chlorostyrene oxide (111 mg) and 7-ethoxycarbonylmethoxy-2-methyl-1,2,3,4-tetrahydro-2-naphthylamine (190 mg) in ethanol (1.5 ml) was refluxed for 3 hours. After cooling, the solution was evaporated in vacuo. The residue was purified by column chromatography on silica gel with a mixture of chloroform and methanol (100:1) as an eluent to afford a mixture of (1R,2'R)- and (1R,2'S)-1-(3-chlorophenyl)-2-[(7-ethoxycarbonylmethoxy-2-methyl-1,2,3,4-tetrahydro-2-naphthyl)amino]ethanol (138 mg) as an oil. The obtained compound was dissolved in ethyl acetate and to the solution was added a solution of oxalic acid (26 mg) in ethyl acetate and the whole was evaporated in vacuo. The residue was triturated with diethyl ether and ethyl acetate to afford a mixture of (1R,2'R)- and (1R,2'S)-1-(3-chlorophenyl)-2-[(7-ethoxycarbonylmethoxy-2-methyl-1,2,3,4-tetrahydro-2-naphthyl)amino]ethanol oxalate.

mp : 127-140°C
IR (Nujol) : 3460, 3200, 2800-2300, 1755 $cm^{-1}$
NMR (DMSO-$d_6$, δ) : 1.21 (3H, t, J=7.1Hz), 1.24 (3H, s), 1.79-2.20 (2H, m), 2.57-3.45 (6H, m), 4.16 (2H, q, J=7.1Hz), 4.72 (2H, s), 4.98 (1H, d, J=9.2Hz), 5.60-8.50 (4H, br m), 6.64 (1H, s), 6.72 (1H, d, J=8.4Hz), 7.04 (1H, d, J=8.4Hz), 7.32-7.63 (4H, m)

Example 13

A mixture of 2-bromo-3'-chlorobutyrophenone (262 mg) and (S)-2-amino-7-ethoxycarbonylmethoxy-1,2,3,4-tetrahydronaphthalene (748 mg) in methanol (2 ml) was stirred at 45°C for 3.5 hours and the solution was standing at ambient temperature overnight. The solution was evaporated in vacuo and to the residue were added saturated sodium hydrogencarbonate and ethyl acetate. The organic layer was separated, washed with brine, dried over sodium sulfate, and evaporated in vacuo. The residue was purified by column chromatography on silica gel with a mixture of chloroform and methanol (1:0 - 20:1) as an eluent to afford 2-[(2S)-7-ethoxycarbonylmethoxy-1,2,3,4-tetrahydro-2-naphthyl)amino]-3'-chlorobutyrophenone (208 mg) as an oil.

IR (Film) : 1745, 1730 $cm^{-1}$

To a solution of the above obtained compound (198 mg) in ethanol (2 ml) was added sodium borohydride (17 mg) and stirred for 1 hour. To the solution was added 1N hydrochloric acid (3 ml) and the mixture was made alkaline with 1N sodium hydroxide solution. The mixture was evaporated in vacuo and brine and ethyl acetate were added to the residue. The organic layer was separated, washed with brine, dried over sodium sulfate, and evaporated in vacuo. The residue was purified by column chromatography on silica gel with a mixture of chloroform and methanol (300:1) as an eluent to afford a mixture of (1R,2'S)- and (1S,2'S)-1-(3-chlorophenyl)-2-[(7-ethoxycarbonylmethoxy-1,2,3,4-tetrahydro-2-naphthyl)amino]butanol (53 mg) as an oil. To a solution of the obtained compound (52 mg) in ethyl acetate was added a solution of oxalic acid (11 mg) in ethyl acetate and the whole was evaporated in vacuo. The residue was triturated with diethyl ether to afford a mixture of (1R,2'S)- and (1S,2'S)-1-(3-chlorophenyl)-2-[(7-ethoxycarbonylmethoxy-1,2,3,4-tetrahydro-2-naphthyl)amino]butanol oxalate.

mp : 159-164°C
IR (Nujol) : 3300, 1740, 1725 $cm^{-1}$

**Claims**

**Claims for the following Contracting States : AT, BE, CH, DE, DK, FR, GB, IT, LI, LU, NL, SE**

**1.** A compound of the formula :

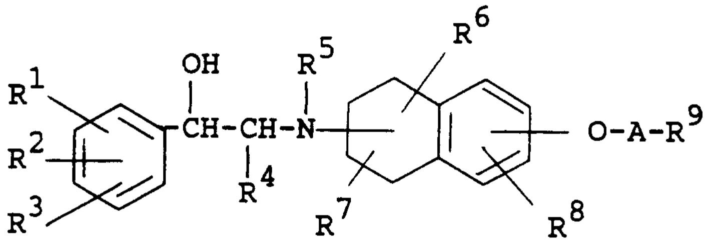

[I]

wherein

| | |
|---|---|
| $R^1$ is | hydrogen or halogen, |
| $R^2$ is | halogen, hydroxy, protected hydroxy, aryloxy, lower alkoxy, halo(lower)alkoxy, nitro, cyano, amino or acylamino, |
| $R^3$ is | hydrogen or halogen, |
| $R^4$ is | hydrogen or lower alkyl, |
| $R^5$ is | hydrogen or lower alkyl, |
| $R^6$ is | hydrogen, hydroxy or lower alkyl, |
| $R^7$ is | hydrogen or lower alkyl, |
| $R^8$ is | hydrogen or halogen, |
| $R^9$ is | carboxy or esterified carboxy, and |
| A is | lower alkylene, |

provided that $R^4$ is lower alkyl; $R^5$ is lower alkyl; $R^6$ is hydroxy or lower alkyl; or $R^8$ is halogen when $R^1$ is hydrogen, $R^2$ is halogen and $R^3$ is hydrogen,
and pharmaceutically acceptable salt thereof.

**2.** A compound according to claim 1,
wherein

| | |
|---|---|
| $R^4$ is | hydrogen and |
| $R^5$ is | hydrogen. |

**3.** A compound according to claim 2,
wherein

| | |
|---|---|
| $R^7$ is | hydrogen and |
| $R^8$ is | hydrogen. |

**4.** A compound according to claim 3,
wherein

| | |
|---|---|
| $R^1$ is | halogen, |
| $R^2$ is | halogen, hydroxy or protected hydroxy, |
| $R^3$ is | hydrogen, |
| $R^6$ is | hydrogen or hydroxy, and |
| $R^9$ is | lower alkoxycarbonyl. |

**5.** A compound of claim 4, which is (-)-1-(3-chloro-4-hydroxyphenyl)-2-[{(2S)-7-ethoxycarbonylmethoxy-1,2,3,4-tetrahydro-2-naphthyl}amino]ethanol or its hydrochloride.

**6.** A process for preparing a compound of the formula :

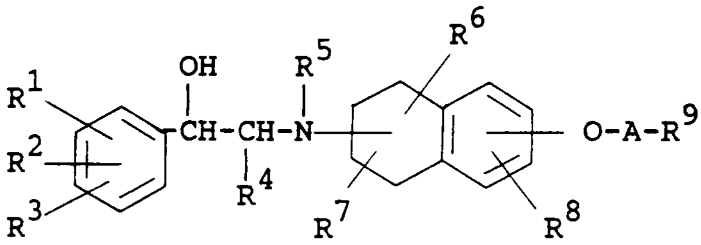

[I]

wherein

| | |
|---|---|
| $R^1$ is | hydrogen or halogen, |
| $R^2$ is | halogen, hydroxy, protected hydroxy, aryloxy, lower alkoxy, halo(lower)alkoxy, nitro, cyano, amino or acylamino, |
| $R^3$ is | hydrogen or halogen, |
| $R^4$ is | hydrogen or lower alkyl, |
| $R^5$ is | hydrogen or lower alkyl, |
| $R^6$ is | hydrogen, hydroxy or lower alkyl, |
| $R^7$ is | hydrogen or lower alkyl, |
| $R^8$ is | hydrogen or halogen, |
| $R^9$ is | carboxy or esterified carboxy, and |
| A is | lower alkylene, |

provided that $R^4$ is lower alkyl; $R^5$ is lower alkyl; $R^6$ is hydroxy or lower alkyl; or $R^8$ is halogen when $R^1$ is hydrogen, $R^2$ is halogen and $R^3$ is hydrogen,
or pharmaceutically acceptable salt thereof,
which comprises,

a) reacting a compound of the formula :

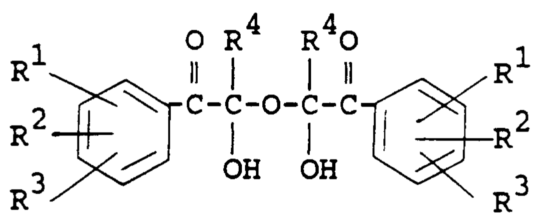

[II]

or its salt with a compound of the formula :

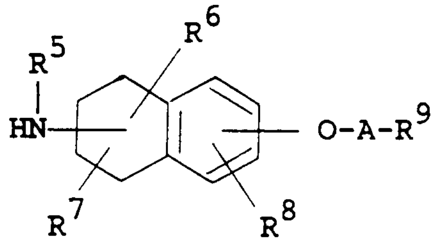

[III]

or its salt in the presence of a reducing agent to provide a compound of the formula :

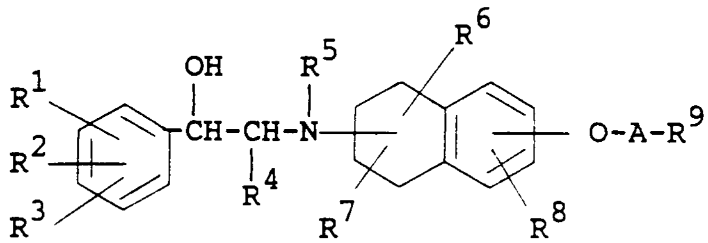

[I]

or its salt, in the above formulas, $R^1$, $R^2$, $R^3$, $R^4$, $R^5$, $R^6$, $R^7$, $R^8$, $R^9$ and A are each as defined above, or

b) reacting a compound of the formula :

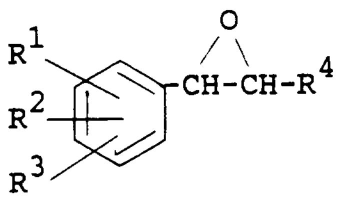

[IV]

or its salt with a compound of the formula :

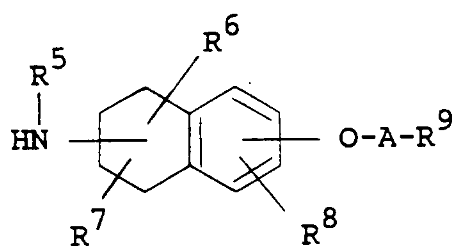

[III]

or its salt to provide a compound of the formula :

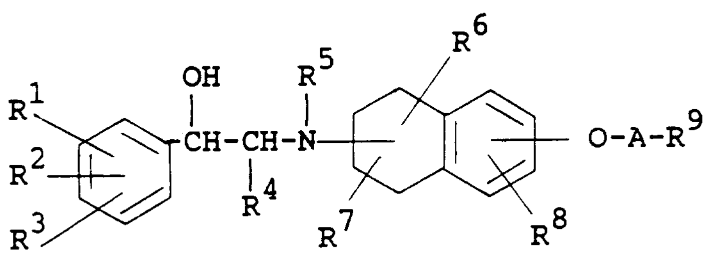

[I]

or its salt, in the above formulas, $R^1$, $R^2$, $R^3$, $R^4$, $R^5$, $R^6$, $R^7$, $R^8$, $R^9$ and A are each as defined above, or

c) subjecting a compound of the formula :

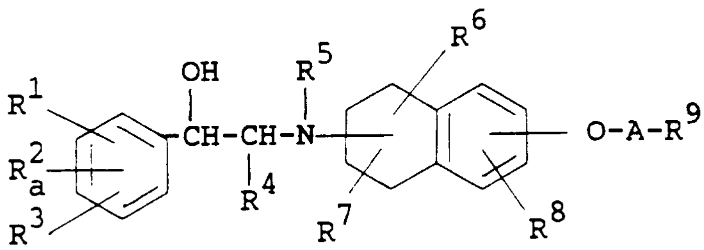

[Ia]

or its salt to elimination reaction of the hydroxy-protective group to provide a compound of the formula :

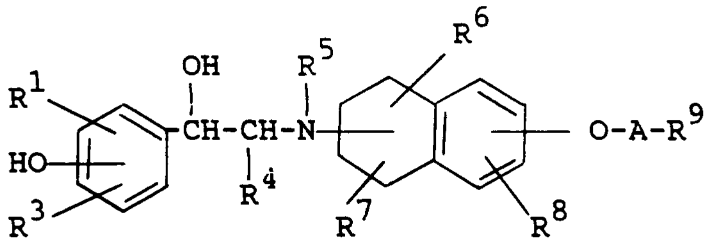

[Ib]

or its salt, in the above formulas, $R^2_a$ is protected hydroxy, and $R^1$, $R^3$, $R^4$, $R^5$, $R^6$, $R^7$, $R^8$, $R^9$ and A are each as defined above, or

d) subjecting a compound of the formula :

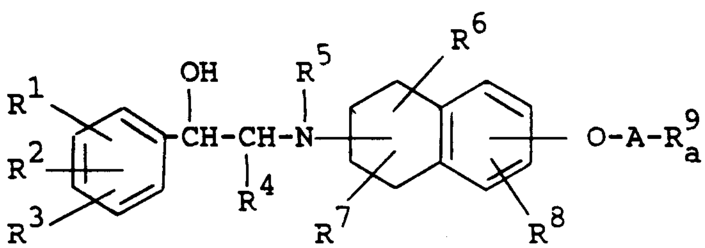

[Ic]

or its salt to deesterification reaction to provide a compound of the formula :

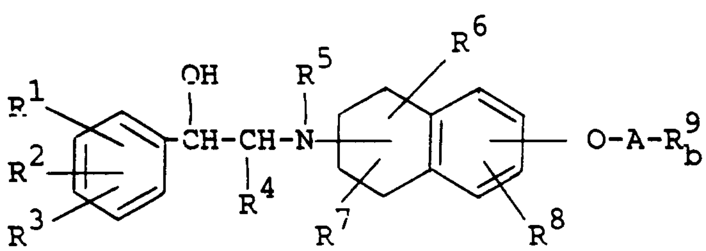

[Id]

or its salt, in the above formulas,

$R^1$, $R^2$, $R^3$, $R^4$, $R^5$ $R^6$, $R^7$, $R^8$ and A are each as defined above,
$R^9_a$ is esterified carboxy, and
$R^9_b$ is carboxy, or

e) reacting a compound of the formula :

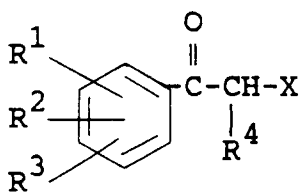

[V]

or its salt with a compound of the formula :

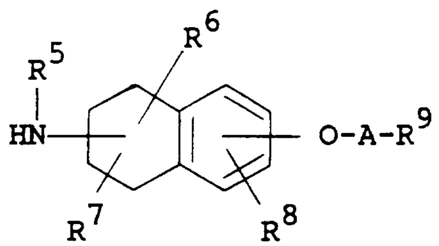

[III]

or its salt, and then subjecting the resultant product to reduction to provide a compound of the formula :

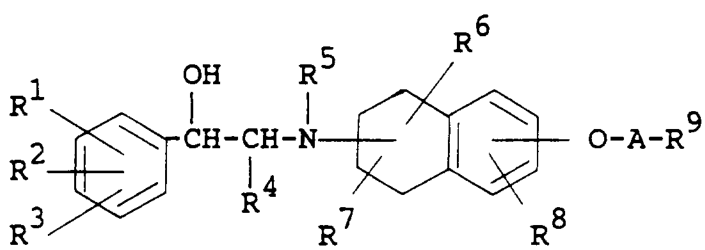

[I]

or its salt, in the above formulas,

$R^1$, $R^2$, $R^3$, $R^4$, $R^5$, $R^6$, $R^7$, $R^8$, $R^9$ and A are each as defined above, and X is halogen.

**7.** A pharmaceutical composition comprising a compound of claim 1, as an active ingredient, in association with a pharmaceutically acceptable, substantially non-toxic carrier or excipient.

**8.** A compound of claim 1 for use as a medicament.

**9.** Use of a compound of claim 1 for the manufacture of a medicament for the treatment and/or prevention of dysuria, spasm or hyperanakinesis in human beings or animals.

**Claims for the following Contracting State : ES**

**1.** A process for preparing a compound of the formula :

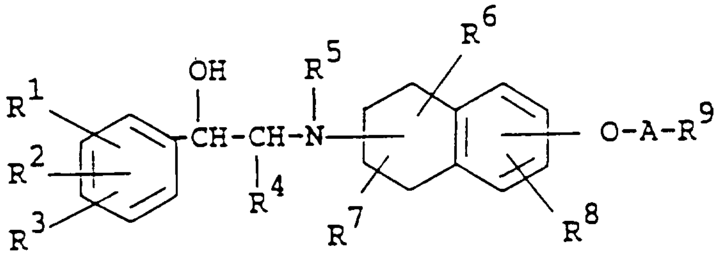

[I]

wherein

- $R^1$ is hydrogen or halogen,
- $R^2$ is halogen, hydroxy, protected hydroxy, aryloxy, lower alkoxy, halo(lower)alkoxy, nitro, cyano, amino or acylamino,
- $R^3$ is hydrogen or halogen,
- $R^4$ is hydrogen or lower alkyl,
- $R^5$ is hydrogen or lower alkyl,
- $R^6$ is hydrogen, hydroxy or lower alkyl,
- $R^7$ is hydrogen or lower alkyl,
- $R^8$ is hydrogen or halogen,
- $R^9$ is carboxy or esterified carboxy, and
- A is lower alkylene,

provided that $R^4$ is lower alkyl; $R^5$ is lower alkyl; $R^6$ is hydroxy or lower alkyl; or $R^8$ is halogen when $R^1$ is hydrogen, $R^2$ is halogen and $R^3$ is hydrogen,
or pharmaceutically acceptable salt thereof,
which comprises,

a) reacting a compound of the formula :

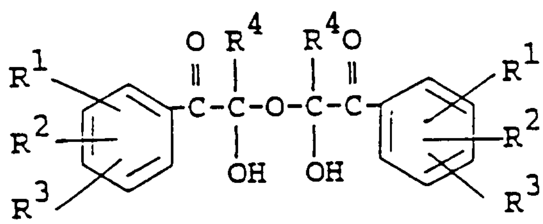

[II]

or its salt with a compound of the formula :

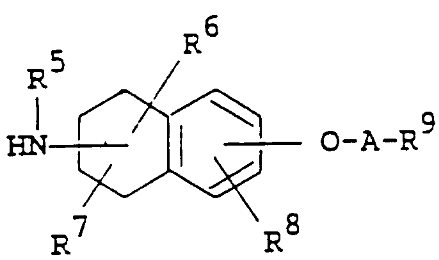

[III]

or its salt in the presence of a reducing agent to provide a compound of the formula :

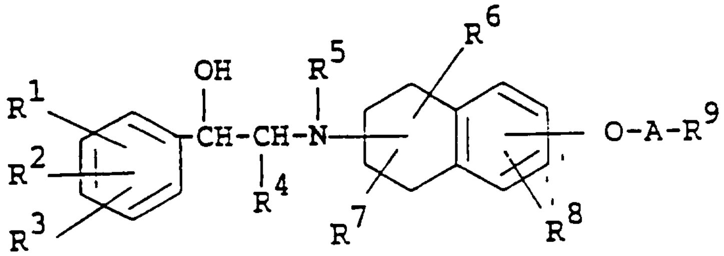

[I]

or its salt, in the above formulas, $R^1$, $R^2$, $R^3$, $R^4$, $R^5$, $R^6$, $R^7$, $R^8$, $R^9$ and A are each as defined above, or

b) reacting a compound of the formula :

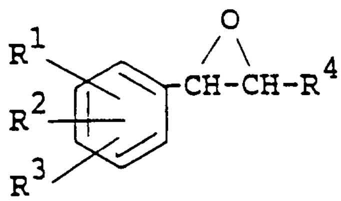

[IV]

or its salt with a compound of the formula :

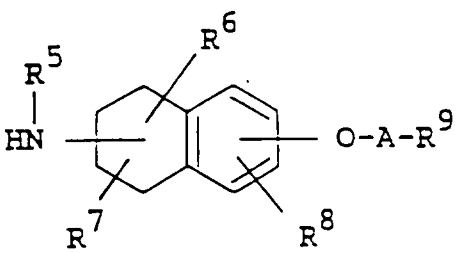

[III]

or its salt to provide a compound of the formula :

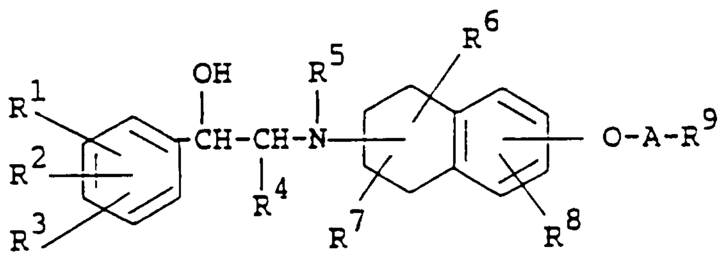

[I]

or its salt, in the above formulas, $R^1$, $R^2$, $R^3$, $R^4$, $R^5$, $R^6$, $R^7$, $R^8$, $R^9$ and A are each as defined above, or

c) subjecting a compound of the formula :

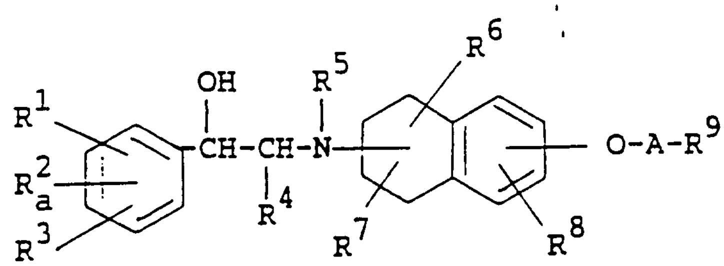

[Ia]

or its salt to elimination reaction of the hydroxy-protective group to provide a compound of the formula :

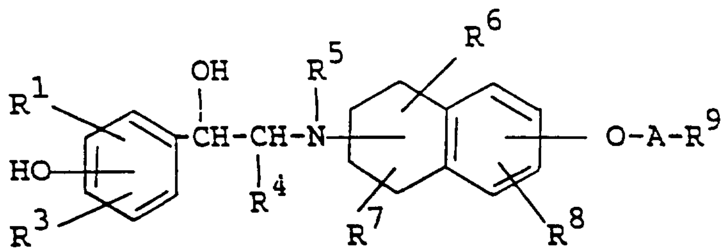

[Ib]

or its salt, in the above formulas, $R^2_a$ is protected hydroxy, and $R^1$, $R^3$, $R^4$, $R^5$, $R^6$, $R^7$, $R^8$, $R^9$ and A are each as defined above, or

d) subjecting a compound of the formula :

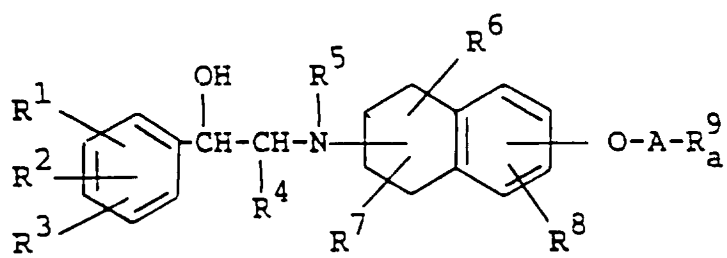

[Ic]

or its salt to deesterification reaction to provide a compound of the formula :

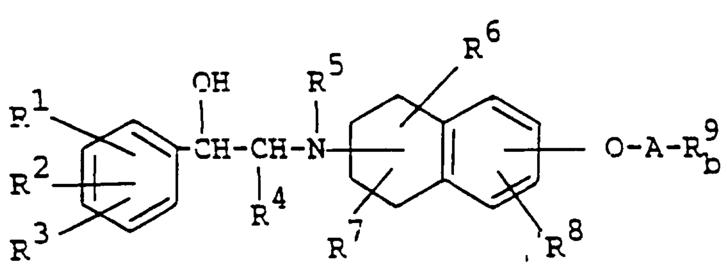

[Id]

or its salt, in the above formulas,

$R^1$, $R^2$, $R^3$, $R^4$, $R^5$, $R^6$, $R^7$, $R^8$ and A are each as defined above,
$R^9_a$ is esterified carboxy, and

$R^9_b$ is carboxy, or

e) reacting a compound of the formula :

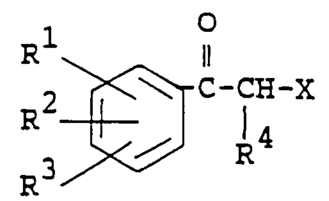

[V]

or its salt with a compound of the formula :

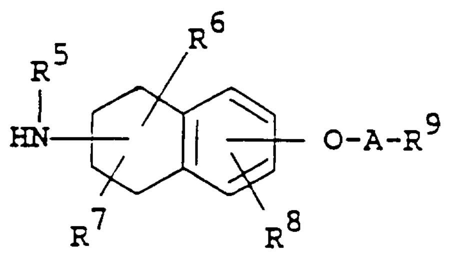

[III]

or its salt, and then subjecting the resultant product to reduction to provide a compound of the formula :

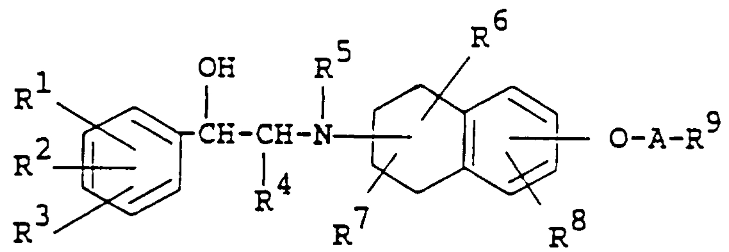

[I]

or its salt, in the above formulas,

$R^1$, $R^2$, $R^3$, $R^4$, $R^5$, $R^6$, $R^7$, $R^8$, $R^9$ and A are each as defined above, and X is halogen.

**2.** A process according to claim 1, wherein

$R^4$ is hydrogen and
$R^5$ is hydrogen.

**3.** A process according to claim 2, wherein

$R^7$ is hydrogen and
$R^8$ is hydrogen.

**4.** A process according to claim 3, wherein

$R^1$ is halogen,

$R^2$ is halogen, hydroxy or protected hydroxy,
$R^3$ is hydrogen,
$R^6$ is hydrogen or hydroxy, and
$R^9$ is lower alkoxycarbonyl.

5. A process for preparing a compound of claim 4, which is (-)-1-(3-chloro-4-hydroxyphenyl)-2-[{(2S)-7-ethoxycarbonylmethoxy-1,2,3,4-tetrahydro-2-naphthyl}amino]ethanol or its hydrochloride.

6. A process for preparing a pharmaceutical composition which comprises, as an active ingredient, a compound of claim 1 or a pharmaceutically acceptable salt thereof characterized by mixing a compound of claim 1 with at least one pharmaceutically acceptable carrier or excipient.

7. A process of claim 6, wherein the pharmaceutical composition is for the treatment and /or prevention of dysuria, spasm or hyperanakinesis in human beings or animals.

**Claims for the following Contracting State : GR**

1. A process for preparing a compound of the formula :

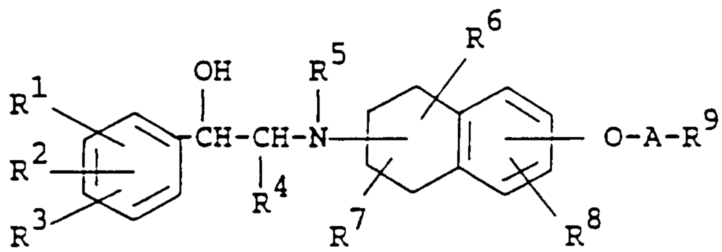

[I]

wherein

$R^1$ is hydrogen or halogen,
$R^2$ is halogen, hydroxy, protected hydroxy, aryloxy, lower alkoxy, halo(lower)alkoxy, nitro, cyano, amino or acylamino,
$R^3$ is hydrogen or halogen,
$R^4$ is hydrogen or lower alkyl,
$R^5$ is hydrogen or lower alkyl,
$R^6$ is hydrogen, hydroxy or lower alkyl,
$R^7$ is hydrogen or lower alkyl,
$R^8$ is hydrogen or halogen,
$R^9$ is carboxy or esterified carboxy, and
A is lower alkylene,

provided that $R^4$ is lower alkyl; $R^5$ is lower alkyl; $R^6$ is hydroxy or lower alkyl;
or $R^8$ is halogen when $R^1$ is hydrogen, $R^2$ is halogen and $R^3$ is hydrogen,
or pharmaceutically acceptable salt thereof,
which comprises,

a) reacting a compound of the formula :

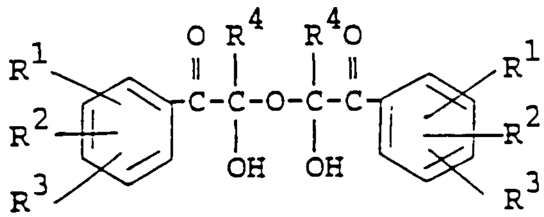

[II]

or its salt with a compound of the formula :

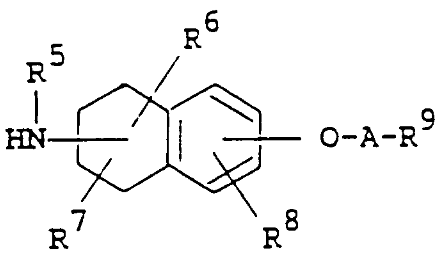

[III]

or its salt in the presence of a reducing agent to provide a compound of the formula :

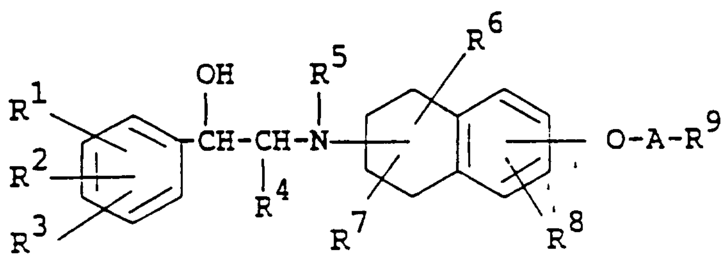

[I]

or its salt, in the above formulas, $R^1$, $R^2$, $R^3$, $R^4$, $R^5$, $R^6$, $R^7$, $R^8$, $R^9$ and A are each as defined above, or

b) reacting a compound of the formula :

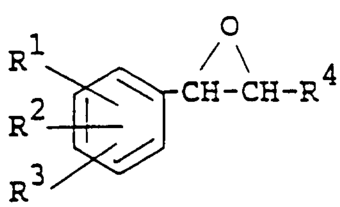

[IV]

or its salt with a compound of the formula :

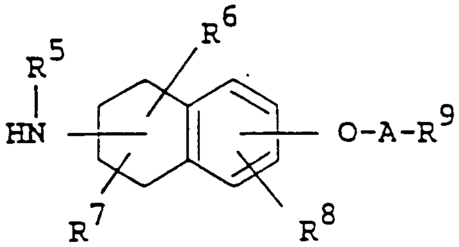

[III]

or its salt to provide a compound of the formula :

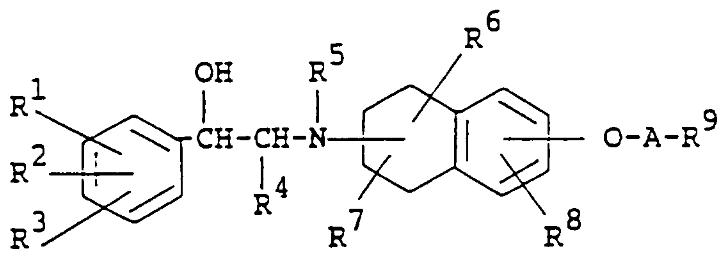

[I]

or its salt, in the above formulas, $R^1$, $R^2$, $R^3$, $R^4$, $R^5$, $R^6$, $R^7$, $R^8$, $R^9$ and A are each as defined above, or

c) subjecting a compound of the formula :

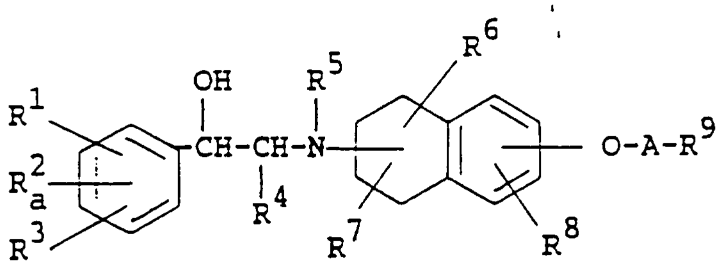

[Ia]

or its salt to elimination reaction of the hydroxy-protective group to provide a compound of the formula :

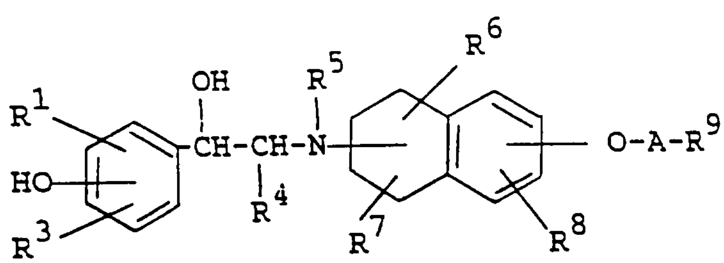

[Ib]

or its salt, in the above formulas, $R^2_a$ is protected hydroxy, and $R^1$, $R^3$, $R^4$, $R^5$, $R^6$, $R^7$, $R^8$, $R^9$ and A are each as defined above, or

d) subjecting a compound of the formula :

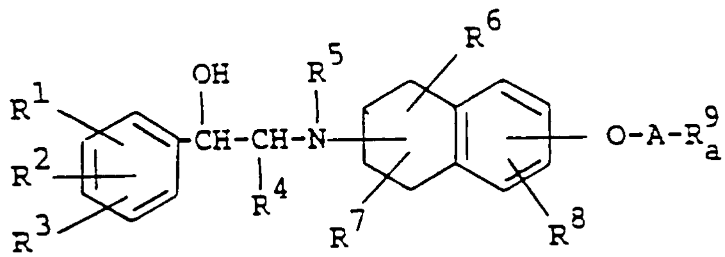

[Ic]

or its salt to deesterification reaction to provide a compound of the formula :

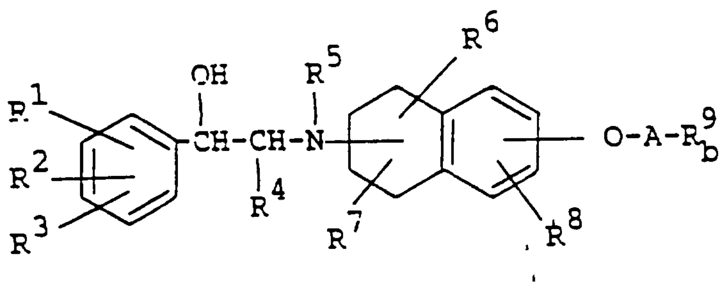

[Id]

or its salt, in the above formulas,

$R^1$, $R^2$, $R^3$, $R^4$, $R^5$, $R^6$, $R^7$, $R^8$ and A are each as defined above,
$R^9_a$ is esterified carboxy, and
$R^9_b$ is carboxy, or

e) reacting a compound of the formula :

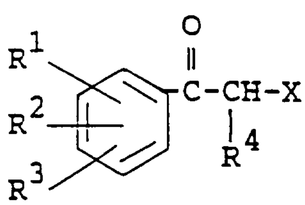

[V]

or its salt with a compound of the formula :

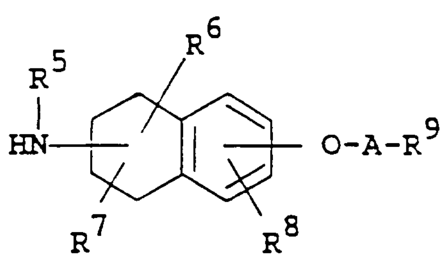

[III]

or its salt, and then subjecting the resultant product to reduction to provide a compound of the formula :

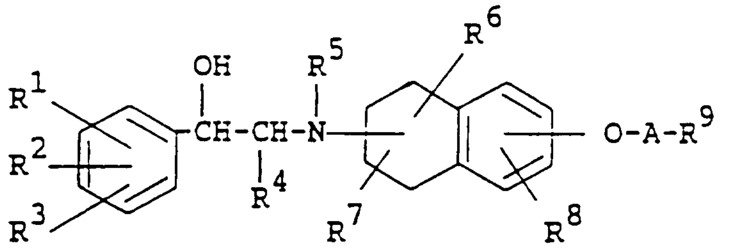 [I]

or its salt, in the above formulas,

$R^1$, $R^2$, $R^3$, $R^4$, $R^5$ $R^6$, $R^7$, $R^8$, $R^9$ and A are each as defined above, and X is halogen.

**2.** A process according to claim 1, wherein

$R^4$ is hydrogen and
$R^5$ is hydrogen.

**3.** A process according to claim 2, wherein

$R^7$ is hydrogen and
$R^8$ is hydrogen.

**4.** A process according to claim 3, wherein

$R^1$ is halogen,
$R^2$ is halogen, hydroxy or protected hydroxy,
$R^3$ is hydrogen,
$R^6$ is hydrogen or hydroxy, and
$R^9$ is lower alkoxycarbonyl.

**5.** A process for preparing a compound of claim 4, which is (-)-1-(3-chloro-4-hydroxyphenyl)-2-[{(2S)-7-ethoxycarbonylmethoxy-1,2,3,4-tetrahydro-2-naphthyl}amino]ethanol or its hydrochloride.

**6.** A process for preparing a pharmaceutical composition which comprises, as an active ingredient, a compound of claim 1 or a pharmaceutically acceptable salt thereof characterized by mixing a compound of claim 1 with at least one pharmaceutically acceptable carrier or excipient.

**7.** A process of claim 6, wherein the pharmaceutical composition is for the treatment and/or prevention of dysuria, spasm or hyperanakinesis in human beings or animals.

**8.** Modification of the process claimed in claim 1 which is characterized by bringing a compound of the formula I or a non-toxic salt thereof, produced by a process claimed in claim 1, into pharmaceutically acceptable form by admixture or presentation of said compound with a pharmaceutically acceptable diluent or carrier.

**Patentansprüche**

**Patentansprüche für folgende Vertragsstaaten : AT, BE, CH, DE, DK, FR, GB, IT, LI, LU, NL, SE**

**1.** Eine Verbindung der Formel:

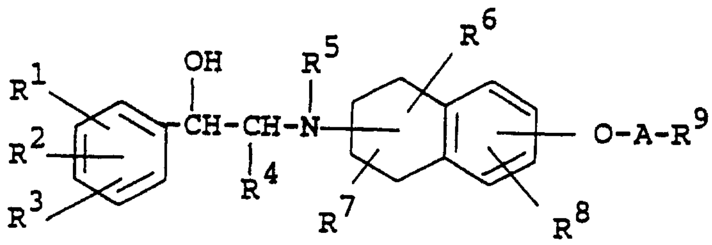

[I]

wobei

$R^1$ Wasserstoff oder Halogen ist,
$R^2$ Halogen, Hydroxy, geschütztes Hydroxy, niedriges Alkoxy, Halo(niedrig)alkoxy, Nitro, Cyano, Amino oder Acylamino ist,
$R^3$ Wasserstoff oder Halogen ist,
$R^4$ Wasserstoff oder niedriges Alkyl ist,
$R^5$ Wasserstoff oder niedriges Alkyl ist,
$R^6$ Wasserstoff, Hydroxy oder niedriges Alkyl ist,
$R^7$ Wasserstoff oder niedriges Alkyl ist,
$R^8$ Wasserstoff oder Halogen ist,
$R^9$ Carboxy oder verestertes Carboxy ist, und
A niedriges Alkylen ist,

vorausgesetzt, daß $R^4$ niedriges Alkyl ist; $R^5$ niedriges Alkyl ist;
$R^6$ Hydroxy oder niedriges Alkyl ist; oder $R^8$ Halogen wenn $R^1$ Wasserstoff ist, $R^2$ Halogen ist und $R^3$ Wasserstoff ist,
und pharmazeutisch verträgliche Salze davon.

2. Verbindung nach Anspruch 1, wobei

$R^4$ Wasserstoff ist und
$R^5$ Wasserstoff ist.

3. Verbindung nach Anspruch 2, wobei

$R^7$ Wasserstoff ist und
$R^8$ Wasserstoff ist.

4. Verbindung nach Anspruch 3, wobei

$R^1$ Halogen ist,
$R^2$ Halogen, Hydroxy oder geschütztes Hydroxy ist,
$R^3$ Wasserstoff ist,
$R^6$ Wasserstoff oder Hydroxy ist, und
$R^9$ niedriges Alkoxycarbonyl ist.

5. Verbindung nach Anspruch 4, welche (-)-1-(3-Chlor-4-hydroxyphenyl)-2-[{(2S)-7-ethoxycarbonylmethoxy-1,2,3,4-tetrahydro-2-naphthyl}amino]ethanol oder ihr Hydrochlorid ist.

6. Verfahren zur Herstellung einer Verbindung der Formel:

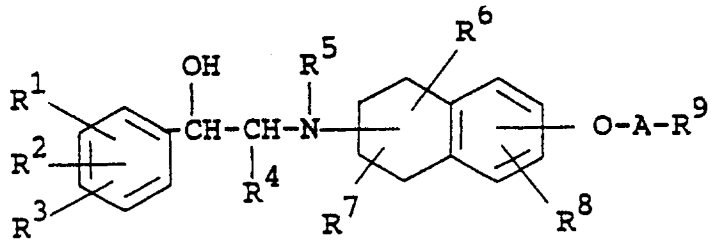

[I]

wobei

$R^1$ Wasserstoff oder Halogen ist,
$R^2$ Halogen, Hydroxy, geschütztes Hydroxy, Aryloxy, niedriges Alkoxy, Halo(niedrig)alkoxy, Nitro, Cyano, Amino oder Acylamino ist,
$R^3$ Wasserstoff oder Halogen ist,
$R^4$ Wasserstoff oder niedriges Alkyl ist,
$R^5$ Wasserstoff oder niedriges Alkyl ist,
$R^6$ Wasserstoff, Hydroxy oder niedriges Alkyl ist,
$R^7$ Wasserstoff oder niedriges Alkyl ist,
$R^8$ Wasserstoff oder Halogen ist,
$R^9$ Carboxy oder verestertes Carboxy ist, und
A niedriges Alkylen ist,

vorausgesetzt, daß $R^4$ niedriges Alkyl ist; $R^5$ niedriges Alkyl ist;
$R^6$ Hydroxy oder niedriges Alkyl ist; oder $R^8$ Halogen ist, wenn $R^1$ Wasserstoff ist, $R^2$ Halogen ist und $R^3$ Wasserstoff ist,
oder eines pharmazeutisch verträglichen Salzes davon, welches umfaßt

a) Umsetzung einer Verbindung der Formel:

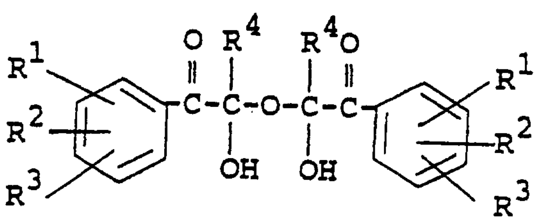

[II]

oder ihres Salzes mit einer Verbindung der Formel:

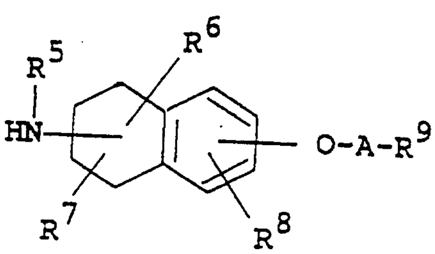

[III]

oder ihres Salzes in Gegenwart eines Reduktionsmittels zur Herstellung einer Verbindung der Formel:

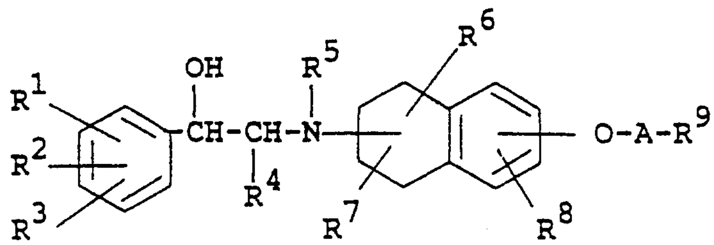

[I]

oder ihres Salzes, wobei in den obigen Formeln $R^1$, $R^2$, $R^3$, $R^4$, $R^5$, $R^6$, $R^7$, $R^8$, $R^9$ und A jeweils wie oben definiert sind, oder

b) Umsetzung einer Verbindung der Formel:

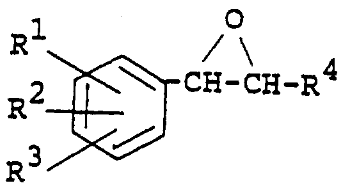

[IV]

oder ihres Salzes mit einer Verbindung der Formel:

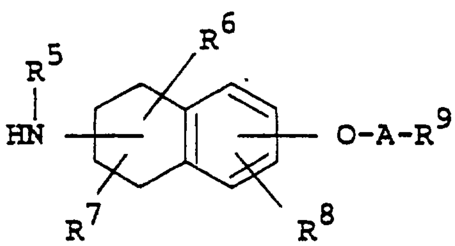

[III]

oder ihres Salzes zur Herstellung einer Verbindung der Formel:

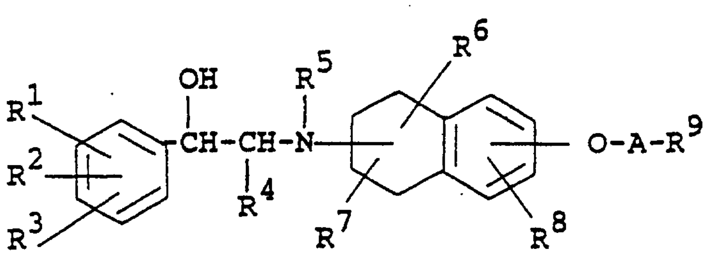

[I]

oder ihres Salzes, wobei in den obigen Formeln $R^1$, $R^2$, $R^3$, $R^4$, $R^5$, $R^6$, $R^7$, $R^8$, $R^9$ und A jeweils wie oben definiert sind, oder

c) Durchführung einer Eliminierungsreaktion der Hydroxyschutzgruppe mit einer Verbindung der Formel:

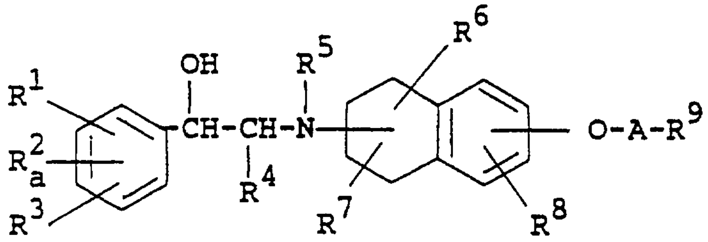

[Ia]

oder ihres Salzes zur Herstellung einer Verbindung der Formel:

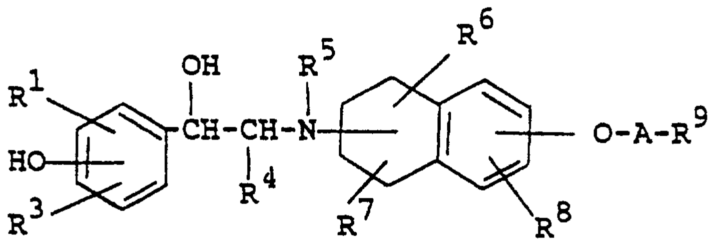

[Ib]

oder ihres Salzes, wobei in den obigen Formeln $R^2_a$ geschütztes Hydroxy ist und $R^1$, $R^3$, $R^4$, $R^5$, $R^6$, $R^7$, $R^8$, $R^9$ und A jeweils wie oben definiert sind, oder

d) Durchführung einer Verseifungsreaktion mit einer Verbindung der Formel:

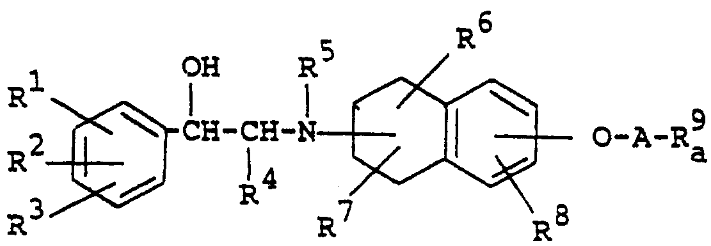

[Ic]

oder ihres Salzes zur Herstellung einer Verbindung der Formel:

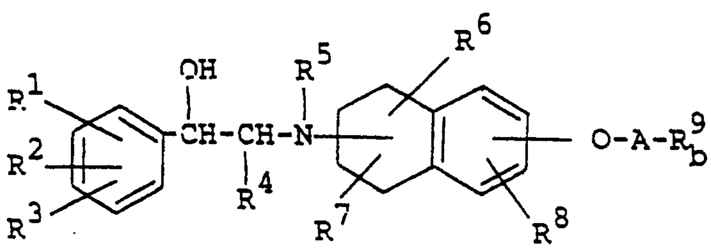

[Id]

oder ihres Salzes, wobei in den obigen Formeln

$R^1$, $R^2$, $R^3$, $R^4$, $R^5$, $R^6$, $R^7$, $R^8$ und A jeweils wie oben definiert sind,
$R^9_a$ verestertes Carboxy ist, und
$R^9_b$ Carboxy ist oder

e) Umsetzung einer Verbindung der Formel:

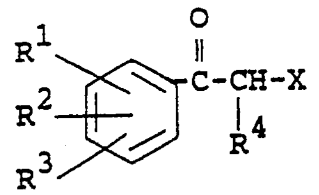

[V]

oder ihres Salzes mit einer Verbindung der Formel:

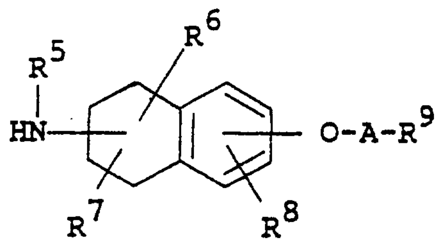

[III]

oder ihres Salzes, und anschließende Durchführung einer Reduktion mit dem resultierenden Produkt zur Herstellung einer Verbindung der Formel:

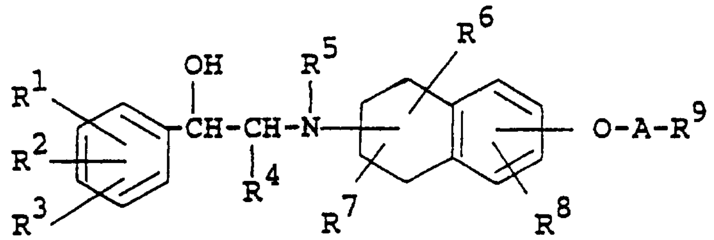

[I]

oder ihres Salzes, wobei in den obigen Formeln $R^1$, $R^2$, $R^3$, $R^4$, $R^5$, $R^6$, $R^7$, $R^8$, $R^9$ und A jeweils wie oben definiert sind und X Halogen ist.

7. Pharmazeutische Zusammensetzung, umfassend eine Verbindung nach Anspruch 1 als aktiven Bestandteil in Verbindung mit einem pharmazeutisch verträglichen, im wesentlichen nicht-toxischen Träger oder Excipienten.

8. Verbindung nach Anspruch 1 zur Verwendung als Medikament.

9. Verwendung einer Verbindung nach Anspruch 1 für die Herstellung eines Medikaments für die Behandlung und/oder Vorbeugung von Dysurie, Spasmen oder Hyperanakinesie bei Menschen oder Tieren.

**Patentansprüche für folgenden Vertragsstaat : ES**

1. Verfahren zur Herstellung einer Verbindung der Formel:

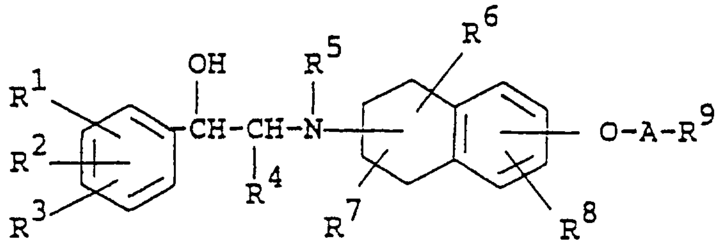

[I]

wobei

$R^1$ Wasserstoff oder Halogen ist,
$R^2$ Halogen, Hydroxy, geschütztes Hydroxy, Aryloxy, niedriges Alkoxy, Halo(niedrig)alkoxy, Nitro, Cyano, Amino oder Acylamino ist,
$R^3$ Wasserstoff oder Halogen ist,
$R^4$ Wasserstoff oder niedriges Alkyl ist,
$R^5$ Wasserstoff oder niedriges Alkyl ist,
$R^6$ Wasserstoff, Hydroxy oder niedriges Alkyl ist,
$R^7$ Wasserstoff oder niedriges Alkyl ist,
$R^8$ Wasserstoff oder Halogen ist,
$R^9$ Carboxy oder verestertes Carboxy ist, und
A niedriges Alkylen ist,

vorausgesetzt, daß $R^4$ niedriges Alkyl ist; $R^5$ niedriges Alkyl ist;
$R^6$ Hydroxy oder niedriges Alkyl ist; oder $R^8$ Halogen ist, wenn $R^1$ Wasserstoff ist, $R^2$ Halogen ist und $R^3$ Wasserstoff ist,
oder eines pharmazeutisch verträglichen Salzes davon, welches umfaßt

a) Umsetzung einer Verbindung der Formel:

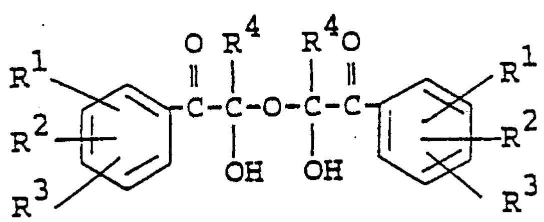

[II]

oder ihres Salzes mit einer Verbindung der Formel:

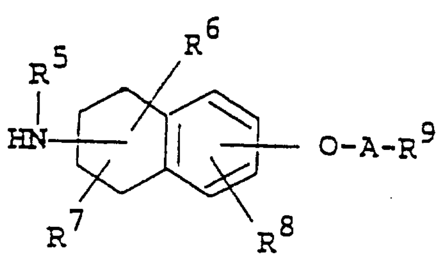

[III]

oder ihres Salzes in Gegenwart eines Reduktionsmittels zur Herstellung einer Verbindung der Formel:

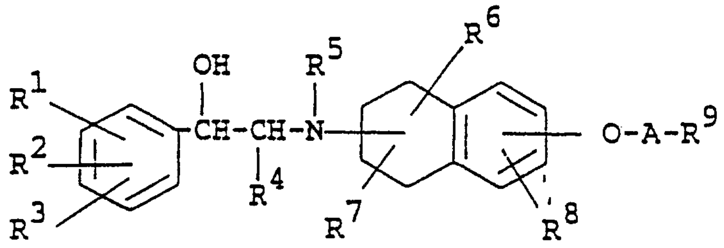

[I]

oder ihres Salzes, wobei in den obigen Formeln $R^1$, $R^2$, $R^3$, $R^4$, $R^5$, $R^6$, $R^7$, $R^8$, $R^9$ und A jeweils wie oben definiert sind, oder

b) Umsetzung einer Verbindung der Formel:

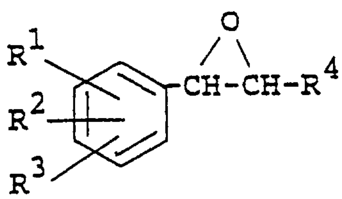

[IV]

oder ihres Salzes mit einer Verbindung der Formel:

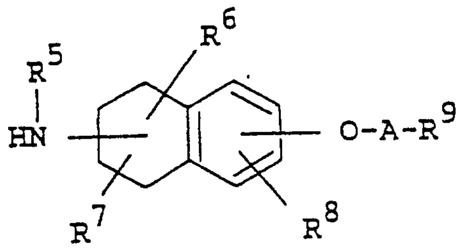

[III]

oder ihres Salzes zur Herstellung einer Verbindung der Formel:

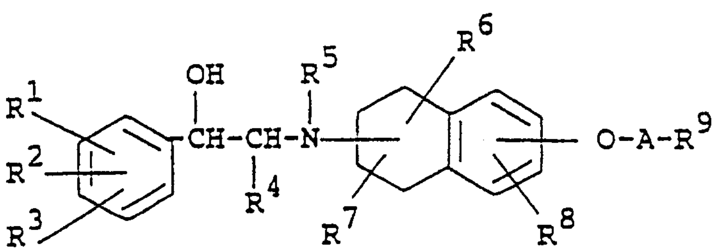

[I]

oder ihres Salzes, wobei in den obigen Formeln $R^1$, $R^2$, $R^3$, $R^4$, $R^5$, $R^6$, $R^7$, $R^8$, $R^9$ und A jeweils wie oben definiert sind, oder

c) Durchführung einer Eliminierungsreaktion der Hydroxyschutzgruppe mit einer Verbindung der Formel:

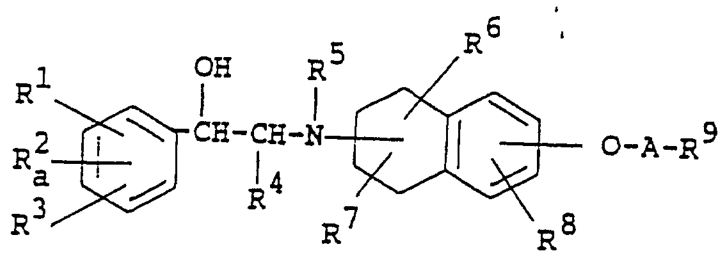

[Ia]

oder ihres Salzes zur Herstellung einer Verbindung der Formel:

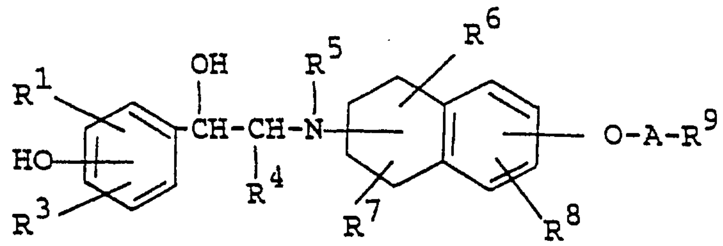

[Ib]

oder ihres Salzes, wobei in den obigen Formeln $R^2_a$ geschütztes Hydroxy ist und $R^1$, $R^3$, $R^4$, $R^5$, $R^6$, $R^7$, $R^8$, $R^9$ und A jeweils wie oben definiert sind, oder

d) Durchführung einer Verseifungsreaktion mit einer Verbindung der Formel:

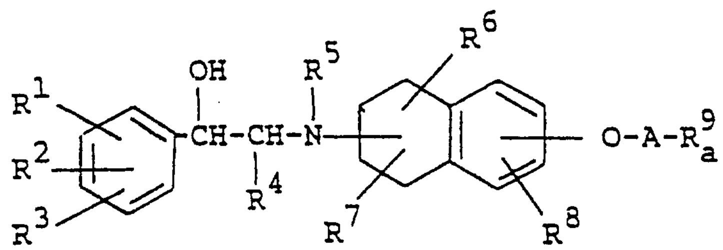

[Ic]

oder ihres Salzes zur Herstellung einer Verbindung der Formel:

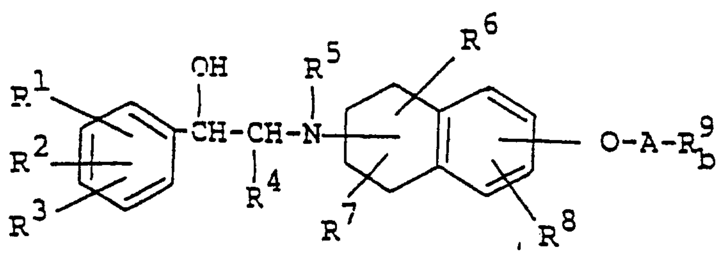

[Id]

oder ihres Salzes, wobei in den obigen Formeln

$R^1$, $R^2$, $R^3$, $R^4$, $R^5$, $R^6$, $R^7$, $R^8$ und A jeweils wie oben definiert sind,
$R^9_a$ verestertes Carboxy ist, und
$R^9_b$ Carboxy ist oder

e) Umsetzung einer Verbindung der Formel:

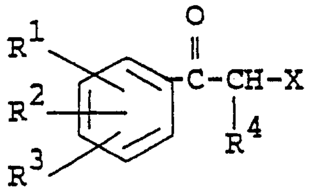

[V]

oder ihres Salzes mit einer Verbindung der Formel:

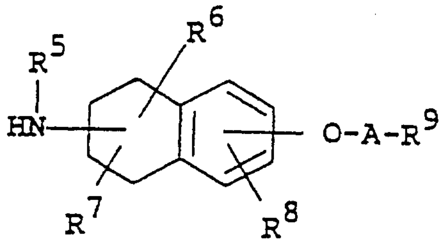

[III]

oder ihres Salzes, und anschließende Durchführung einer Reduktion mit dem resultierenden Produkt zur Herstellung einer Verbindung der Formel:

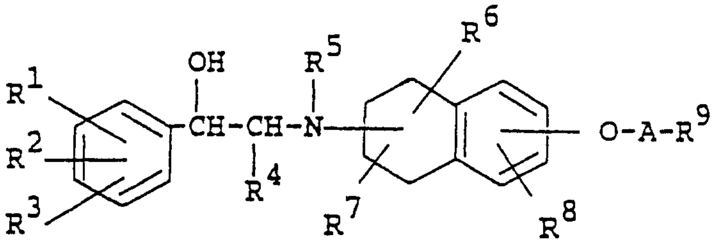

[I]

oder ihres Salzes, wobei in den obigen Formeln $R^1$, $R^2$, $R^3$, $R^4$, $R^5$, $R^6$, $R^7$, $R^8$, $R^9$ und A jeweils wie oben definiert sind und X Halogen ist.

2. Verfahren nach Anspruch 1, wobei

   $R^4$ Wasserstoff ist und
   $R^5$ Wasserstoff ist.

3. Verfahren nach Anspruch 2, wobei

   $R^7$ Wasserstoff ist und
   $R^8$ Wasserstoff ist.

4. Verfahren nach Anspruch 3, wobei

   $R^1$ Halogen ist,
   $R^2$ Halogen, Hydroxy oder geschütztes Hydroxy ist,
   $R^3$ Wasserstoff ist,
   $R^6$ Wasserstoff oder Hydroxy ist, und
   $R^9$ niedriges Alkoxycarbonyl ist.

5. Verfahren zur Herstellung einer Verbindung nach Anspruch 4, welche (-)-1-(3-Chlor-4-hydroxyphenyl)-2-[{(2S)-7-ethoxycarbonylmethoxy-1,2,3,4-tetrahydro-2-naphthyl)amino]ethanol oder ihr Hydrochlorid ist.

6. Verfahren zur Herstellung einer pharmazeutischen Zusammensetzung, welche als aktiven Bestandteil eine Verbindung nach Anspruch 1 oder ein pharmazeutisch verträgliches Salz davon umfaßt, charakterisiert durch Mischen einer Verbindung nach Anspruch 1 mit mindestens einem pharmazeutisch verträglichen Träger oder Excipienten.

7. Verfahren nach Anspruch 6, wobei die pharmazeutische Zusammensetzung für die Behandlung und/oder Vorbeugung von Dysurie, Spasmen oder Hyperanakinesie beim Menschen oder bei Tieren vorgesehen ist.

**Patentansprüche für folgenden Vertragsstaat : GR**

1. Verfahren zur Herstellung einer Verbindung der Formel:

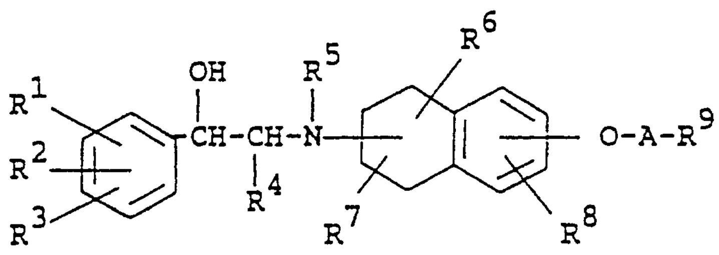

[I]

wobei

$R^1$ Wasserstoff oder Halogen ist,
$R^2$ Halogen, Hydroxy, geschütztes Hydroxy, Aryloxy, niedriges Alkoxy, Halo(niedrig)alkoxy, Nitro, Cyano, Amino oder Acylamino ist,
$R^3$ Wasserstoff oder Halogen ist,
$R^4$ Wasserstoff oder niedriges Alkyl ist,
$R^5$ Wasserstoff oder niedriges Alkyl ist,
$R^6$ Wasserstoff, Hydroxy oder niedriges Alkyl ist,
$R^7$ Wasserstoff oder niedriges Alkyl ist,
$R^8$ Wasserstoff oder Halogen ist,
$R^9$ Carboxy oder verestertes Carboxy ist, und
A niedriges Alkylen ist,

vorausgesetzt, daß $R^4$ niedriges Alkyl ist; $R^5$ niedriges Alkyl ist; $R^6$ Hydroxy oder niedriges Alkyl ist; oder $R^8$ Halogen ist, wenn $R^1$ Wasserstoff ist, $R^2$ Halogen ist und $R^3$ Wasserstoff ist,
oder eines pharmazeutisch verträglichen Salzes davon, welches umfaßt

a) Umsetzung einer Verbindung der Formel:

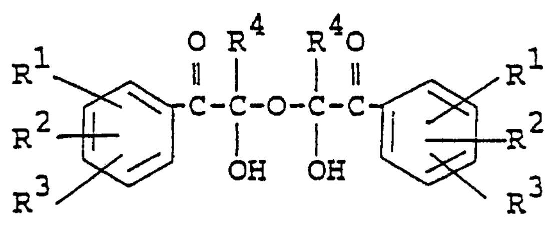

[II]

oder ihres Salzes mit einer Verbindung der Formel:

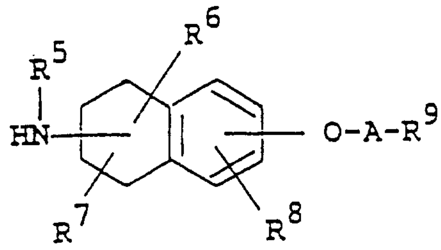

[III]

oder ihres Salzes in Gegenwart eines Reduktionsmittels zur Herstellung einer Verbindung der Formel:

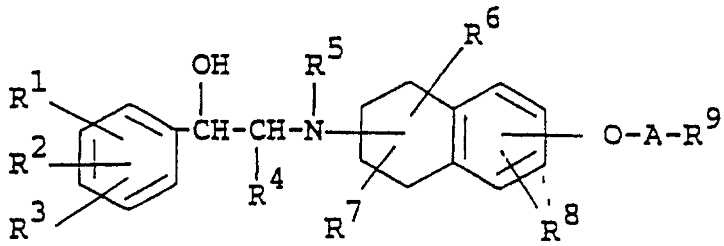

[I]

oder ihres Salzes, wobei in den obigen Formeln $R^1$, $R^2$, $R^3$, $R^4$, $R^5$, $R^6$, $R^7$, $R^8$, $R^9$ und A jeweils wie oben definiert sind, oder

b) Umsetzung einer Verbindung der Formel:

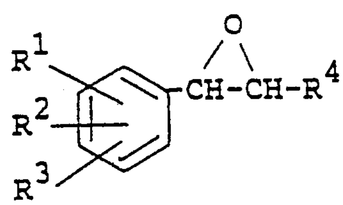

[IV]

oder ihres Salzes mit einer Verbindung der Formel:

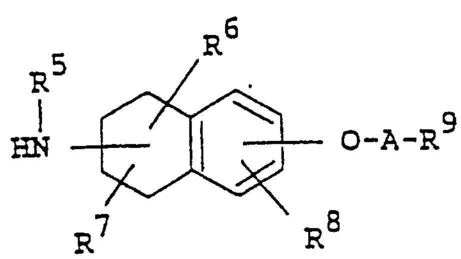

[III]

oder ihres Salzes zur Herstellung einer Verbindung der Formel:

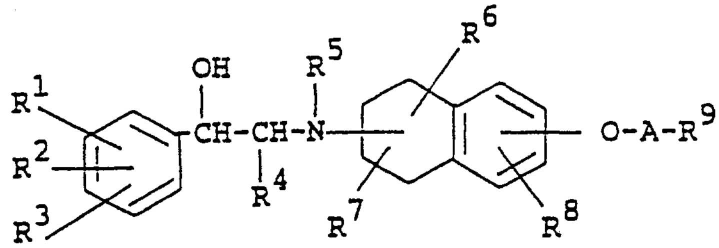

[I]

oder ihres Salzes, wobei in den obigen Formeln $R^1$, $R^2$, $R^3$, $R^4$, $R^5$, $R^6$, $R^7$, $R^8$, $R^9$ und A jeweils wie oben definiert sind, oder

c) Durchfuhrung einer Eliminierungsreaktion der Hydroxyschutzgruppe mit einer Verbindung der Formel:

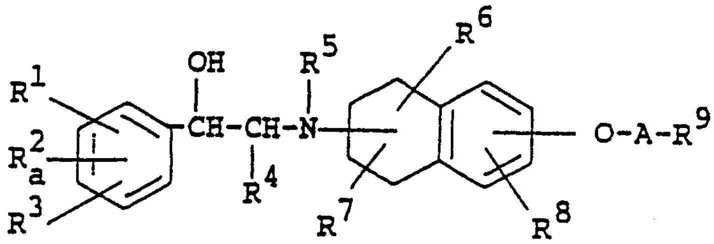

[Ia]

oder ihres Salzes zur Herstellung einer Verbindung der Formel:

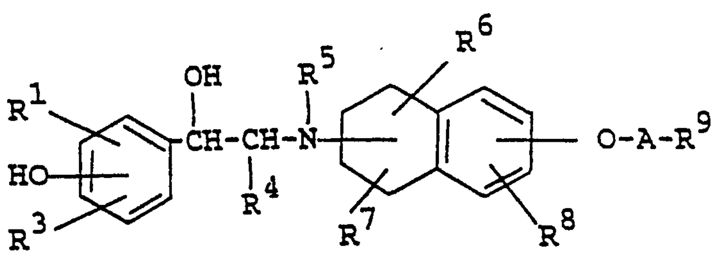

[Ib]

oder ihres Salzes, wobei in den obigen Formeln $R^2_a$ geschütztes Hydroxy ist und $R^1$, $R^3$, $R^4$, $R^5$, $R^6$, $R^7$, $R^8$, $R^9$ und A jeweils wie oben definiert sind, oder

d) Durchführung einer Verseifungsreaktion mit einer Verbindung der Formel:

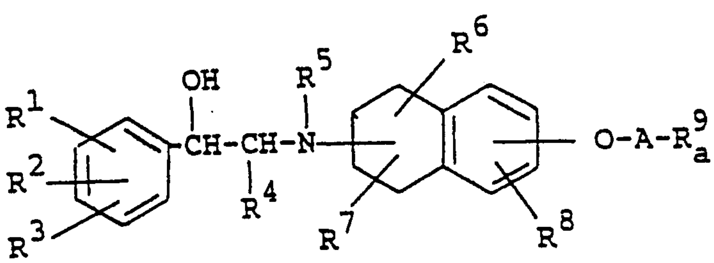

[Ic]

oder ihres Salzes zur Herstellung einer Verbindung der Formel:

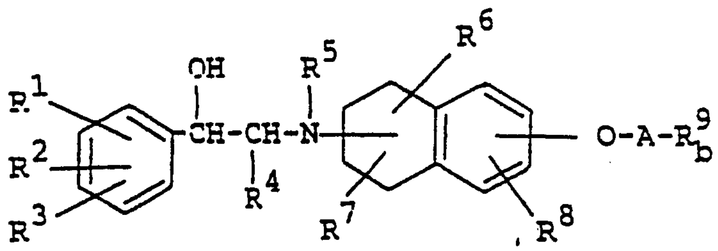

[Id]

oder ihres Salzes, wobei in den obigen Formeln

$R^1$, $R^2$, $R^3$, $R^4$, $R^5$, $R^6$, $R^7$, $R^8$ und A jeweils wie oben definiert sind,
$R^9{}_a$ verestertes Carboxy ist, und
$R^9{}_b$ Carboxy ist oder

e) Umsetzung einer Verbindung der Formel:

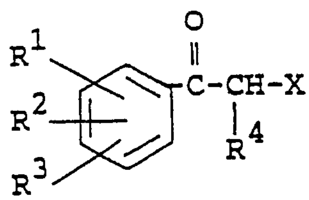

[V]

oder ihres Salzes mit einer Verbindung der Formel:

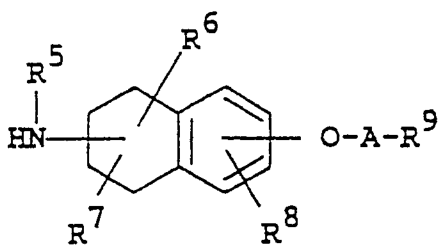

[III]

oder ihres Salzes, und anschließende Durchführung einer Reduktion mit dem resultierenden Produkt zur Herstellung einer Verbindung der Formel:

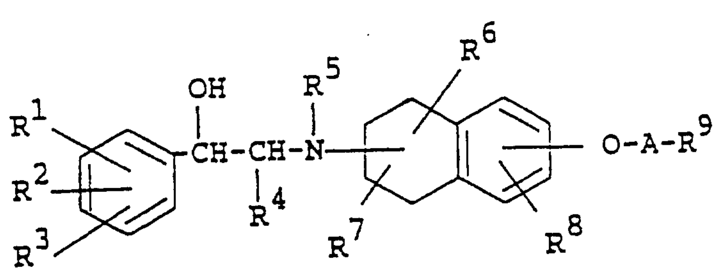

[I]

oder ihres Salzes, wobei in den obigen Formeln $R^1$, $R^2$, $R^3$, $R^4$, $R^5$, $R^6$, $R^7$, $R^8$, $R^9$ und A jeweils wie oben definiert sind und X Halogen ist.

**2.** Verfahren nach Anspruch 1, wobei

$R^4$ Wasserstoff ist und
$R^5$ Wasserstoff ist.

3. Verfahren nach Anspruch 2, wobei

$R^7$ Wasserstoff ist und
$R^8$ Wasserstoff ist.

4. Verfahren nach Anspruch 3, wobei

$R^1$ Halogen ist,
$R^2$ Halogen, Hydroxy oder geschütztes Hydroxy ist,
$R^3$ Wasserstoff ist,
$R^6$ Wasserstoff oder Hydroxy ist, und
$R^9$ niedriges Alkoxycarbonyl ist.

5. Verfahren zur Herstellung einer Verbindung nach Anspruch 4, welche (-)-1-(3-Chlor-4-hydroxyphenyl)-2-[{(2S)-7-ethoxycarbonylmethoxy-1,2,3,4-tetrahydro-2-naphthyl}amino]ethanol oder ihr Hydrochlorid ist.

6. Verfahren zur Herstellung einer pharmazeutischen Zusammensetzung, welche als aktiven Bestandteil eine Verbindung nach Anspruch 1 oder ein pharmazeutisch verträgliches Salz davon umfaßt, charakterisiert durch Mischen einer Verbindung nach Anspruch 1 mit mindestens einem pharmazeutisch verträglichen Träger oder Excipienten.

7. Verfahren nach Anspruch 6, wobei die pharmazeutische Zusammensetzung für die Behandlung und/oder Vorbeugung von Dysurie, Spasmen oder Hyperanakinesie beim Menschen oder bei Tieren vorgesehen ist.

8. Modifikation des in Anspruch 1 beanspruchten Verfahrens, welche dadurch charakterisiert ist, daß eine Verbindung der Formel I oder ein nicht-toxisches Salz davon, hergestellt nach einem in Anspruch 1 beanspruchten Verfahren, durch Mischen oder Präsentieren der Verbindung mit einem pharmazeutisch verträglichen Verdünner oder Träger in pharmazeutisch verträgliche Form gebracht wird.

**Revendications**

**Revendications pour les Etats contractants suivants : AT, BE, CH, DE, DK, FR, GB, IT, LI, LU, NL, SE**

1. Composé répondant à la formule :

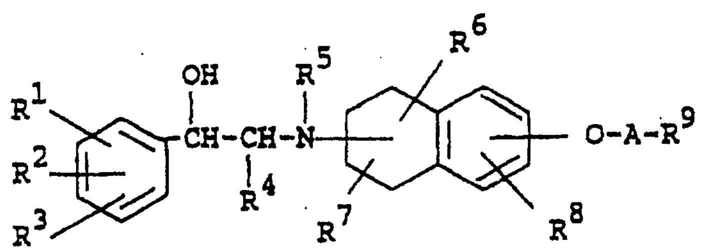

[I]

dans laquelle

$R^1$ est un atome d'hydrogène ou un atome d'halogène,
$R^2$ est un atome d'halogène, un groupe hydroxy, hydroxy protégé, aryloxy, alcoxy inférieur, halo(alcoxy inférieur), nitro, cyano, amino ou acylamino,
$R^3$ est un atome d'hydrogène ou d'halogène,
$R^4$ est un atome d'hydrogène ou un groupe alkyle inférieur,
$R^5$ est un atome d'hydrogène ou un groupe alkyle inférieur,

$R^6$ est un atome d'hydrogène, un groupe hydroxy ou alkyle inférieur,
$R^7$ est un atome d'hydrogène ou un groupe alkyle inférieur,
$R^8$ est un atome d'hydrogène ou d'halogène,
$R^9$ est un groupe carboxy ou carboxy estérifié, et
A est un groupe alkylène inférieur,

sous réserve que $R^4$ est un groupe alkyle inférieur; $R^5$ est un groupe alkyle inférieur; $R^6$ est un groupe hydroxy ou alkyle inférieur; ou $R^8$ est un atome d'halogène lorsque $R^1$ est un atome d'hydrogène, $R^2$ est un atome d'halogène et $R^3$ est un atome d'hydrogène,
et ses sels pharmaceutiquement acceptables.

**2.** Composé selon la revendication 1, dans lequel

$R^4$ est un atome d'hydrogène et
$R^5$ est un atome d'hydrogène.

**3.** Composé selon la revendication 2, dans lequel

$R^7$ est un atome d'hydrogène et
$R^8$ est un atome d'hydrogène.

**4.** Composé selon la revendication 3, dans lequel

$R^1$ est un atome d'halogène,
$R^2$ est un atome d'halogène, un groupe hydroxy ou hydroxy protégé,
$R^3$ est un atome d'hydrogène,
$R^6$ est un atome d'hydrogène ou un groupe hydroxy, et
$R^9$ est un groupe (alcoxy inférieur)carbonyle.

**5.** Composé selon la revendication 4, qui est le (-)-1-(3-chloro-4-hydroxyphényl)-2-[{(2S)-7-éthoxycarbonylméthoxy-1,2,3,4-tétrahydro-2-naphtyl}amino]éthanol ou son chlorhydrate.

**6.** Procédé pour la préparation d'un composé répondant à la formule :

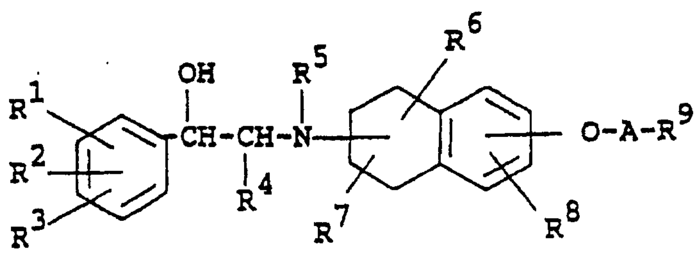

[I]

dans laquelle

$R^1$ est un atome d'hydrogène ou d'halogène,
$R^2$ est un atome d'halogène, un groupe hydroxy, hydroxy protégé, aryloxy, alcoxy inférieur, halo(alcoxy inférieur), nitro, cyano, amino ou acylamino,
$R^3$ est un atome d'hydrogène ou d'halogène,
$R^4$ est un atome d'hydrogène ou un groupe alkyle inférieur,
$R^5$ est un atome d'hydrogène ou un groupe alkyle inférieur,
$R^6$ est un atome d'hydrogène, un groupe hydroxy ou alkyle inférieur,
$R^7$ est un atome d'hydrogène ou un groupe alkyle inférieur,
$R^8$ est un atome d'hydrogène ou d'halogène,

$R^9$ est un groupe carboxy ou carboxy estérifié, et
A est un groupe alkylène inférieur,

sous réserve que $R^4$ est un groupe alkyle inférieur; $R^5$ est un groupe alkyle inférieur; $R^6$ est un groupe hydroxy ou alkyle inférieur; ou $R^8$ est un atome d'halogène lorsque $R^1$ est un atome d'hydrogène, $R^2$ est un atome d'halogène et $R^3$ est un atome d'hydrogène,
ou ses sels pharmaceutiquement acceptables,
qui comprend :

a) le fait de faire réagir un composé répondant à la formule :

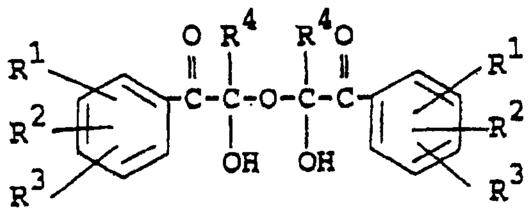

[II]

ou un de ses sels, avec un composé répondant à la formule :

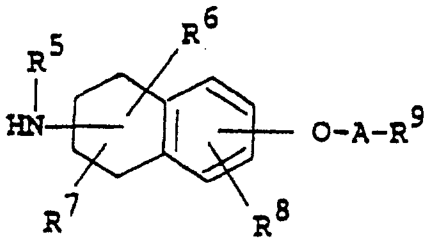

[III]

ou un de ses sels, en présence d'un agent réducteur pour donner un composé répondant à la formule :

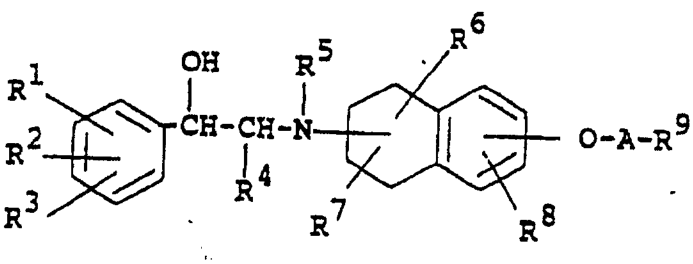

[I]

ou un de ses sels; dans les formules ci-dessus, $R^1$, $R^2$, $R^3$, $R^4$, $R^5$, $R^6$, $R^7$, $R^8$, $R^9$ et A sont chacun tels que définis ci-dessus, ou
b) le fait de faire réagir un composé répondant à la formule :

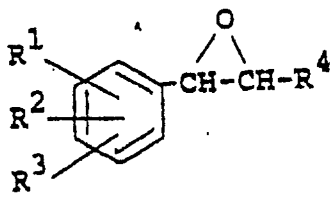

[IV]

ou un de ses sels, avec un composé répondant à la formule :

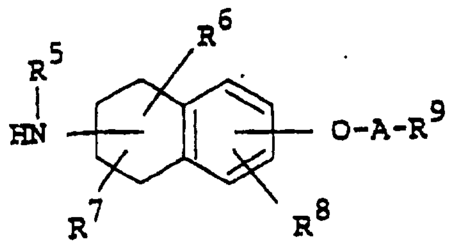

[III]

ou un de ses sels, pour donner un composé répondant à la formule :

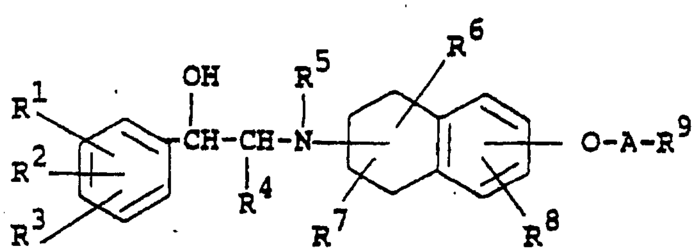

[I]

ou un de ses sels; dans les formules ci-dessus, $R^1$, $R^2$, $R^3$, $R^4$, $R^5$, $R^6$, $R^7$, $R^8$, $R^9$ et A sont chacun tels que définis ci-dessus, ou

c) le fait de soumettre un composé répondant à la formule :

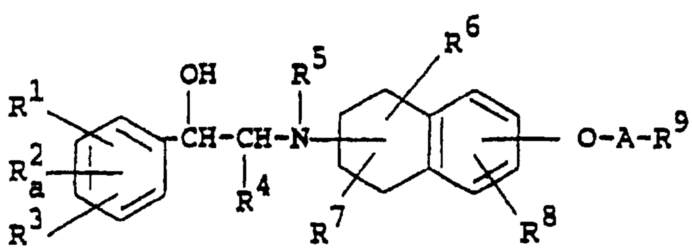

[Ia]

ou un de ses sels à une réaction d'élimination du groupe protecteur du groupe hydroxy pour donner un composé répondant à la formule :

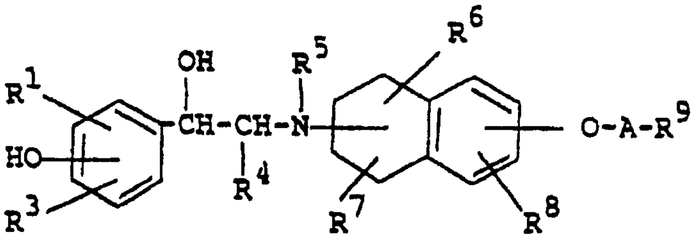

[Ib]

ou un de ses sels, dans les formules ci-dessus, $R_a^2$ est un groupe hydroxy protégé, et $R^1$, $R^3$, $R^4$, $R^5$, $R^6$, $R^7$, $R^8$, $R^9$ et A sont chacun tels que définis ci-dessus, ou

d) le fait de soumettre un composé répondant à la formule :

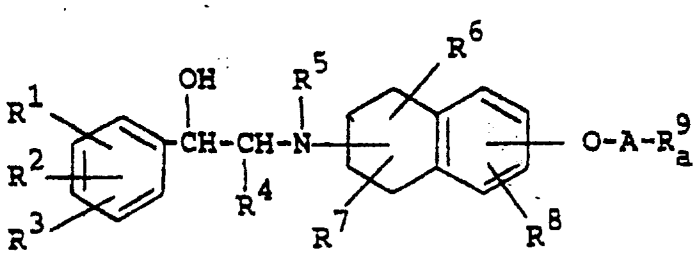

[Ic]

ou un de ses sels à une réaction de désestérification pour donner un composé répondant à la formule :

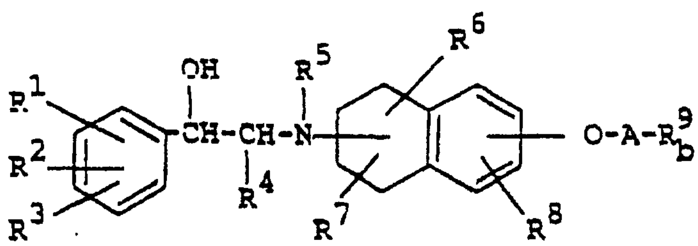

[Id]

ou un de ses sels; dans les formules ci-dessus,

$R^1$, $R^2$, $R^3$, $R^4$, $R^5$, $R^6$, $R^7$, $R^8$ et A sont chacun tels que définis ci-dessus,
$R_a^9$ est un groupe carboxy estérifié, et
$R_b^9$ est un groupe carboxy, ou

e) le fait de faire réagir un composé répondant à la formule :

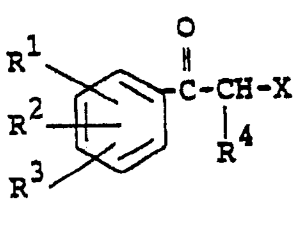

[V]

ou un de ses sels, avec un composé répondant à la formule :

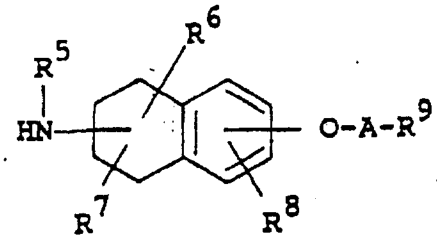

[III]

ou un de ses sels, puis de soumettre le produit obtenu à une réduction pour donner un composé répondant à la formule :

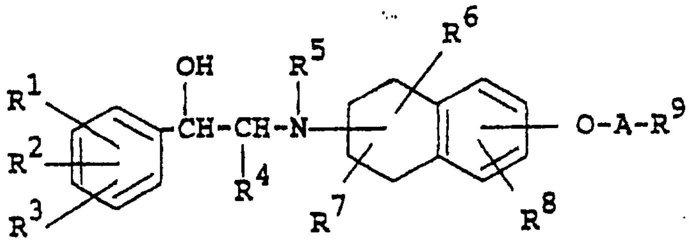

[I]

ou un de ses sels; dans les formules ci-dessus, $R^1$, $R^2$, $R^3$, $R^4$, $R^5$, $R^6$, $R^7$, $R^8$, $R^9$ et A sont chacun tels que définis ci-dessus, et X est un atome d'halogène.

**7.** Composition pharmaceutique comprenant un composé selon la revendication 1, comme ingrédient actif, associé avec un support ou excipient pharmaceutiquement acceptable, pratiquement non toxique.

**8.** Composé selon la revendication 1 pour l'utilisation comme médicament.

**9.** Utilisation d'un composé selon la revendication 1 pour la fabrication d'un médicament pour le traitement et/ou la prévention de la dysurie, du spasme ou de l'hyperkinésie chez l'homme ou les animaux.

**Revendications pour l'Etat contractant suivant : ES**

**1.** Procédé de préparation d'un composé répondant à la formule :

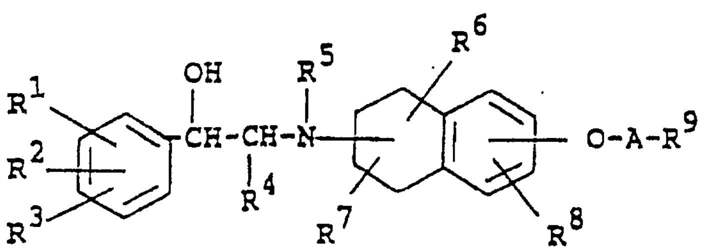

[I]

dans laquelle

| | |
|---|---|
| $R^1$ est | un atome d'hydrogène ou d'halogène, |
| $R^2$ est | un atome d'halogène, un groupe hydroxy, hydroxy protégé, aryloxy, alcoxy inférieur, halo(alcoxy inférieur), nitro, cyano, amino ou acylamino, |
| $R^3$ est | un atome d'hydrogène ou d'halogène, |
| $R^4$ est | un atome d'hydrogène ou un groupe alkyle inférieur, |
| $R^5$ est | un atome d'hydrogène ou un groupe alkyle inférieur, |

| | |
|---|---|
| $R^6$ est | un atome d'hydrogène, un groupe hydroxy ou alkyle inférieur, |
| $R^7$ est | un atome d'hydrogène, ou un groupe alkyle inférieur, |
| $R^8$ est | un atome d'hydrogène ou d'halogène, |
| $R^9$ est | un groupe carboxy ou un groupe carboxy estérifié, et |
| A est | un groupe alkylène inférieur, |

sous réserve que $R^4$ est un groupe alkyle inférieur; $R^5$ est un groupe alkyle inférieur; $R^6$ est un groupe hydroxy ou alkyle inférieur; ou $R^8$ est un atome d'halogène lorsque $R^1$ est un atome d'hydrogène, $R^2$ est un atome d'halogène et $R^3$ est un atome d'hydrogène,
ou un de ses sels pharmaceutiquement acceptables,
qui comprend :

a) le fait de faire réagir un composé répondant à la formule :

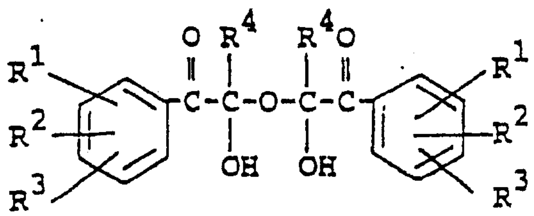

[II]

ou un de ses sels, avec un composé répondant à la formule :

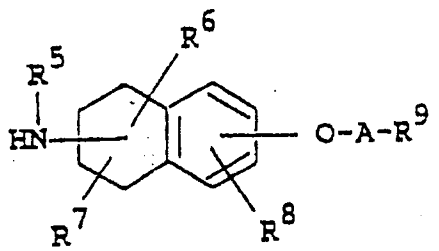

[III]

ou un de ses sels, en présence d'un agent réducteur pour donner un composé répondant à la formule :

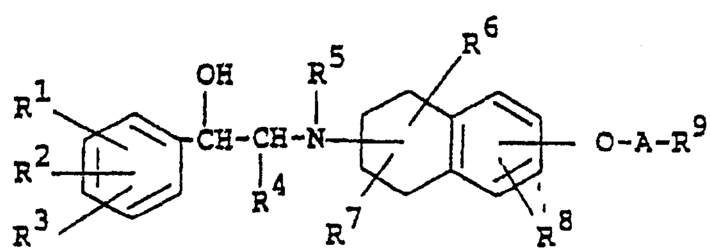

[I]

ou un de ses sels; dans les formules ci-dessus, $R^1$, $R^2$, $R^3$, $R^4$, $R^5$, $R^6$, $R^7$, $R^8$, $R^9$ et A sont chacun tels que définis ci-dessus, ou
b) le fait de faire réagir un composé répondant à la formule :

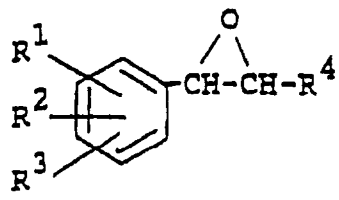

[IV]

ou un de ses sels, avec un composé répondant à la formule :

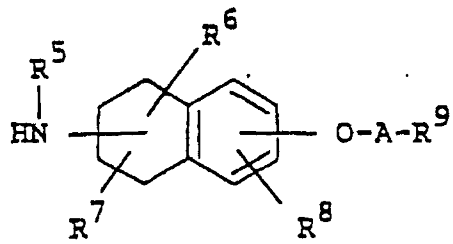

[III]

ou un de ses sels, pour donner un composé répondant à la formule :

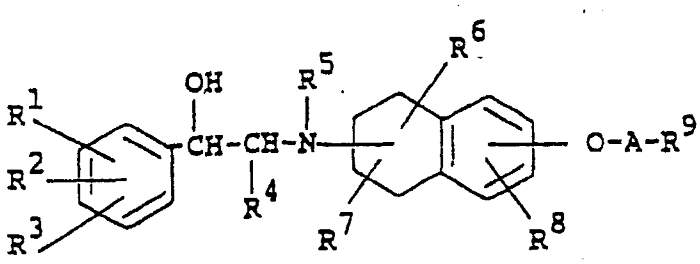

[I]

ou un de ses sels; dans les formules ci-dessus, $R^1$, $R^2$, $R^3$, $R^4$, $R^5$, $R^6$, $R^7$, $R^8$, $R^9$ et A sont chacun tels que définis ci-dessus, ou

c) le fait de soumettre un composé répondant à la formule :

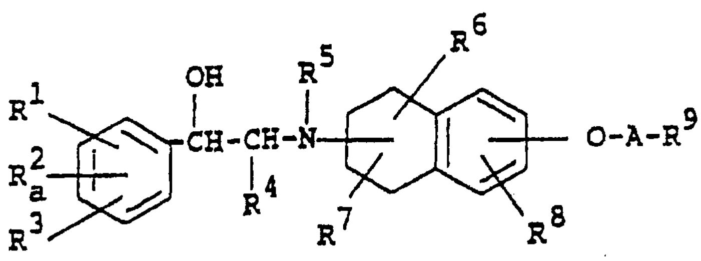

[Ia]

ou un de ses sels à une réaction d'élimination du groupe protecteur du groupe hydroxy pour donner un composé répondant à la formule :

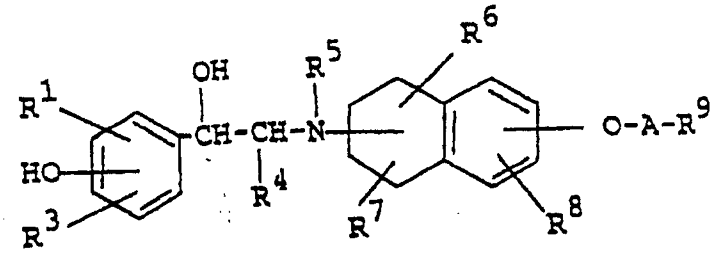

[Ib]

ou un de ses sels; dans les formules ci-dessus, $R_a^2$ est un groupe hydroxy protégé, et $R^1$, $R^3$, $R^4$, $R^5$, $R^6$, $R^7$, $R^8$, $R^9$ et A sont chacun tels que définis ci-dessus, ou

d) le fait de soumettre un composé répondant à la formule :

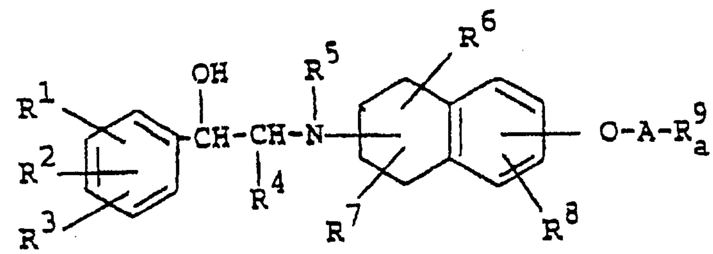

[Ic]

ou un de ses sels, à une réaction de désestérification pour donner un composé répondant à la formule :

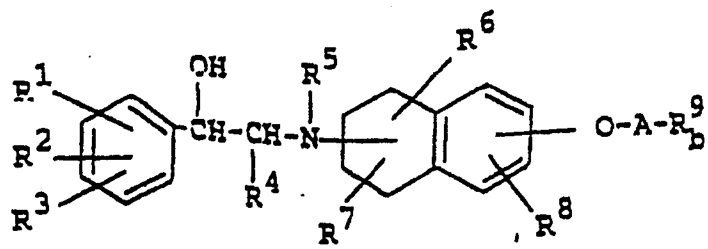

[Id]

ou un de ses sels; dans les formules ci-dessus,

$R^1$, $R^2$, $R^3$, $R^4$, $R^5$, $R^6$, $R^7$, $R^8$ et A sont chacun tels que définis ci-dessus,
$R_a^9$ est un groupe carboxy estérifié, et
$R_b^9$ est un groupe carboxy, ou

e) le fait de faire réagir un composé répondant à la formule :

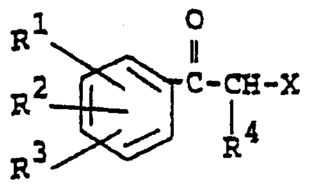

[V]

ou un de ses sels, avec un composé répondant à la formule :

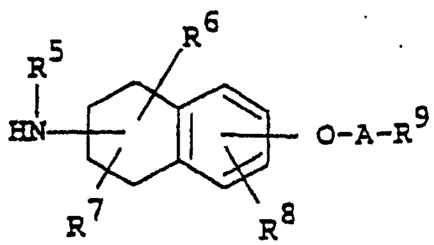

[III]

ou un de ses sels, puis de soumettre le produit obtenu à une réduction pour donner un composé répondant à la formule :

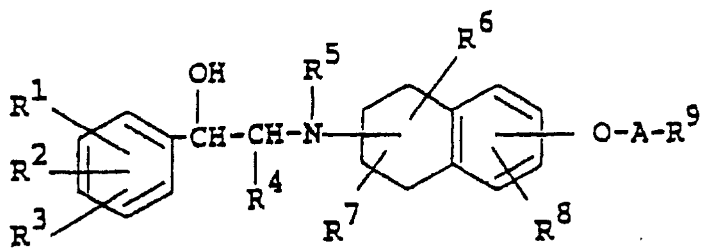

[I]

ou un de ses sels; dans les formules ci-dessus, $R^1$, $R^2$, $R^3$, $R^4$, $R^5$, $R^6$, $R^7$, $R^8$, $R^9$ et A sont chacun tels que définis ci-dessus, et X est un atome d'halogène.

2. Procédé selon la revendication 1, dans lequel

$R^4$ est un atome d'hydrogène et
$R^5$ est un atome d'hydrogène.

3. Procédé selon la revendication 2, dans lequel

$R^7$ est un atome d'hydrogène et
$R^8$ est un atome d'hydrogène.

4. Procédé selon la revendication 3, dans lequel

$R^1$ est un atome d'halogène,
$R^2$ est un atome d'halogène, un groupe hydroxy ou hydroxy protégé,
$R^3$ est un atome d'hydrogène,

$R^6$ est un atome d'hydrogène ou un groupe hydroxy, et
$R^9$ est un groupe (alcoxy inférieur)carbonyle.

5. Procédé de préparation d'un composé selon la revendication 4 qui est le (-)-1-(3-chloro-4-hydroxyphényl)-2-[{(2S)-7-éthoxycarbonylméthoxy-1,2,3,4-tétrahydro-2-naphtyl}amino]éthanol ou son chlorhydrate.

6. Procédé de préparation d'une composition pharmaceutique qui comprend, comme ingrédient actif, un composé répondant à la revendication 1 ou un de ses sels pharmaceutiquement acceptables, caractérisé en ce qu'on mélange un composé répondant à la revendication 1 avec au moins un support ou excipient pharmaceutiquement acceptable.

7. Procédé selon la revendication 6, dans lequel la composition pharmaceutique est destinée au traitement et/ou à la prévention de la dysurie, du spasme ou de l'hyperkinésie chez l'homme ou les animaux.

**Revendications pour l'Etat contractant suivant : GR**

1. Procédé de préparation d'un composé répondant à la formule :

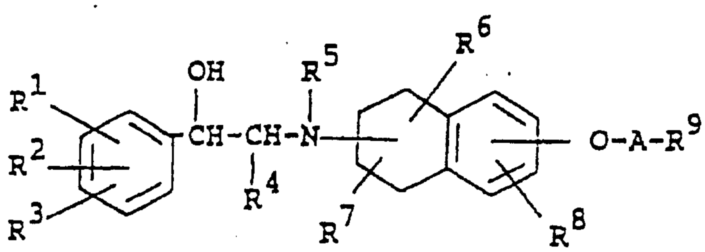

[I]

dans laquelle

$R^1$ est un atome d'hydrogène ou d'halogène,
$R^2$ est un atome d'halogène, un groupe hydroxy, hydroxy protégé, aryloxy, alcoxy inférieur, halo(alcoxy inférieur), nitro, cyano, amino ou acylamino,
$R^3$ est un atome d'hydrogène ou d'halogène,
$R^4$ est un atome d'hydrogène ou un groupe alkyle inférieur,
$R^5$ est un atome d'hydrogène ou un groupe alkyle inférieur,
$R^6$ est un atome d'hydrogène, un groupe hydroxy ou un groupe alkyle inférieur,
$R^7$ est un atome d'hydrogène ou un groupe alkyle inférieur,
$R^8$ est un atome d'hydrogène ou d'halogène,
$R^9$ est un groupe carboxy ou carboxy estérifié, et
A est un groupe alkylène inférieur,

sous réserve que $R^4$ est un groupe alkyle inférieur; $R^5$ est un groupe alkyle inférieur; $R^6$ est un groupe hydroxy ou alkyle inférieur; ou $R^8$ est un atome d'halogène lorsque $R^1$ est un atome d'hydrogène, $R^2$ est un atome d'halogène et $R^3$ est un atome d'hydrogène,
ou un de ses sels pharmaceutiquement acceptables,
qui comprend :

a) le fait de faire reagir un composé répondant à la formule :

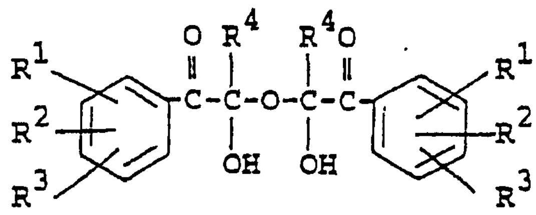

[II]

ou un de ses sels, avec un composé répondant à la formule :

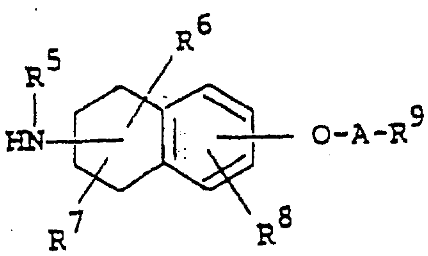

[III]

ou un de ses sels, en présence d'un agent réducteur pour donner un composé répondant à la formule :

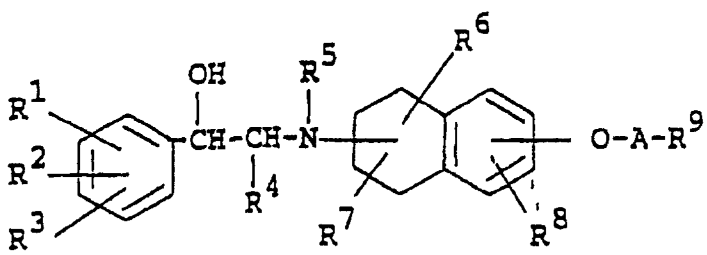

[I]

ou un de ses sels; dans les formules ci-dessus, $R^1$, $R^2$, $R^3$, $R^4$, $R^5$, $R^6$, $R^7$, $R^8$, $R^9$ et A sont chacun tels que définis ci-dessus, ou

b) le fait de faire réagir un composé répondant à la formule :

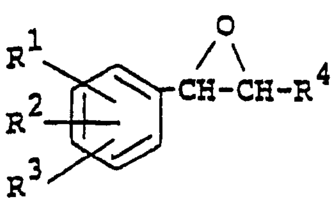

[IV]

ou un de ses sels, avec un composé répondant à la formule :

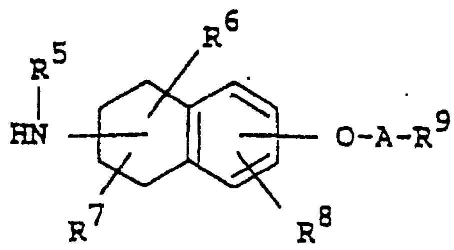

[III]

ou un de ses sels, pour donner un composé répondant à la formule :

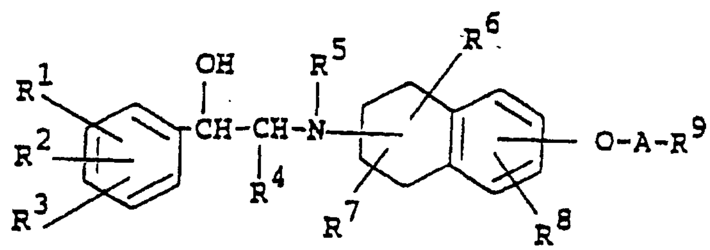

[I]

ou un de ses sels; dans les formules ci-dessus, $R^1$, $R^2$, $R^3$, $R^4$, $R^5$, $R^6$, $R^7$, $R^8$, $R^9$ et A sont chacun tels que définis ci-dessus, ou

c) le fait de soumettre un composé répondant à la formule :

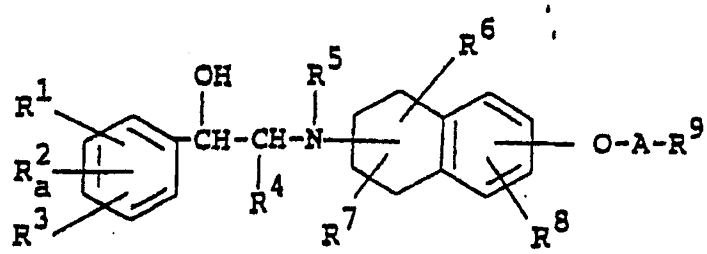

[Ia]

ou un de ses sels, à une réaction d'élimination du groupe protecteur du groupe hydroxy pour donner un composé répondant à la formule :

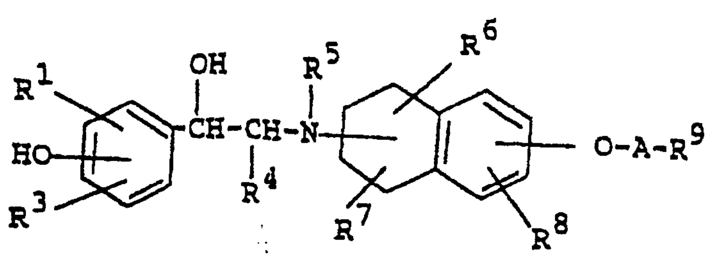

[Ib]

ou un de ses sels; dans les formules ci-dessus, $R^2_a$ est un groupe hydroxy protégé, et $R^1$, $R^3$, $R^4$, $R^5$, $R^6$, $R^7$, $R^8$, $R^9$ et A sont chacun tels que définis ci-dessus, ou

d) le fait de soumettre un composé répondant à la formule :

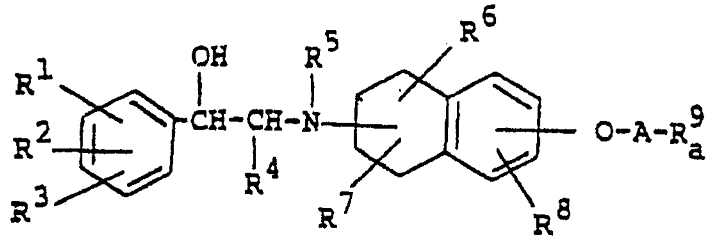

[Ic]

ou un de ses sels, à une réaction de désestérification pour donner un composé répondant à la formule :

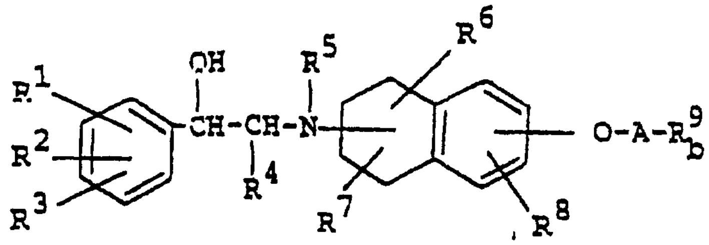

[Id]

ou un de ses sels; dans les formules ci-dessus,

$R^1$, $R^2$, $R^3$, $R^4$, $R^5$, $R^6$, $R^7$, $R^8$ et A sont chacun tels que définis ci-dessus,
$R^9_a$ est un groupe carboxy estérifié, et
$R^9_b$ est un groupe carboxy, ou

e) le fait de faire réagir un composé répondant à la formule :

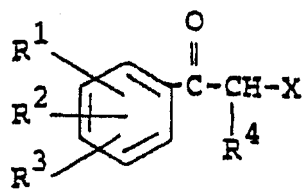

[V]

ou un de ses sels, avec un composé répondant à la formule :

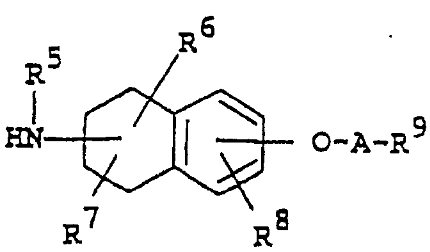

[III]

ou un de ses sels, puis de soumettre le produit obtenu à une réduction pour obtenir un composé répondant à la formule :

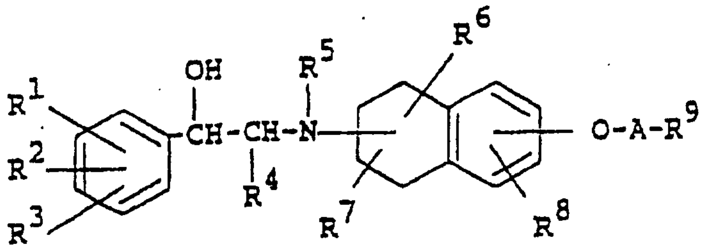

[I]

ou un de ses sels; dans les formules ci-dessus, $R^1$, $R^2$, $R^3$, $R^4$, $R^5$, $R^6$, $R^7$, $R^8$, $R^9$ et A sont chacun tels que définis ci-dessus, et X est un atome d'halogène.

**2.** Procédé selon la revendication 1, dans lequel

$R^4$ est un atome d'hydrogène et
$R^5$ est un atome d'hydrogène.

**3.** Procédé selon la revendication 2, dans lequel

$R^7$ est un atome d'hydrogène et
$R^8$ est un atome d'hydrogène.

**4.** Procédé selon la revendication 3, dans lequel

$R^1$ est un atome d'halogène,
$R^2$ est un atome d'halogène, un groupe hydroxy ou hydroxy protégé,
$R^3$ est un atome d'hydrogène,
$R^6$ est un atome d'hydrogène ou un groupe hydroxy, et
$R^9$ est un groupe (alcoxy inférieur)carbonyle.

**5.** Procédé pour préparer un composé selon la revendication 4, qui est le (-)-1-(3-chloro-4-hydroxyphényl)-2-[{(2S)-7-éthoxycarbonylméthoxy-1,2,3,4-tétrahydro-2-naphtyl}amino]éthanol ou son chlorhydrate.

**6.** Procédé pour préparer une composition pharmaceutique qui comprend, comme ingrédient actif, un composé répondant à la revendication 1 ou un de ses sels pharmaceutiquement acceptables, caractérisé en ce qu'on mélange un composé répondant à la revendication 1 avec au moins un support ou excipient pharmaceutiquement acceptable.

**7.** Procédé selon la revendication 6, dans lequel la composition pharmaceutique est destinée au traitement et/ou à la prévention de la dysurie, du spasme ou de l'hyperkinésie chez l'homme ou les animaux.

**8.** Variante du procédé selon la revendication 1, qui est caractérisée en ce qu'on amène un composé répondant à la formule I ou un de ses sels non-toxiques, préparés par un procédé revendiqué dans la revendication 1, sous une forme pharmaceutiquement acceptable par mélange ou présentation de ce composé avec un diluant ou support pharmaceutiquement acceptable.